# FASCICULE DE BREVET EUROPEEN

(11) **EP 0 752 991 B1**
(45) Date de publication et mention de la délivrance du brevet: **26.05.1999**
(21) Numéro de dépôt: 95914403.1
(22) Date de dépôt: 23.03.1995
(51) Int. Cl.: C07D 487/04, A61K 31/495, C07D 471/14, C07D 495/14, C07D 491/147

(54) **IMIDAZO[1,2-a]INDENO[1,2-e]PYRAZIN-4-ONES ANTAGONISTES DES RECEPTEURS AMPA ET NMDA**
IMIDAZO[1,2-a]INDENO [1,2-e] PYRAZINONE ANTAGONISTEN DER AMPA UND NMDA REZEPTOREN
IMIDAZO[1,2-a]INDENO[1,2-e]PYRAZIN-4-ONES ANTAGONISTS OF AMPA AND NMDA RECEPTORS

(30) Priorité: 28.03.1994 FR 9403581
(43) Date de publication de la demande: 15.01.1997
(73) Titulaire: RHONE-POULENC RORER S.A., 92160 Antony (FR)
(72) Inventeur: ALOUP, Jean-Claude, F-94290 Villeneuve-le-Roi (FR); AUDIAU, François, F-94220 Charenton-le-Pont (FR); BARREAU, Michel, F-91230 Montgeron (FR); DAMOUR, Dominique, F-75014 Paris (FR); GENEVOIS-BORELLA, Arielle, F-94320 Thiais (FR); JIMONET, Patrick, F-78450 Villepreux (FR); MIGNANI, Serge, F-92290 Chatenay-Malabry (FR); RIBEILL, Yves, F-91360 Villemoisson-sur-Orge (FR)
(74) Mandataire: Savina, Jacques
(86) Numéro de dépôt international: FR9500357
(87) Numéro de publication internationale: WO9526349

(56) Documents cités:
- EP-A- 0 518 530
- WO-A-94/00124
- WO-A-94/07893
- WO-A-95/02601
- WO-A-95/02602

## Description

La présente invention concerne des composés de formule : 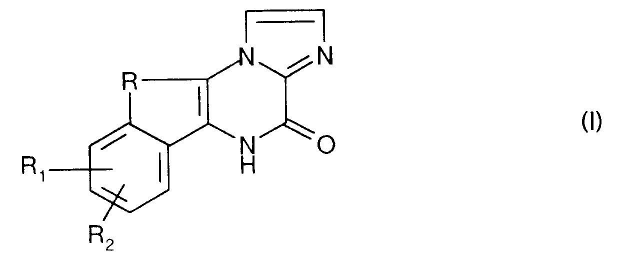 leurs sels, leur préparation et les médicaments les contenant.

Les documents WO-94/07893, WO-95/02601 et WO-95/02602, publiés après la date de priorité de la présente demande, ainsi que EP-518530 décrivent des dérivés d'imidazo[1,2-a]pyrazin-4-one ayant des propriétés antagonistes des récepteurs AMPA et NMDA.

Dans la formule (I),
- R représente un radical C=R₃ ou CH-R₆,
- R₁ représente un radical hydroxy, polyfluoroalcoxy, carboxy, -NH-CHO, -N(alk)-CO-NR₈R₉, -NH-CO-NR₉R₁₂, -NH-CS-NR₈R₉, -NH-CO-R₁₀, -NH-SO₂-NR₇R₉, -CO-NR₇R₉, -NH-SO₂-alk, -NR₉R₁₁, -S-alk-Ar, -SO₂-NR₇R₉, 2-oxo-1-imidazolidinyle,
- R₂ représente un atome d'hydrogène,
- R₃ représente un atome d'oxygène ou un radical NOH,
- R₆ représente un atome d'hydrogène ou un radical amino,
- R₇ représente un atome d'hydrogène ou un radical alkyle,
- R₈ représente un atome d'hydrogène ou un radical alkyle, -alk-NR₉R₇ ou phényle,
- R₉ représente un atome d'hydrogène ou un radical alkyle,
- R₁₀ représente un radical alkyle (5-9 C en chaîne droite ou ramifiée), alcoxy, -alk-COOR₂₁, -alk-NR₉R₇, phénylalkyle, phényle ou un hétérocycle choisi parmi pyrrolidine, azétidine et morpholine,
- R₁₁ représente un hétérocycle choisi parmi imidazoline et thiazoline,
- R₁₂ représente un atome d'hydrogène ou un radical alkyle, -alk-COOR₂₁, phénylalkyle dont le noyau phényle est éventuellement substitué par un ou plusieurs substituants choisis parmi les atomes d'halogène et les radicaux alcoxy et -COOR₂₁ et phényle substitué par un ou plusieurs substituants choisis parmi les atomes d'halogène et les radicaux alcoxy, nitro, amino, -COOR₂₁ et cyano,
- R₂₁ représente un atome d'hydrogène ou un radical alkyle,
- Ar représente un radical phényle,
- alk représente un radical alkyle ou alkylène.

Sauf mention contraire, dans les définitions qui précédent et celles qui suivent, les radicaux et portions alkyle et alcoxy contiennent 1 à 6 atomes de carbone en chaîne droite ou ramifiée et les atomes d'halogène sont choisis parmi le fluor, le chlore, le brome et l'iode.

De préférence, les radicaux polyfluoroalcoxy sont des radicaux trifluorométhoxy.

Les composés de formule (I) pour lesquels R₃ représente un radical NOH, présentent des formes isomères (E et Z). Ces isomères et leurs mélanges font partie de l'invention.

Les énantiomères et diastéréoisomères des composés de formule (I) pour lesquels R représente un radical CH-R₆ font également partie de l'invention.

Les composés de formule (I) pour lesquels R représente un radical C=R₃ dans lequel R₃ représente un atome d'oxygène peuvent être préparés par hydrolyse des composés de formule (I) correspondants pour lesquels R représente un radical C=R₃ et R₃ représente un radical NOH.

Cette réaction s'effectue généralement au moyen d'un acide, en milieu aqueux, à la température d'ébullition du milieu réactionnel. Comme acide, on utilise de préférence l'acide chlorhydrique.

Les composés de formule (I) pour lesquels R représente un radical C=R₃ et R₃ représente un radical NOH peuvent être préparés par action d'un nitrite d'alkyle sur un composé de formule (I) correspondant pour lequel R représente un radical CH-R₆ et R₆ représente un atome d'hydrogène.

Cette réaction s'effectue de préférence au sein d'un solvant inerte tel que le diméthylsulfoxyde, en présence d'un hydrure de métal alcalin tel que l'hydrure de sodium, à une température voisine de 20°C. De préférence, on utilise le nitrite d'isoamyle.

Les composés de formule (I) pour lesquels R représente un radical CH-R₆ et R₆ représente un atome d'hydrogène peuvent être préparés par désalkylation et désalification des dérivés de formule : 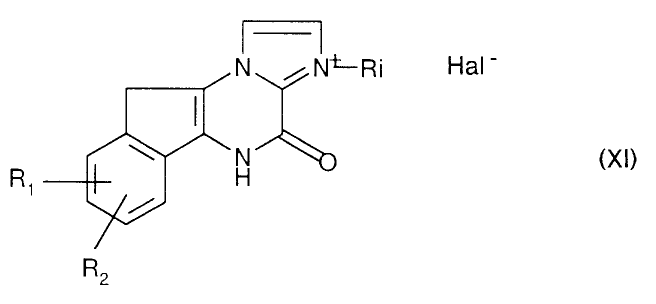 dans laquelle R₁ et R₂ ont les mêmes significations que dans la formule (I), Ri représente un radical alkyle et Hal représente un atome d'halogène et, de préférence, un atome de brome.

Cette réaction s'effectue, de préférence, en présence d'imidazole, à une température comprise entre 100 et 200°C et en particulier à 160°C.

Les dérivés de formule (XI) peuvent être obtenus par action d'un 1-alkyl 1H-imidazole-2-carboxamide sur une 2-halogénoindanone de formule : 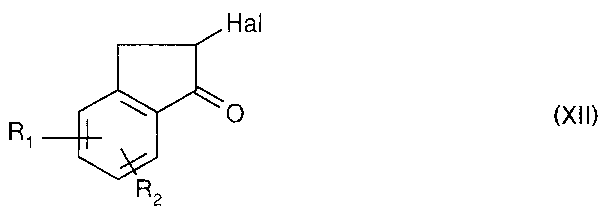 dans laquelle R₁ et R₂ ont les mêmes significations que dans la formule (I) et Hal représente un atome d'halogène et, de préférence, un atome de brome.

Cette réaction s'effectue généralement au sein d'un solvant inerte tel que le diméthylformamide, à une température comprise entre 50 et 150°C et, de préférence à 115°C.

Les dérivés de formule (XII) peuvent être obtenus par halogénation des indanones correspondantes au moyen d'un agent d'halogénation tel que le brome, le chlore, au sein d'un solvant inerte tel qu'un solvant chloré (chlorure de méthylène, chloroforme par exemple), à une température de -15°C ou au sein de l'acide acétique, à une température voisine de 20°C, ou un halogènure de cuivre, au sein du dioxanne, à une température voisine de 100°C ou par application ou adaptation des méthodes décrites par K. MORI, Agr. Biol. Chem., 27 (1), 22 (1963); J. CHAKRAVARTY, Indian J. Chem., 7 (3), 215 (1969), F. G. HOLLIMAN et coll., J. Chem. Soc., 9 (1960), D. MUKHOPADHYA et coll., J. Indian Chem. Soc., 47 (5), 450 (1970) et dans les brevets DE 2640358, EP 346107.

Les indanones peuvent être obtenues par application ou adaptation des méthodes décrites par M. OLIVIER et coll., Bull. Soc. Chim. de France, 3092 (1973), R. SEKA et coll., Chem. Ber., 75B, 1730 (1942), J.J. HOWBERT et coll., Synth. Commun., 20 (20), 3197 (1990), D.F. BIGGS et coll., J. Med. Chem., 19 (4), 472 (1976), C.K. INGOLD et coll., J. Chem. Soc., 1469 (1923), dans les brevets US 4263319, 4096173, JP 80161237 et EP 314400 et dans les exemples.

Les composés de formule (I) pour lesquels R représente un radical CH-R₆ et R₆ représente un atome d'hydrogène peuvent également être préparés par cyclisation éventuellement en présence d'acétate d'ammonium d'un dérivé de formule : 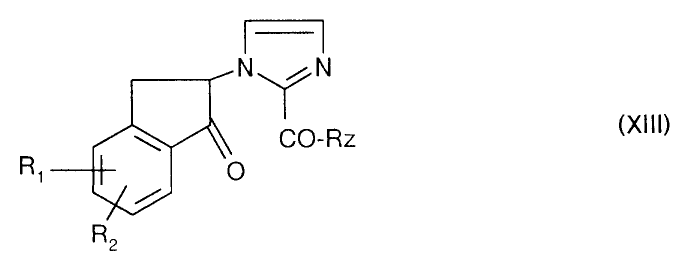 dans laquelle R₁ et R₂ ont les mêmes significations que dans la formule (I) et Rz représente un radical -NH₂ ou -Oalk dans lequel alk représente un radical alkyle.

Cette cyclisation s'effectue au moyen d'un acide tel que l'acide acétique, l'acide chlorhydrique, en milieu aqueux ou au sein d'un alcool tel que l'éthanol ou le méthanol, à la température d'ébullition du milieu réactionnel.

Les dérivés de formule (XIII) pour lesquels Rz représente un radical -NH₂ peuvent être obtenus par action d'ammoniac sur un dérivé de formule (XIII) correspondant pour lequel Rz représente un radical -Oalk.

Cette réaction s'effectue généralement au sein d'un solvant inerte tel qu'un alcool, à une température comprise entre 20°C et la température d'ébullition du milieu réactionnel.

Les dérivés de formule (XIII) pour lesquels Rz représente un radical -Oalk peuvent être obtenus par action d'une 2-halogénoindanone de formule (XII) sur un 2-alcoxycarbonylimidazole.

Cette réaction s'effectue soit par fusion à une température comprise entre 130 et 180°C, soit au sein d'un solvant inerte tel que le diméthylformamide en présence d'une base telle qu'un hydrure de métal alcalin (hydrure de sodium par exemple), à une température voisine de 20°C, soit au sein d'un solvant inerte tel qu'un solvant chloré tel que le chloroforme en présence d'une base organique azotée (1,8-diazabicyclo[5,4,0] undéc-7-ène par exemple), à une température voisine de 20°C, soit au sein d'un solvant inerte tel qu'un alcool (éthanol, propanol par exemple), un solvant aromatique tel que le toluène, un solvant chloré (chloroforme par exemple), éventuellement en présence d'iodure de sodium, à la température d'ébullition du milieu réactionnel, soit au sein de l'acétone, en présence d'un carbonate de métal alcalin, à une température comprise entre 20°C et la température d'ébullition du milieu réactionnel.

Les 2-alcoxycarbonylimidazoles peuvent être obtenus par application ou adaptation de la méthode décrite dans le brevet US 3600399.

Les composés de formule (I) pour lesquels R représente un radical CH-R₆ dans lequel R₆ représente un radical amino peuvent être préparés par hydrolyse d'un composé de formule : 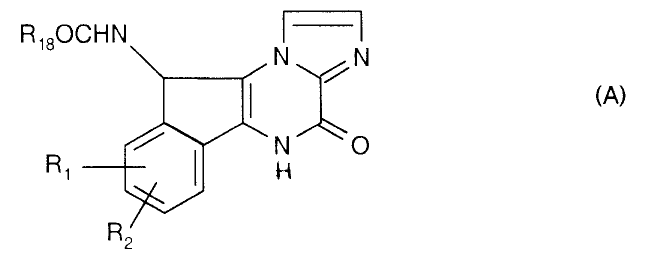 dans laquelle R₁ et R₂ ont les mêmes significations que dans la formule (I) et R₁₈ représente un radical alkyle.

Cette hydrolyse s'effectue généralement au moyen d'un acide tel que l'acide chlorhydrique, en milieu aqueux, à la température d'ébullition du milieu réactionnel.

Les composés de formule (I) pour lesquels R représente un radical CH-R₆ dans lequel R₆ représente un radical amino peuvent aussi être préparés par réduction d'un composé de formule (I) correspondant pour lequel R représente un radical C=R₃ dans lequel R₃ représente un radical NOH.

Cette réduction s'effectue généralement au moyen de zinc, en présence d'acétate d'ammonium et d'ammoniaque à 28%, au sein d'un alcool aliphatique tel que l'éthanol, à la température d'ébullition du milieu réactionnel.

Les composés de formule (A) peuvent être préparés par action d'un agent réducteur sur un composé de formule (I) correspondant pour lequel R représente un radical C=R₃ et R₃ représente un radical NOH puis d'un traitement avec un anhydride (RkCO)₂O pour lequel Rk représente un radical alkyle (1-5C).

Cette réaction s'effectue généralement au sein de l'acide acétique, à une température comprise entre 50 et 100°C. Comme agent réducteur on utilise de préférence le zinc.

Les composés de formule (A) peuvent être préparés par action d'un composé de formule (I) correspondant pour lequel R représente un radical CH-R₆, R₆ représente un radical amino sur un halogènure de formule Hal-Rl dans laquelle Rl représente un radical -COR₁₈ dans lequel R₁₈ représente un radical alkyle.

Cette réaction s'effectue de préférence au sein d'un solvant inerte tel que le diméthylformamide, le tétrahydrofuranne ou le diméthylsulfoxyde, en présence d'une base telle qu'une amine tertiaire (la triéthylamine par exemple) ou aromatique (pyridine par exemple) ou une base minérale telle qu'un hydroxyde de métal alcalin (soude, potasse par exemple), à une température comprise entre 20°C et la température d'ébullition du milieu réactionnel.

Les halogénures Hal-Rl sont commercialisés ou peuvent être obtenus à partir des acides carboxyliques correspondants par adaptation des méthodes décrites par B. HELFERICH et coll., Organic Synth., I, 147; R. ADAMS et coll., Organic Synth., I, 394 ou J. CASON, Organic Synth., III, 169.

Les composés de formule (I) pour lesquels R₁ représente un radical hydroxy peuvent également être préparés par hydrolyse d'un dérivé de formule : 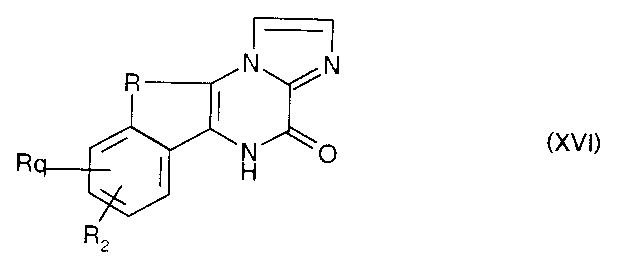 dans laquelle R et R₂ ont les mêmes significations que dans la formule (I) et Rq représente un radical alcoxy.

Cette réaction s'effectue par toute méthode connue d'hydrolyse d'une fonction alcoxy en fonction hydroxy qui ne modifie pas le reste de la molécule. De préférence, cette hydrolyse s'effectue au moyen d'acide bromhydrique, à la température d'ébullition du milieu réactionnel.

Les dérivés de formule (XVI) pour lesquels Rq représente un radical alcoxy peuvent être obtenus par analogie avec les procédés précédemment mentionnés pour la préparation des composés de formule (I) et dans les exemples.

Les composés de formule (I) pour lesquels R₁ représente un radical carboxy peuvent être également préparés par hydrolyse d'un composé de formule (XVI) pour lequel R et R₂ ont les mêmes significations que dans la formule (I) et Rq représente un radical cyano.

Cette hydrolyse s'effectue généralement en présence d'un acide fort tel que l'acide chlorhydrique ou l'acide sulfurique, à une température voisine de 100°C, par adaptation des méthodes décrites par E. REITZ, Organic Synth., III, 851 ou R. ADAMS et coll., Organic Synth., I, 436.

Les dérivés de formule (XVI) pour lesquels Rq représente un radical cyano peuvent être obtenus par analogie avec les procédés généraux décrits précédemment pour la préparation des composés de formule (I).

Les composés de formule (I) pour lesquels R₁ représente un radical -NHCHO peuvent être également préparés par action d'un composé de formule (XVI) pour lequel R et R₂ ont les mêmes significations que dans la formule (I) et Rq représente un radical amino, sur H₃C-COOCHO.

Cette réaction s'effectue généralement au sein de l'acide formique, à une température voisine de 20°C éventuellement en présence d'acétate de sodium comme accepteur d'acide lorsque le composé engagé dans la réaction est sous forme salifiée.

Les composés de formule (I) pour lesquels R₁ représente un radical -N(alk)-CONR₈R₉ ou -NH-CSNR₈R₉ pour lesquels R₈ et R₉ ne sont pas des atomes d'hydrogène, -NH-CO-NR₉R₁₂ pour lequel R₉ et R₁₂ ne sont pas des atomes d'hydrogène, -NH-COR₁₀, -NH-SO₂-NR₇R₉ pour lequel R₇ est un radical alkyle ou -NH-SO₂-alk peuvent être également préparés par action d'un composé de formule (XVI) pour lequel R et R₂ ont les mêmes significations que dans la formule (I) et Rq représente un radical -NH-alk ou -NH₂ dans lequel alk a les mêmes significations que dans la formule (I) sur un dérivé de formule Hal-Rr dans laquelle Hal représente un atome d'halogène, Rr représente un radical -CONR₈R₉ ou -CSNR₈R₉ pour lesquels R₈ et R₉ ont les mêmes significations que dans la formule (I) à l'exception d'hydrogène, -CO-NR₉R₁₂ pour lequel R₉ et R₁₂ ont les mêmes significations que dans la formule (I) à l'exception d'hydrogène, -COR₁₀ dans lequel R₁₀ a les mêmes significations que dans la formule (I), -SO₂-NR₇R₉ dans lequel R₉ a les mêmes significations que dans la formule (I) et R₇ est un radical alkyle ou -SO₂-alk dans lequel alk a les mêmes significations que dans la formule (I).

Cette réaction s'effectue de préférence au sein d'un solvant inerte tel que le diméthylformamide, en présence d'un accepteur d'acide tel qu'une trialkyamine (triéthyamine par exemple), à une température comprise entre 20°C et la température d'ébullition du milieu réactionnel.

Les dérivés Hal-Rr pour lesquels Rr représente un radical -CONR₈R₉ ou -CONR₉R₁₂ sont commercialisés ou ceux pour lesquels Hal représente un atome de chlore peuvent être préparés par action de l'amine HNR₈R₉ ou HNR₉R₁₂ avec le phosgène par application ou adaptation de la méthode décrite par H. TILLES, J. Am. Chem. Soc., 81, 714 (1959).

Les amines HNR₈R₉ et HNR₉R₁₂ sont commercialisées ou peuvent être obtenues par réaction de l'amine primaire H₂NR₈ ou H₂NR₁₂ sur un dérivé HalR₉ dans lequel Hal représente un atome d'halogène (chlore, brome par exemple) et R₉ a les mêmes significations que dans la formule (I) à l'exception d'hydrogène. Cette réaction s'effectue généralement au sein d'un solvant inerte tel que le diméthylformamide, en présence d'un trialkylamine (triéthylamine par exemple), à une température voisine de 20°C.

Les amines primaires H₂NR₈ pour lesquelle R₈ représente un radical -alk-NR₉R₇, alk, R₇ et R₉ ayant les mêmes significations que dans la formule (I) à l'exception d'hydrogène, sont commercialisées ou peuvent être obtenues par action du sel de potassium du phtalimide ou de NaN(SiMe₃)₂ sur un dérivé Hal-alk-NR₉R₇ où Hal représente un atome d'halogène (chlore, brome par exemple), alk, R₇ et R₉ ayant les mêmes significations que dans la formule (I) à l'exception d'hydrogène puis hydrolyse en milieu acide (acide chlorhydrique ou bromhydrique par exemple. Ces réactions s'effectuent au sein d'un solvant inerte tel que le diméthylformamide en présence d'une base organique telle que la triéthylamine ou la pyridine à une température comprise entre 0°C et la température d'ébullition du milieu réactionnel. Les réactions d'hydrolyse s'effectuent au moyen d'une solution aqueuse d'acide (acide chlorhydrique par exemple), à une température comprise entre 20 et 100°C. Les dérivés Hal-alk-NR₇R₉ sont commercialisés ou peuvent être obtenus par action de Hal-alk-Hal et d'une amine HNR₉R₇, Hal représente un atome d'halogène (chlore, brome par exemple), alk, R₇ et R₉ ayant les mêmes significations que dans la formule (I) à l'exception d'hydrogène. Cette réaction s'effectue au sein d'un solvant inerte tel que le diméthylformamide, en présence d'une base organique telle que la triéthylamine ou la pyridine à une température comprise entre 0°C et la température d'ébullition du milieu réactionnel.

Les amines primaires H₂NR₁₂ pour lesquelles R₁₂ représente un radical -alk-CO₂R₂₁ sont commercialisées ou peuvent être obtenues par application ou adaptation des méthodes décrites par D.J.G. IVES et coll., J. Chem. Soc., 516 (1943) et dans le brevet DP 597305. De préférence, on fait réagir le cyanure de sodium ou de potassium sur un dérivé Hal-alk-CO₂R₂₁ où Hal représente un atome d'halogène (chlore, brome par exemple), alk et R₂₁ ont les mêmes significations que dans la formule (I) suivie d'une réaction de réduction.

Les amines primaires H₂NR₁₂ pour lesquelles R₁₂ représente un radical -alk-CO₂R₂₁ peuvent également être obtenues par réaction du sel de potassium du phtalimide ou de NaN(SiMe₃)₂ sur des dérivés Hal-alk-CO₂R₂₁ ou Hal représente un atome d'halogène (chlore, brome par exemple), alk et R₂₁ ont les mêmes significations que dans la formule (I) par adaptation ou application des méthodes décrites dans HOUBEN-WEYL, Band E16 d, TEIL2 page 665 (1992).

Les dérivés Hal-Rr pour lesquels Rr représente un radical -CS-NR₈R₉ peuvent être obtenus par application ou adaptation des méthodes décrites par E. LIEBER et coll., Can. J. Chem., 41, 1643 (1963) ou par U. HASSERODT, Chem. Ber., 101, 113 (1968).

Les dérivés Hal-CO-R₁₀ sont commercialisés ou peuvent être préparés à partir des acides carboxyliques correspondants par adaptation des méthodes décrites par B. HELFERICH et coll., Organic Synth., I, 147; R. ADAMS et coll., Organic Synth., I., 394 ou I. CASON, Organic Synth., III, 109.

Les dérivés de formule (XVI) pour lesquels Rq représente un radical -NH₂ ou -NH-alk peuvent être obtenus par analogie avec les procédés décrits précédemment pour la préparation des composés de formule (I).

Les composés de formule (I) pour lesquels R₁ représente un radical -NH-CO-NR₉R₁₂ ou -N(alk)-CO-NR₈R₉ peuvent également être préparés par action d'un dérivé de formule (XVI) pour lequel R et R₂ ont les mêmes significations que dans la formule (I) et Rq représente un radical -NH₂ ou -NH-alk pour lesquels alk a les mêmes significations que dans la formule (I) sur un halogénotrialkylsilane tel que le chlorotriméthylsilane en présence d'un hydrure de métal alcalin (hydrure de sodium par exemple), suivie de l'action d'un agent de couplage tel que le carbodiimidazole puis d'une amine de formule HN-R₉R₁₂ ou HN-R₈R₉ dans laquelle R₈, R₉ et R₁₂ ont les mêmes significations que dans la formule (I).

Cette réaction s'effectue de préférence au sein d'un solvant inerte tel que le diméthylformamide, à une température comprise entre 20 et 100°C.

Les composés de formule (I) pour lesquels R₁ représente un radical -NH-CO-NR₉R₁₂ ou -N(alk)-CO-NR₈R₉ peuvent également être préparés par action d'un dérivé de formule (XVI) pour lequel R et R₂ ont les mêmes significations que dans la formule (I) et Rq représente un radical -NH₂ ou -NH-alk pour lesquels alk a les mêmes significations que dans la formule (I) sur un dérivé de formule : 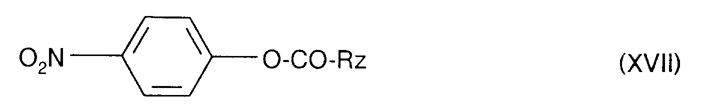 dans laquelle Rz représente un radical -NR₉R₁₂ ou -NR₈R₉, R₈, R₉ et R₁₂ ayant les mêmes significations que dans la formule (I).

Cette réaction s'effectue de préférence au sein d'un solvant inerte tel que le diméthylformamide, à une température comprise entre 20°C et la température d'ébullition du milieu réactionnel.

Les dérivés de formule (XVII) peuvent être obtenus par application ou adaptation de la méthode décrite par T. KONAKAHARA et coll., Synthesis, 103 (1993).

Les composés de formule (I) pour lesquels R₁ représente un radical -NH-CONR₉R₁₂, R₉ représente un atome d'hydrogène et R₁₂ représente un radical phényle substitué par un radical amino peuvent être également préparés par réduction d'un composé de formule (I) correspondant pour lequel R₁ représente un radical -NH-CONR₉R₁₂, R₉ représente un atome d'hydrogène et R₁₂ représente un radical phényle substitué par un radical nitro.

Cette réaction s'effectue par toute méthode connue de réduction d'une fonction nitro en fonction amino. De préférence, on opère au moyen de fer et d'acide chlorhydrique, au sein d'un solvant inerte tel que le diméthylformamide, un alcool (méthanol, éthanol par exemple) ou un mélange de ces solvants, à une température voisine de 80°C.

Les composés de formule (I) pour lesquels R₁ représente un radical -NH-CONR₉R₁₂, -N(alk)CONR₈R₉ ou -NHCSNR₈R₉ dans lesquels R₉ représente un atome d'hydrogène, R₈ représente un atome d'hydrogène ou un radical alkyle ou phényle et R₁₂ représente un atome d'hydrogène ou un radical alkyle, phénylalkyle éventuellement substitué, -alk-COOR₂₁ ou phényle substitué peuvent être également préparés par action d'un composé de formule (XVI) pour lequel R et R₂ ont les mêmes significations que dans la formule (I) et Rq représente un radical -NH₂ ou -NHalk, alk ayant les mêmes significations que dans la formule (I), sur un dérivé de formule Rs=C=N-Rt dans lequel Rt représente un radical -Si(CH₃)₃, alkyle, phénylalkyle dont le noyau phényle est éventuellement substitué par un ou plusieurs substituants choisis parmi les atomes d'halogène et les radicaux alcoxy et -COOR₂₁, -alk-COOR₂₁ ou phényle éventuellement substitué par un ou plusieurs substituants choisis parmi les atomes d'halogène et les radicaux alcoxy, nitro, amino, cyano et -COOR₂₁ dans lesquels alk et R₂₁ ont les mêmes significations que dans la formule (I) et Rs représente un atome d'oxygène ou de soufre.

Cette réaction s'effectue généralement au sein d'un solvant inerte tel que le diméthylformamide, éventuellement en présence d'une trialkylamine (triéthylamine par exemple), à une température comprise entre 20 et 100°C.

Les dérivés Rs=C=N-Rt peuvent être obtenus à partir des amines primaires correspondantes par action de phosgène ou de thiophosgène par application ou adaptation des méthodes décrites par R.L. SHRINER et coll., Organic Synth., II, 453 et G.M. DYON, Organic Synth., I, 165. Les amines primaires peuvent être préparées par exemple par réduction des amides primaires correspondants par adaptation des méthodes décrites par R.C. LAROCK, "Comprehensive Organic Transformations", Ed. VCH, page 432 (1989) qui consiste à utiliser comme agent de réduction soit BH₃, BH₃S(CH₃)₂, NaBH₄ ou AlLiH₄. Les amides primaires correspondants peuvent être obtenus à partir des acides carboxyliques correpondants par action de l'urée par adaptation de la méthode décrite par J.L. GUTHRIE et coll., Org. Syntheses, IV, 513.

Les dérivés Rs=C=N-Rt pour lesquels Rt est un radical phényle substitué par un ou plusieurs substituants choisis parmi les atomes d'halogène et les radicaux alcoxy, nitro, amino, -COOR₂₁ et cyano peuvent être obtenus par action de trichlorométhylchloroformiate en présence de l'aniline correspondante par adaptation de la méthode décrite par R. KATAKAI, J. Org. Chem., 50, 715 (1985). Les anilines correspondantes sont soit commercialisées soit obtenues par réduction des dérivés nitrés correspondants par adaptation des méthodes décrites par R.C. LAROCK, "Comprehensive Organic Tranformations", Ed. VCH, page 441 (1989) qui consiste généralement à réduire les dérivés nitrés à l'aide d'hydrogène, en présence d'un catalyseur tel que le nickel de Raney ou le palladium sur charbon; ou à partir des dérivés halogénés correspondants par adaptation des méthodes décrites par R.C. LAROCK, "Comprehensive Organic Transformations", Ed. VCH, page 399 (1989) qui consistent à préparer le magnésien du benzène considéré et de faire réagir par exemple NH₂Cl, NH₂OCH₃ ou N₃CH₂Si(CH₃)₃.

Les composés de formule (I) pour lesquels R₁ représente un radical -NH-CO-R₁₀ dans lequel R₁₀ représente un radical alcoxy peuvent également être préparés par action d'un dérivé de formule (XVI) dans laquelle R et R₂ ont les mêmes significations que dans la formule (I) et Rq représente un radical amino sur un dérivé Hal-COOalk pour lequel Hal représente un atome d'halogène et alk représente un radical alkyle et chauffage du dialcoxy obtenu éventuellement en présence d'une amine.

Cette réaction s'effectue généralement au sein d'un solvant inerte tel que le dioxanne ou le tétrahydrofuranne, en présence d'un hydrure de métal alcalin tel que l'hydrure de sodium, à une température comprise entre 20°C et la température d'ébullition du milieu réactionnel. Le dialcoxy est ensuite chauffé au sein du même solvant éventuellement en présence d'une base telle qu'une amine (triéthylamine, N-méthylpipérazine par exemple).

Les composés de formule (I) pour lesquels R₁ représente un radical -CO-NR₇R₉ peuvent être également préparés par action d'un composé de formule (I) correspondant pour lequel R₁ représente un radical carboxy ou un dérivé réactif de cet acide par toute méthode connue de l'homme du métier permettant de passer d'un acide à une amide. De préférence, on fait réagir ce composé sur une amine HNR₇R₉.

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel. Lorsque l'on met en oeuvre un ester, on opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les composés de formule (I) pour lesquels R₁ représente un radical -NR₉R₁₁ peuvent être également préparés par action d'un composé de formule (XVI) pour lequel R et R₂ ont les mêmes significations que dans la formule (I) et Rq représente un radical -NH₂ ou -NHalk dans lequel alk a les mêmes significations que dans la formule (I) sur un dérivé Hal-R₁₁ dans lequel Hal représente un atome d'halogène et R₁₁ a les mêmes significations que dans la formule (I).

Cette réaction s'effectue de préférence au sein d'un solvant inerte tel que le diméthylformamide, le diméthylsulfoxyde, en présence d'un hydrure de métal alcalin tel que l'hydrure de sodium, à une température comprise entre 20 et 100°C.

Les dérivés Hal-R₁₁ peuvent être obtenus par halogénation des hétérocycles correspondants par adaptation des méthodes décrites par H. M. GILOW et coll., J. Org. Chem., 2221 (1981); K.H.R. WOOLDRIDGE, Adv. Heterocycl. Chem., 14, 1 (1972).

Les composés de formule (I) pour lesquels R₁ représente un radical 2-oxo-1-imidazolidinyle peuvent également être préparés par cyclisation d'un dérivé de formule (XVI) pour lequel R et R₂ ont les mêmes significations que dans la formule (I) et Rq représente un radical -NH-CO-NH-(CH₂)ₙ-Hal dans lequel Hal représente un atome d'halogène, alk représente un radical alkyle et n est égal à 2.

Cette réaction s'effectue de préférence au sein d'un solvant inerte tel que le diméthlsulfoxyde, en présence d'un hydrure de métal alcalin tel que l'hydrure de sodium, à une température comprise entre 0°C et 60°C.

Les dérivés de formule (XVI) pour lesquels R et R₂ ont les mêmes significations que dans la formule (I) et Rq représente un radical -NH-CO-NH-(CH₂)ₙ-Hal dans lequel Hal représente un atome d'halogène et n est égal à 2 peuvent être préparés par analogie avec les procédés décrits précédemment pour les composés de formule (I) et dans les exemples.

Les composés de formule (I) pour lesquels R₁ représente un radical -NH-CO-NR₉R₁₂ ou -NH-COR₁₀ dans lesquels R₁₂ et R₁₀ représentent des radicaux -alk-COOR₂₁ et R₂₁ représente un atome d'hydrogène peuvent également être préparés par hydrolyse d'un composé de formule (I) correspondant pour lequel R₁ représente un radical -NH-CO-NR₉R₁₂ ou -NH-COR₁₀ dans lesquels R₁₂ et R₁₀ représentent des radicaux -alk-COOR₂₁ et R₂₁ représente un radical alkyle.

Cette hydrolyse s'effectue au moyen d'une base telle qu'un hydroxyde de métal alcalin (soude, potasse par exemple), au sein d'un solvant inerte tel qu'un alcool (éthanol, méthanol par exemple), à une température comprise entre 20 et 60°C.

Les composés de formule (I) pour lesquels R₁ représentent un radical -NR₉R₁₁, R₉ représente un atome d'hydrogène, R₁₁ représente un radical imidazolinyle-2 peuvent également être préparés par cyclisation d'un dérivé de formule (XVI) pour lequel R et R₂ ont les mêmes significations que dans la formule (I) et Rq représente un radical -N=C(SCH₃)-NH-(CH₂)₂-NH₂.

Cette réaction s'effectue généralement au sein d'un solvant inerte tel que'un alcool aliphatique inférieur (méthanol ou éthanol par exemple), en présence d'un alcoolate de métal alcalin tel que le méthylate de sodium, à une température voisine de 20°C.

Les dérivés de formule (XVI) pour lesquels R et R₂ ont les mêmes significations que dans la formule (I) et Rq représente un radical -N=C(SCH₃)-NH-(CH₂)₂-NH₂ peuvent être obtenus par application ou adaptation des procédés décrits par A. KATSUHIKO et coll., J. Org. Chem., 57, 417 (1992).

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane. Comme exemples de groupes protecteurs de la fonction hydroxy, on peut citer les triéthylsilyle, benzyle. Comme groupes protecteurs des fonctions carboxy, on peut citer les esters (méthoxyméthylester, tétrahydropyranylester, benzylester par exemple), les oxazoles et les 2-alkyl-1,3-oxazolines. D'autres groupes protecteurs utilisables sont décrits par W. GREENE et coll., Protecting Groups in Organic Synthesis, second edition, 1991, Jonh Wiley & Sons.

Les composés de formule (I) peuvent être purifiés par les méthodes connues habituelles, par exemple par cristallisation, chromatographie ou extraction.

Les énantiomères des composés de formule (I) pour lesquels R représente un radical CH-R₆ peuvent être obtenus par dédoublement des racémiques par exemple par chromatographie sur colonne chirale selon W.H. PIRCKLE et coll., asymetric synthesis, vol. 1, Academic Press (1983) ou par synthèse à partir des précurseurs chiraux.

Les diastéréoisomères des composés de formule (I) pour lesquels R représente un radical CH-R₆ comportant un ou plusieurs carbones chiraux et les différents isomères E et Z des composés de formule (I) peuvent être séparés par les méthodes connues habituelles, par exemple par cristallisation ou chromatographie.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, ariginine, leucine, lysine, N-méthyl glucamine).

Les composés de formule (I) présentent des propriétés pharmacologiques intéressantes. Ces composés sont des antagonistes du récepteur de l'acide α-amino-3-hydroxy-5-méthyl-4-isoxazolepropionique (AMPA), connu aussi sous le nom de récepteur du quisqualate.

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux, un arrêt cardiaque, une hypotension artérielle, une intervention chirurgicale cardiaque ou pulmonaire ou une hypoglycémie sévére. Ils sont également utiles dans le traitement des effets dus à une anoxie, qu'elle soit périnatale ou consécutive à une noyade ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, en tant qu'analgésiques (DICKENSON et coll., Neurosc. Letters, 121, 263 (1991)), antiinflammatoire (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA, ainsi que les troubles neurologiques associés aux maladies virales telles que le sida (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention des symptômes d'abstinence aux drogues et à l'alcool et de l'inhibition de l'accoutumance et de la dépendance aux opiacés. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est inférieure ou égale à 100 µM.
L'affinité des composés de formule (I) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est inférieure ou égale à 100 µM.

Les composés de formule (I) présentent une toxicité faible. Leur DL50 est supérieure à 50 mg/kg par voie IP chez la souris.

Sont particulièrement intéressants les composés suivants :
- 9-phénylacétamido-5H,10H-imidazo[1,2-a]indéno[1,2-e]pyrazine-4-one,
- 8-éthoxycarbonylamino-5H,10H-imidazo[1,2-a]indéno[1,2-e]pyrazine-4-one,
- 8-[3-(3-cyanophényl)uréido]-5H,10H-imidazo[1,2-a]indéno[1,2-e]pyrazine-4-one,
- 8-[3-(3-méthoxyphényl)uréido]-5H,10H-imidazo[1,2-a]indéno[1,2-e]pyrazine-4-one,
- 8-phénylacétamido-5H,10H-imidazo[1,2-a]indéno[1,2-e]pyrazine-4-one,
- 8-(3-phénéthyluréido)-5H,10H-imidazo[1,2-a]indéno[1,2-e]pyrazine-4-one,
- 8-(3-méthyluréido)-5H,10H-imidazo[1,2-a]indéno[1,2-e]pyrazine-4-one,
- 8-(3-benzyluréido)-5H,10H-imidazo[1,2-a]indéno[1,2-e]pyrazine-4-one,
- 8-(3-tert-butyluréido)-5H,10H-imidazo[1,2-a]indéno[1,2-e]pyrazine-4-one,
- 8-(3-phénylpropionamido)-5H,10H-imidazo[1,2-a]indéno[1,2-e]pyrazine-4-one,
- 8-benzamido-5H,10H-imidazo[1,2-a]indéno[1,2-e]pyrazine-4-one,
- 8-(4-phénylbutyrylamino-5H,10H-imidazo[1,2-a]indéno[1,2-e]pyrazine-4-one,
- 8-(5-phénylvalérylamino-5H,10H-imidazo[1,2-a]indéno[1,2-e]pyrazine-4-one,
- 8-(3-éthoxycarbonylméthyluréido)-5H,10H-imidazo[1,2-a]indéno[1,2-e]pyrazine-4-one,
- 8-(3-carboxyméthyluréido)-5H,10H-imidazo[1,2-a]indéno[1,2-e]pyrazine-4-one,
- 8-(3,3-diméthyluréido)-5H,10H-imidazo[1,2-a]indéno[1,2-e]pyrazine-4-one,
- 8-hydroxy-5H,10H-imidazo[1,2-a]indéno[1,2-e]pyrazine-4-one,
- 8-(3-aminopropionamido-5H,10H-imidazo[1,2-a]indéno[1,2-e]pyrazine-4-one,
- 8-aminoacétamido-5H,10H-imidazo[1,2-a]indéno[1,2-e]pyrazine-4-one,
- 8-[3-(3-nitrophényl)uréido]-5H,10H-imidazo[1,2-a]indéno[1,2-e]pyrazine-4-one,
- 8-[3-(2-méthoxyphényl)uréido]-5H,10H-imidazo[1,2-a]indéno[1,2-e]pyrazine-4-one,
- 8-[3-(2-nitrophenyl)ureido]-5H,10H-imidazo[1,2-a]indéno[1,2-e]pyrazine-4-one,
- 8-[3-(4-aminophényl)uréido]-5H,10H-imidazo[1,2-a]indéno[1,2-e]pyrazine-4-one,
- 8-[3-(4-méthoxyphényl)uréido]-5H,10H-imidazo[1,2-a]indéno[1,2-e]pyrazine-4-one,
- 8-(4-méthylpentanoyl)amino-5H,10H-imidazo[1,2-a]indéno[1,2-e]pyrazine-4-one,
- N,N-diméthyl-4-oxo-4,5-dihydro-10H-imidazo[1,2-a]indéno[1,2-e]pyrazine-8-sulfonamide,
- 8-(3-phénylthiouréido)-5H,10H-imidazo[1,2-a]indéno[1,2-e]pyrazine-4-one,
- 8-(3-méthylthiouréido)-5H,10H-imidazo[1,2-a]indéno[1,2-e]pyrazine-4-one,
- 8-(2-oxo-1-imidazolinyl)-5H,10H-imidazo[1,2-a]indéno[1,2-e]pyrazine-4-one,
- 8-formamido-5H,10H-imidazo[1,2-a]indéno[1,2-e]pyrazine-4-one,
- 8-(3-éthoxycarbonylpropionylamino)-5H,10H-imidazo[1,2-a]indéno[1,2-e] pyrazine-4-one,
- 8-[3-(2-éthoxycarbonyléthyl)uréido]-5H,10H-imidazo[1,2-a]indéno[1,2-e]pyrazine-4-one,
- 8-[3-(2-carboxyéthyl)uréido]-5H,10H-imidazo[1,2-a]indéno[1,2-e]pyrazine-4-one,
- 8-[3-(4-fluorophényl)uréido]-5H,10H-imidazo[1,2-a]indéno[1,2-e]pyrazine-4-one,
- 8-[3-(3-fluorophényl)uréido]-5H,10H-imidazo[1,2-a]indéno[1,2-e]pyrazine-4-one,
- 8-[3-(2-fluorophényl)uréido]-5H,10H-imidazo[1,2-a]indéno[1,2-e]pyrazine-4-one,
- 8-(3-éthyluréido)-5H,10H-imidazo[1,2-a]indéno[1,2-e]pyrazine-4-one,
- 8-[3-morpholino-uréido]-5H,10H-imidazo[1,2-a]indéno[1,2-e]pyrazine-4-one,
- 10-amino-8-(3-méthyluréido)-5H,10H-imidazo[1,2-a]indéno[1,2-e]pyrazine-4-one,
- 10-hydroxyimino-8-(3-méthyluréido)-5H,10H-imidazo[1,2-a]indéno[1,2-e]pyrazine-4-one,
- 8-(3-méthyluréido)-5H-imidazo[1,2-a]indéno[1,2-e]pyrazine-4,10-dione,
- 8-[3-(3-aminophényl)uréido]-5H,10H-imidazo[1,2-a]indéno[1,2-e]pyrazine-4-one,
- 8-[(1-azétidinyl)carbonylamino]-5H,10H-imidazo[1,2-a]indéno[1,2-e]pyrazine-4-one,
- 8-(3-propyluréido)-5H,10H-imidazo[1,2-a]indéno[1,2-e]pyrazine-4-one,
- 8-(3-isopropyluréido)-5H,10H-imidazo[1,2-a]indéno[1,2-e]pyrazine-4-one,
- 8-(3-butyluréido)-5H,10H-imidazo[1,2-a]indéno[1,2-e]pyrazine-4-one,
- 8-[(2-thiazolin-2-yl)amino]-5H,10H-imidazo[1,2-a]indéno[1,2-e]pyrazine-4-one,
- 8-[3-(3-carbométhoxyphényl)uréido]-5H,10H-imidazo[1,2-a]indéno[1,2-e]pyrazine-4-one,
- 8-[3-(4-carbométhoxybenzyl)uréido]-5H,10H-imidazo[1,2-a]indéno[1,2-e]pyrazine-4-one,
- 8-[3-(4-carboxybenzyl)uréido]-5H,10H-imidazo[1,2-a]indéno[1,2-e]pyrazine-4-one,
- 8-[3-(2-fluorobenzyl)uréido]-5H,10H-imidazo[1,2-a]indéno[1,2-e]pyrazine-4-one,
- 8-[3-(3-fluorobenzyl)uréido]-5H,10H-imidazo[1,2-a]indéno[1,2-e]pyrazine-4-one,
- 8-[3-(4-fluorobenzyl)uréido]-5H,10H-imidazo[1,2-a]indéno[1,2-e]pyrazine-4-one,
- 9-(N-éthylcarboxamido)-5H,10H-imidazo[1,2-a]indéno[1,2-e]pyrazine-4-one,
- 9-(3-méthyluréido)-5H,10H-imidazo[1,2-a]indéno[1,2-e]pyrazine-4-one,
- 8-[3-(4-méthoxybenzyl)-uréido]-5H,10H-imidazo[1,2-a]indéno[1,2-e]pyrazine-4-one,
   et leurs sels.

Les exemples suivants illustrent l'invention.

### EXEMPLE 1

A une suspension de 0,63 g de chlorhydrate de 9-amino-5H,10H-imidazo[1,2-a]indéno[1,2-e]pyrazine-4-one dans 25 ml de diméthylformamide, on ajoute goutte à goutte 0,84 ml de triéthylamine puis 0,61 ml de chlorure de phénylacétyle; la température du milieu réactionnel s'élève alors à 30°C. La suspension est portée à reflux pendant 2 heures (dissolution totale). Après refroidissement à une température voisine de 20°C, l'insoluble est éliminé et le filtrat concentré à sec sous pression réduite (15 mm Hg; 2 kPa). L'huile ainsi obtenue est reprise dans un mélange d'acétate d'éthyle et d'eau distillée. Le solide formé est filtré, lavé avec 50 ml d'eau bouillante, puis 50 ml d'acétone. 0,40 g d'hydrate de 9-phénylacétamido-5H,10H-imidazo[1,2-a]indéno[1,2-e]pyrazine-4-one sont ainsi obtenus sous forme de solide grisverdâtre se décomposant vers 215°C (Analyse C21H18N4O3; 0,3H2O % calculé C : 67,37; H : 4,85; N : 14,96; % trouvé C : 67,2; H :4,6; N : 15,2).

Le chlorhydrate de 9-amino-5H,10H-imidazo[1,2-a]indéno[1,2-e]pyrazine-4-one peut être préparé de la manière suivante : 0,84 g de 9-acétamido-5H,10H-imidazo[1,2-a]indéno[1,2-e]pyrazine-4-one dans 6 ml d'acide chlorhydrique 6N sont chauffés à reflux jusqu'à solubilisation complète. Après refroidissement à une température voisine de 20°C, le précipité formé est filtré, lavé à l'acétate d'éthyle puis séché sous vide partiel (1 mm Hg; 0,13 kPa) à 45°C. On obtient ainsi 0,79 g de produit attendu sous forme de poudre beige dont le point de fusion est supérieur à 260°C.

La 9-acétamido-5H,10H-imidazo[1,2-a]indéno[1,2-e]pyrazine-4-one peut être obtenue de la manière suivante : 1 g de 4-acétamido-2-(2-éthoxycarbonyl-1H-imidazol-1-yl)-indanone et 26 g d'acétate d'ammonium dans 33 ml d'acide acétique sont chauffés à reflux pendant 3 heures. Après refroidissement à une température voisine de 20°C, le précipité formé est filtré, lavé à l'eau et séché sous pression réduite (1 mm Hg; 0,13 kPa) à 45°C. On obtient ainsi 0,69 g de produit attendu sous forme de poudre brune dont le point de fusion est supérieur à 260°C.

La 4-acétamido-2-(2-éthoxycarbonyl-1H-imidazol-1-yl)-indanone peut être préparée selon le protocole suivant : 14,87 g de 1H-imidazole-2-carboxylate d'éthyle et 14,14 g de 4-acétamido-2-bromo-indanone (pure à 65%) sont chauffés à 110°C pendant 15 minutes. Après refroidissement à une température voisine de 20°C, le produit brut est purifié par flash-chromatographie sur colonne de silice en utilisant de l'acétate d'éthyle puis un mélange d'acétate d'éthyle et de méthanol (90/10 en volumes) comme éluants. 4,34 g de produit attendu sont ainsi isolés sous forme de solide gris-verdâtre fondant à 160°C.

La 4-acétamido-2-bromo-indanone peut être obtenue de la façon suivante : à 9,92 g de 4-acétamido-indanone et 1,6 ml d'acide bromhydrique concentré en solution dans 110 ml d'acide acétique, on ajoute goutte à goutte en environ 1 heure, 1,6 ml de brome en solution dans 5 ml d'acide acétique. Après 2 heures d'agitation à une température voisine de 20°C, on ajoute à nouveau 1,6 ml de brome en solution dans 5 ml d'acide acétique. La réaction est poursuivie 1 heure, puis le milieu réactionnel versé dans de l'eau glacée. La phase organique est extraite par de l'acétate d'éthyle, lavée à l'eau et séchée pour conduire à 13,44 g de produit attendu sous forme de solide beige (pur à 65%) utilisé tel quel dans les synthèses intérieures.

La 4-acétamido-indanone peut être préparée selon le procédé suivant : à 17,5 g de 4-amino-indanone en solution dans 120 ml de tétrahydrofuranne à une température voisine de 10°C, on ajoute 16,7 ml de triéthylamine puis 8,5 ml de chlorure d'acétyle. La réaction est poursuivie 15 heures à une température voisine de 20°C. Le milieu réactionnel est concentré à sec sous pression réduite (15 mm Hg; 2 kPa) et le résidu obtenu repris dans un mélange d'acétate d'éthyle et d'eau distillée. La phase organique est lavée à l'eau, séchée et concentrée à sec sous pression réduite. Après purification par flash-chromatographie sur colonne de silice en utilisant de l'acétate d'éthyle comme éluant, on isole 10,84 g de produit attendu sous forme de poudre beige fondant à 145°C.

La 4-amino-indanone peut être obtenue selon la méthode décrite par V. Hach et M. Protiva, Collect. Czech. Chem. Commun.,23, 1902 (1958).

### EXEMPLE 2

A 0,5 g de 8-éthoxycarbonylamino-4-éthoxycarbonyloxy-10H-imidazo[1,2-a]indéno[1,2-e]pyrazine en solution dans 10 ml de tétrahydrofuranne à une température voisine de 20°C, on ajoute goutte à goutte 0,36 ml de N-méthyl-pipérazine. La réaction est poursuivie 30 minutes à la même température, puis le milieu réactionnel est concentré à sec sous pression réduite (15 mm Hg; 2 kPa). Le résidu est repris dans un mélange de dichlorométhane et de méthanol, le précipité formé filtré, lavé avec 10 ml de méthanol et séché sous vide partiel (1 mm Hg; 0,13 kPa) à 40°C. On obtient ainsi 0,21 g de 8-éthoxycarbonylamino-5H,10H-imidazo[1,2-a]indéno[1,2-e]pyrazine-4-one sous forme de poudre rougeâtre fondant à 260°C (Analyse C16H14N4O3; 0,6H2O % calculé C : 61,92; H : 4,55; N : 18,06; % trouvé C : 61,9; H : 4,5; N : 18,3).

La 8-éthoxycarbonylamino-4-éthoxycarbonyloxy-10H-imidazo[1,2-a]indéno [1,2-e]pyrazine peut être préparée de la manière suivante : à une suspension de 3,5 g de 8-amino-5H,10H-imidazo[1,2-a]indéno[1,2-e] pyrazine-4-one dans 200 ml de dioxanne, on ajoute progressivement 1,22 g d'hydrure de sodium à 60%. Le milieu réactionnel est alors chauffé à 55°C pendant 2 heures. Après refroidissement à une température voisine de 20°C, 4,2 ml de chloroformiate d'éthyle sont ajoutés rapidement au milieu réactionnel et la réaction poursuivie 2 heures à une température voisine de 20°C. L'hydrolyse conduit à un solide qui est filtré; le filtrat est concentré à sec sous pression réduite (15 mm Hg; 2 kPa) et le résidu repris dans l'eau, filtré et séché. La purification par flash-chromatographie sur colonne de silice, en utilisant un mélange de dichlorométhane et de méthanol (95/5 en volumes) comme éluant, conduit à 2 g de produit attendu sous forme de solide rose pâle fondant à 169°C après recristallisation dans l'éthanol absolu (Analyse C19H18N4O5 % calculé C : 59,69; H : 4,74; N : 14,65; % trouvé C : 59,9; H :5,1; N :14,8).

La 8-amino-5H,10H-imidazo[1,2-a]indéno[1,2-e]pyrazine-4-one peut être préparée de la manière suivante : un mélange de 9,7 g de 5H,10H-8-nitroimidazo[1,2-a]indéno[1,2-e]pyrazine-4-one, de 370 ml de soude aqueuse 0,1 N et de 0,3 g de charbon palladié à 10 % est hydrogéné à une température voisine de 20°C sous une pression de 1,2 bar pendant 23 heures. La suspension est acidifiée par 80 ml d'acide chlorhydrique 1 N, puis elle est filtrée. On reprend le solide obtenu dans 600 ml d'eau bouillante. Le mélange est additionné de noir animal et filtré à chaud sur cellite. Le filtrat cristallise après refroidissement dans un bain de glace. Les cristaux sont séparés par filtration et lavés deux fois avec 50 ml d'éther éthylique. On obtient ainsi 4,7 g de monochlorhydrate de 5H,10H-8-amino-imidazo[1,2-a]indéno[1,2-e]pyrazine-4-one sous forme d'un solide beige fondant au-dessus de 260°C (Spectre de R.M.N. : (300 MHz; DMSO d6; δ en ppm) : 4,02 (s, 2H : -CH2- en 10); 7,00 (d large, J =8 Hz, 1H : -H7); 7,20 (s large, 1H : -H9); 7,74 (d, J = 8 Hz, 1H : -H6); 7,77 et 8,07 (2s larges, 1H chacun : -H de l'imidazole); 12,65 (mf, 1H : -CO-NH-)).

La 5H,10H-8-nitro-imidazo[1,2-a]indéno[1,2-e]pyrazine-4-one peut être préparée de la manière suivante : 1 g de nitrate de potassium est ajouté en 10 minutes à une température voisine de 5°C à une solution de 2,6 g de chlorhydrate de 5H,10H-imidazo[1,2-a]indéno[1,2-e]pyrazine-4-one dans 20 ml d'acide sulfurique concentré (d = 1,83). Le mélange est agité pendant 30 minutes à la même température et pendant 3 heures à 25°C puis est versé sur 150 ml d'eau glacée. Les cristaux apparus sont séparés par filtration, lavés avec de l'eau distillée puis avec de l'acétone et séchés sous pression réduite (1 mm Hg; 0,13 kPa) à 80°C. On obtient ainsi 2,1 g de 8-nitro-5H,10H-imidazo[1,2-a]indéno[1,2-e]pyrazine-4-one se décomposant sans fondre au-dessus de 300°C (Spectre de R.M.N. : (200 MHz; DMSO d6; δ en ppm) : 4,23 (s, 2H : -CH2- en 10); 7,68 et de 8,12 (2s larges, 1H chacun : -H de l'imidazole); 8,07 (dd, J =8,5 Hz, 1H : -H6); 8,38 (dd, J =8,5 et 1,5 Hz, 1H : -H7); 8,50 (d, J =1,5 Hz, 1H : -H9); 12,64 (mf, 1H : -CONH-)).

La 5H,10H-imidazo[1,2-a]indéno[1,2-e]pyrazine-4-one peut être préparée de la manière suivante : une solution de 4,8 g de bromure de 3-méthyl-4-oxo-5H,10H-imidazo[1,2-a]indéno[1,2-e]pyrazinium dans 30 g d'imidazole est chauffée pendant 24 heures à 160°C, refroidie à 100°C puis versée sur un mélange agité de 75 g de glace et de 75 g d'eau distillée. L'insoluble est filtré, lavé 2 fois avec 20 ml au total d'eau distillée puis séché sous pression réduite (10 mm Hg; 1,3 kPa) à 50°C. Le produit ainsi obtenu (4 g) est dissous dans 80 ml de diméthylformamide et la solution additionnée de 20 g de silice est concentrée à sec sous pression réduite (15 mm Hg, 2 kPa) à 100°C. Le mélange est introduit dans une colonne de 4,2 cm de diamètre contenant 240 g de silice puis est élué par un mélange dichlorométhaneméthanol (97-3 en volumes) en recueillant des fractions de 60 ml . Les fractions 10 à 70 sont réunies, additionnées de 1,5 g de noir décolorant, filtrées et concentrées à sec sous pression réduite (15 mm Hg; 2 kPa) à 55°C. Le produit obtenu (1,7 g) est dissous dans 350 ml de méthanol bouillant et la solution additionnée de 0,1 g de noir décolorant est filtrée à chaud, concentrée sous pression réduite (15 mm Hg; 2 kPa) à 40°C pour ramener son volume à environ 30 ml puis conservée à 5°C pendant 60 heures. Les cristaux sont séparés par filtration, lavés 2 fois avec 20 ml au total de méthanol glacé et séchés sous pression réduite (1 mm Hg; 0,13 kPa) à 60°C. On obtient ainsi 1,1 g de 5H,10H-imidazo[1,2-a]indéno[1,2-e]pyrazine-4-one se décomposant sans fondre à 350°C [Rf= 0,77, chromatographie sur couche mince de gel de silice, solvant : dichlorométhane-méthanol (8-2 en volumes)].

Le bromure de 3-méthyl-4-oxo-5H,10H-imidazo[1,2-a]indéno[1,2-e] pyrazinium peut être préparé de la manière suivante : une solution de 5 g de 1-méthyl-1H-imidazole-2-carboxamide et de 12 g de 2-bromoindanone à 85% dans 100 ml de diméthylformamide anhydre est agitée pendant 28 heures à 115°C puis refroidie à une température voisine de 20°C. L'insoluble est séparé par filtration, lavé 2 fois avec 20 ml au total de diméthylformamide glacé et séché sous pression réduite (10 mm Hg; 1,3 kPa). On obtient ainsi 4,8 g de bromure de 3-méthyl-4-oxo-5H,10H-imidazo[1,2-a]indéno[1,2-e]pyrazinium (Spectre de R.M.N. : (200 MHz; DMSO d6; δ en ppm) : 4,13 (s, 2H : -CH2 en 10); 4,34 (s, 3H : N⁺ -CH3); 7,47 (mt, 2H :- H7 et -H8); 7,68 et 7,96 (2d, J =7,5 Hz, 1H chacun : -H6 et -H9); 8,32 et 8,45 (2d, J = 1 Hz, 1H chacun : H de l'imidazole); 13,60 (mf, 1H : NH)).

Le 1-méthyl-1H-imidazole-2-carboxamide peut être préparée selon le procédé décrit par D.D. DAVEY, J. Org. Chem., 52, 4379 (1987).

### EXEMPLE 3

A une suspension de 2 g de chlorhydrate de 8-amino-5H,10H-imidazo[1,2-a]indéno[1,2-e]pyrazine-4-one dans 30 ml de diméthylformamide, on ajoute goutte à goutte 2 ml de triéthylamine, puis 2,1 g de 3-cyanophénylisocyanate en solution dans 10 ml de diméthylformamide. Le milieu réactionnel est chauffé 2 heures à 60°C puis laissé la nuit à une température voisine de 20°C. Le précipité formé est filtré, lavé avec de l'eau distillée puis avec de l'oxyde d'isopropyle et séché sous pression réduite (1 mm Hg; 0,13 kPa) à 40°C. On obtient ainsi 1,2 g d'hydrate de 8-[3-(3-cyanophényl)uréido]-5H,10H-imidazo[1,2-a]indéno[1,2-e]pyrazine-4-one sous forme de solide beige dont le point de fusion est supérieur à 260°C (Analyse C21H14N6O2; 0,4H2O % calculé C : 63,00; H : 4,03; N : 20,99; % trouvé C : 63,1; H : 4,4; N : 21,3).

### EXEMPLE 4

A une suspension de 2 g de chlorhydrate de 8-amino-5H,10H-imidazo[1,2-a]indéno[1,2-e]pyrazine-4-one dans 30 ml de diméthylformamide, on ajoute goutte à goutte 2 ml de triéthylamine, puis 2,16 g de 3-méthoxyphénylisocyanate en solution dans 10 ml de diméthylformamide. La réaction est poursuivie 5 heures à une température voisine de 20°C. Le précipité formé est filtré, lavé à l'eau puis avec de l'oxyde d'isopropyle et séché sous pression réduite (1 mm Hg; 0,13 kPa) à 40°C. On obtient 1,4 g de 8-[3-(3-méthoxyphényl)uréido]-5H,10H-imidazo[1,2-a]indéno[1,2-e]pyrazine-4-one sous forme de solide beige dont le point de fusion est supérieur à 260°C (Analyse C21H17N5O3; 0,69H2O % calculé C : 65,11; H : 4,42; N : 18,08; % trouvé C : 64,8; H : 4,4; N : 17,8).

### EXEMPLE 5

A une suspension de 1 g de chlorhydrate de 8-amino-5H,10H-imidazo[1,2-a]indéno[1,2-e]pyrazine-4-one dans 45 ml de diméthylformamide, on ajoute 1,6 ml de triéthylamine puis après dissolution 1 ml de chlorure de phénylacétyle. Le milieu réactionnel est porté à reflux pendant 6 heures. Après refroidissement à une température voisine de 20°C, le précipité formé est filtré puis recristallisé dans 30 ml de diméthylformamide. Le solide ainsi obtenu est filtré, lavé à l'eau puis avec de l'éther éthylique et séché sous vide partiel (1 mm Hg; 0,13 kPa) à 80°C. On obtient 0,9 g de 8-phénylacétamido-5H,10H-imidazo[1,2-a]indéno[1,2-e]pyrazine-4-one sous forme de solide jaune dont le point de fusion est supérieur est 260°C (Analyse C21H16N4O2; 1,6H2O; 0,4DMF % calculé C : 70,78; H : 4,53; N : 15,72; % trouvé C : 70,8; H : 4,2; N : 16,0).

### EXEMPLE 6

A 1,5 g de chlorhydrate de 8-amino-5H,10H-imidazo[1,2-a]indéno [1,2-e]pyrazine-4-one en suspension dans 50 ml de diméthylsulfoxyde, on ajoute goutte à goutte 2,3 ml de triéthylamine suivis d'une solution de 1,61 g de phénéthylisocyanate dans 10 ml de diméthylsulfoxyde. La réaction est poursuivie 15 heures à une température voisine de 20°C. Le précipité formé est filtré, lavé à l'eau et recristallisé dans 5 ml de diméthylformamide. Après filtration, lavage à l'eau puis à l'acétone et séchage sous pression réduite (1 mm Hg; 0,13 kPa) à 80°C, on obtient 0,23 g de 8-(3-phénéthyluréido)-5H,10H-imidazo[1,2-a]indéno[1,2-e]pyrazine-4-one sous forme de solide jaune dont le point de fusion est supérieur à 260°C (Analyse C21H16N4O2; 0,78H2O; 0,25DMF; 0,25DMSO % calculé C : 68,56; H : 4,97; N : 18,17; % trouvé C : 68,3; H : 4,6; N : 18,6).

### EXEMPLE 7

A 1,5 g de chlorhydrate de 8-amino-5H,10H-imidazo[1,2-a]indéno[1,2-e]pyrazine-4-one en suspension dans 50 ml de diméthylformamide, on ajoute 2,3 ml de triéthylamine, puis après dissolution, 0,65 ml de méthylisocyanate en solution dans 10 ml de diméthylformamide. La réaction est poursuivie 15 heures à une température voisine de 20°C. On ajoute à nouveau 0,65 ml de méthylisocyanate en solution dans 10 ml de diméthylformamide et on maintient l'agitation 4 heures supplémentaires à la même température. L'insoluble est alors filtré, lavé à l'eau et recristallisé dans le diméthylformamide puis dans le diméthylsulfoxyde. Après lavage à l'eau et à l'acétone, le produit est séché sous pression réduite (1 mm Hg; 0,13 kPa) à 100°C. On obtient ainsi 0,3 g de 8-(3-méthyluréido)-5H,10H-imidazo[1,2-a]indéno[1,2-e]pyrazine-4-one sous forme de solide beige dont le point de fusion est supérieur à 260°C (Analyse C15H13N5O2; 1,26H2O % calculé C : 61,01; H : 4,44; N : 23,72; % trouvé C : 61,4; H : 4,8; N : 24,1).

### EXEMPLE 8

On opère comme dans l'exemple 7, à partir de 1,5 g de chlorhydrate de 8-amino-5H,10H-imidazo[1,2-a]indéno[1,2-e]pyrazine-4-one, 2,3 ml de triéthylamine, 2 fois 1,25 ml de benzylisocyanate et 60 ml de diméthylformamide. L'insoluble ainsi formé est filtré puis recristallisé dans le diméthylformamide. Après filtration, lavage abondant à l'eau et à l'acétone, et séchage sous vide, on obtient 0,74 g de 8-(3-benzyluréido)-5H,10H-imidazo[1,2-a]indéno[1,2-e]pyrazine-4-one sous forme de solide écru se décomposant à 230°C (Analyse C21H17N5O2; 1,55H2O % calculé C : 67,91; H : 4,61; N : 18,86; % trouvé C : 67,8; H : 4,5; N : 18,9).

### EXEMPLE 9

On opère comme dans l'exemple 7, à partir de 1,2 g de chlorhydrate de 8-amino-5H,10H-imidazo[1,2-a]indéno[1,2-e]pyrazine-4-one, 1,8 ml de triéthylamine, 2 fois 0,6 ml de tert-butylisocyanate et 25 ml de diméthylformamide. Après addition de 100 ml d'eau distillée au milieu réactionnel, le précipité formé est filtré et recristallisé dans le diméthylformamide. Le solide ainsi obtenu est lavé à l'eau puis avec de l'éther éthylique et séché sous pression réduite (1 mm Hg; 0,13 kPa) à 80°C. On obtient 0,31 g de 8-(3-tert-butyluréido)-5H,10H-imidazo[1,2-a]indéno[1,2-e]pyrazine-4-one sous forme de solide brun dont le point de fusion est supérieur à 260°C (Analyse C18H19N5O2; 0,83H2O; 0,03DMF % calculé C : 64,08; H : 5,68; N : 20,76; % trouvé C : 63,7; H : 5,9; N : 20,6).

### EXEMPLE 10

A 1 g de 8-amino-5H,10H-imidazo[1,2-a]indéno[1,2-e]pyrazine-4-one solubilisé dans 75 ml de diméthylformamide sont additionnés goutte à goutte 1,25 ml de chlorure d'hydrocinnamoyle à une température voisine de 20°C. Le milieu réactionnel est porté 6 heures à reflux. Après refroidissement à une température voisine de 20°C, le précipité formé est filtré et recristallisé dans le diméthylformamide. Le solide ainsi obtenu est filtré, lavé à l'eau puis à l'éther éthylique et séché sous vide partiel (1 mm Hg; 0,13 kPa) à 80°C. 0,69 g de 8-(3-phénylpropionamido)-5H,10H-imidazo[1,2-a]indéno[1,2-e]pyrazine-4-one sous forme de solide jaune se décomposant à 200°C (Analyse C22H18N4O2; 0,93H2O; 0,15DMF % calculé C : 71,34; H : 4,90; N : 15,13; % trouvé C : 71,0; H : 5,0; N : 15,2).

### EXEMPLE 11

On opère comme dans l'exemple 10, à partir de 1 g de 8-amino-5H,10H-imidazo[1,2-a]indéno[1,2-e]pyrazine-4-one et de 1 ml de chlorure de benzoyle dans 75 ml de diméthylformamide. La réaction est poursuivie 8 heures à reflux. La suspension est filtrée et le filtrat concentré à sec sous pression réduite (15 mm Hg; 2 kPa). Le résidu est repris dans le diméthylformamide à chaud et la solution abandonnée une nuit à 0°C. Le précipité formé est filtré, lavé à l'eau et séché sous vide (1 mm Hg; 0,13 kPa) à 80°C. On obtient ainsi 0,28 g de 8-benzamido-5H,10H-imidazo[1,2-a]indéno[1,2-e]pyrazine-4-one sous forme de solide rose dont le point de fusion est supérieur à 260°C (Analyse C20H14N4O2; 0,79H2O; 0,40DMF % calculé C : 70,17; H : 4,12; N : 16,37; % trouvé C : 70,0; H : 3,9; N : 16,4).

### EXEMPLE 12

A 5 g d'acide 4-phénylbutyrique en solution dans 60 ml de dichlorométhane anhydre à une température voisine de 20°C, on ajoute quelques gouttes de diméthylformamide puis, goutte à goutte, 2,8 ml de chlorure de thionyle. Après une nuit de réaction à la même température, le milieu réactionnel est concentré à sec sous pression réduite. Le chlorure de l'acide 4-phénylbutyrique ainsi préparé est ajouté goutte à goutte à 1 g de 8-amino-5H,10H-imidazo[1,2-a]indéno[1,2-e]pyrazine-4-one en solution dans 75 ml de diméthylformamide à une température voisine de 20°C. Le milieu réactionnel est chauffé à reflux pendant 7 heures. Le précipité formé est filtré et recristallisé dans le diméthylformamide. Après filtration, lavage à l'eau puis à l'éther éthylique et séchage sous vide partiel (1 mm Hg; 0,13 kPa) à 80°C, on obtient 0,64 g de 8-(4-phénylbutyrylamino-5H,10H-imidazo[1,2-a]indéno[1,2-e]pyrazine-4-one sous forme de solide ocre se décomposant à 190°C (Analyse C23H20N4O2; 0,63H2O; 0,79DMF % calculé C : 71,86; H : 5,24; N : 14,57; % trouvé C : 71,5; H : 5,1; N : 14,8).

### EXEMPLE 13

On opère comme dans l'exemple 12, à partir de 5 g d'acide 5-phénylvalérique, 2,5 ml de chlorure de thionyle, 60 ml de dichlorométhane, 1 g de 8-amino-5H,10H-imidazo[1,2-a]indéno[1,2-e]pyrazine-4-one et 75 ml de diméthylformamide. Après 7 heures de reflux l'insoluble est filtré et recristallisé dans le diméthylformamide. Après filtration, lavage à l'eau et séchage sous pression réduite (1 mm Hg,0,13 kPa) à 80°C on obtient 0,58 g de 8-(5-phénylvalérylamino-5H,10H-imidazo[1,2-a]indéno[1,2-e]pyrazine-4-one sous forme de solide jaune se décomposant à 170°C (Analyse C24H22N4O2; 0,41 DMF % calculé C : 72,34; H : 5,56; N : 14,06; % trouvé C : 72,0; H :5,6; N :13,8).

### EXEMPLE 14

On opère comme dans l'exemple 7, à partir de 3 g de 8-amino-5H,10H-imidazo[1,2-a]indéno[1,2-e]pyrazine-4-one et de 2 fois 2,8 ml de 2-isocyanatoacétate d'éthyle dans 170 ml de diméthylformamide. L'insoluble ainsi obtenu est filtré et recristallisé dans le diméthylformamide. Après filtration, lavage à l'eau et séchage sous pression réduite (1 mm Hg; 0,13 kPa) à 60°C, on obtient 2,6 g de 8-(3-éthoxycarbonylméthyluréido)-5H,10H-imidazo[1,2-a] indéno[1,2-e]pyrazine-4-one sous forme de solide rose dont le point de fusion est supérieur à 260°C (Analyse C18H17N5O4; 0,75H2O; 0,20DMF % calculé C : 58,85; H : 4,66; N : 19,06; % trouvé C : 58,9; H : 5,0; N : 18,9).

### EXEMPLE 15

A 1 g de 8-(3-éthoxycarbonylméthyluréido)-5H,10H-imidazo[1,2-a]indéno[1,2-e]pyrazine-4-one en suspension dans 40 ml d'éthanol, on ajoute 20 ml de soude à 30% à une température voisine de 20°C. La réaction est poursuivie 18 heures à 50°C. Après refroidissement à une température voisine de 20°C, le précipité formé est filtré, puis repris dans 20 ml d'eau distillée et le milieu acidifié à l'aide d'acide chlorhydrique concentré. La suspension est agitée 2 heures à une température voisine de 20°C puis filtrée, lavée à l'eau et séchée sous vide partiel (1 mm Hg; 0,13 kPa) à 100°C. Après recristallisation dans le diméthylformamide, on obtient 0,22 g de dihydrate de 8-(3-carboxyméthyluréido)-5H,10H-imidazo[1,2-a]indéno[1,2-e]pyrazine-4-one sous forme de solide beige dont le point de fusion est supérieur à 260°C (Analyse C16H12N5O4; 2,0H2O; 0,1DMF % calculé C : 56,64; H : 3,86; N : 20,64; % trouvé C : 56,7; H : 3,8; N : 21,0).

### EXEMPLE 16

A 1,5 g de 8-amino-5H,10H-imidazo[1,2-a]indéno[1,2-e]pyrazine-4-one et 0,15 g de 4-diméthylaminopyridine dans 45 ml de pyridine, on ajoute goutte à goutte 1,2 ml de chlorure de diméthylcarbamoyle. Après 5 heures de réaction à une température voisine de 20°C, on ajoute de nouveau 1,2 ml de chlorure de diméthylcarbamoyle. La réaction est poursuivie une nuit à la même température puis le milieu réactionnel chauffé à 50°C pendant 1 heure. Après refroidissement, l'insoluble est filtré, lavé à l'eau et séché. Le solide ainsi obtenu est recristallisé dans le diméthylformamide, lavé à l'eau puis séché sous vide partiel (1 mm Hg; 0,13 kPa) à 50°C. On obtient 0,3 g de dihydrate de 8-(3,3-diméthyluréido)-5H,10H-imidazo[1,2-a]indéno[1,2-e]pyrazine-4-one sous forme de solide rose dont le point de fusion est supérieur à 260°C (Analyse C16H15N5O2; 2H2O % calculé C : 62,13; H : 4,89; N : 22,64; % trouvé C : 62,2; H : 4,6; N : 22,4).

### EXEMPLE 17

0,5 g de 8-méthoxy-5H,10H-imidazo[1,2-a]indéno[1,2-e]pyrazine-4-one en suspension dans 20 ml d'acide bromhydrique concentré sont chauffés à reflux pendant 6 heures. Après refroisissement à une température voisine de 20°C, l'insoluble est filtré et recristallisé dans le diméthylformamide pour conduire, après filtration, lavage à l'éther éthylique et séchage sous vide partiel (1 mm Hg; 0,13 kPa) à 80°C, à 0,14 g de 8-hydroxy-5H,10H-imidazo[1,2-a]indéno[1,2-e]pyrazine-4-one sous forme de solide jaune dont le point de fusion est supérieur à 260°C (spectre de R.M.N. [200 MHz; DMSO-d6; δ en ppm] : 3,96 (s,2H,10-CH₂); 6,84 (d large, J = 8 Hz,1H,7-H); 7,04 (s large,1H, 9-H); 7,69 (d, J = 8 Hz,1H,6-H); 7,86 et 8,15 (2s,1H chacun, H de l'imidazole); 9,73 (mf,1H, OH); 12,80 (s large,1H, CONH)).

La 8-méthoxy-5H,10H-imidazo[1,2-a]indéno[1,2-e]pyrazine-4-one peut être obtenue de la manière suivante : on dissout 6,3 g de 1-[2-(5-méthoxy-1-oxo-indanyl)]imidazole-2-carboxylate d'éthyle dans une solution 4 N d'acétate d'ammonium dans de l'acide acétique glacial. Après 12 heures de reflux, le mélange est refroidi à une température voisine de 20°C et filtré. Le solide obtenu est lavé à l'eau jusqu'à pH neutre et avec 50 ml d'acétone, puis il est recristallisé à chaud dans 75 ml de diméthylformamide. Les cristaux formés sont séparés par filtration et rincés successivement par 50 ml d'eau et 50 ml d'acétone. On obtient ainsi 3,3 g du produit sous forme d'un solide beige fondant au-dessus de 260°C (Spectre de R.M.N. : (200 MHz; DMSO d6; δ en ppm) : 3,82 (s, 3H : -OCH3); 3,98 (s, 2H : -CH2- en 10); 6,98 (dd, J =8 et 1,5 Hz, 1H : -H7); 7,22 (d, J =1,5 Hz, 1H : -H9); 7,56 et 7,97 (2s, 1H chacun : -H de l'imidazole); 7,78 (d, J =8 Hz, 1H : -H6); 12,30 (s large, 1H : -CO-NH-)).

Le 1-[2-(5-méthoxy-1-oxo-indanyl)]imidazole-2-carboxylate d'éthyle peut être préparé de la façon suivante : un mélange de 13,9 g de 2-bromo-5-méthoxy-1-indanone et de 16,2 g de 2-éthoxycarbonylimidazole dans 300 ml de toluène est porté au reflux pendant 9 heures. Le toluène est alors évaporé sous pression réduite et le résidu est repris par du dichlorométhane et de l'eau. La phase organique est extraite avec du dichlorométhane et lavée à l'eau. Après traitement habituel, le produit brut est purifié par deux chromatographies successives sur colonne de silice avec respectivement des mélanges de dichlorométhane et d'acétate d'éthyle (70/30 en volumes) et de dichlorométhane et de méthanol (98/2 en volumes). On obtient ainsi 7,5 g de 1-[2-(5-méthoxy-1-oxo-indanyl)]imidazole-2-carboxylate d'éthyle (Rf = 0,38, chromatographie sur couche mince de gel de silice, éluant : dichlorométhane-acétate d'éthyle (70-30 en volumes)).

La 2-bromo-5-méthoxy-1-indanone peut être synthétisée comme décrit par D. MUKHOPADHYA, D. N. CHAUDHURY, J. Indian Chem. Soc., 47(5), 450 (1970).

### EXEMPLE 18

A une suspension de 5 g de N-(9-fluorènylméthyloxycarbonyl)-β-alanine dans 100 ml de dichlorométhane anhydre, on ajoute quelques gouttes de diméthylformamide puis, goutte à goutte, 1,5 ml de chlorure de thionyle à une température voisine de 20°C. Après une nuit d'agitation à la même température, le milieu réactionnel est concentré à sec sous pression réduite (1 mm Hg; 0,13 kPa). Le chlorure d'acide ainsi préparé est dissous dans 30 ml de diméthylformamide anhydre et ajouté goutte à goutte à une solution préparée à partir de 2 g de chlorhydrate de 8-amino-5H,10H-imidazo[1,28a]indéno[1,2-e]pyrazine-4-one et 4,5 ml de triéthylamine dans 50 ml de diméthylformamide à une température voisine de 20°C. La réaction est poursuivie 7 heures à 100°C. Le précipité, formé après refroidissement à 0°C, est filtré puis repris dans 25 ml de pipéridine. La suspension est agitée 1 heure à une température voisine de 20°C, l'insoluble filtré et recristallisé dans un mélange de dioxanne et d'acide chlorhydrique 1N (50/50 en volumes). Après filtration, lavage au dioxanne puis séchage sous vide partiel (1 mm Hg; 0,13 kPa) à 50°C, on obtient 0,65 g de chlorhydrate de 8-(3-aminopropionamido-5H,10H-imidazo[1,2-a]indéno[1,2-e]pyrazine-4-one sous forme de solide jaune dont le point de fusion est supérieur à 260°C (Analyse C16H16ClN5O2; 2,0H2O; 0,86HCl; 0,15DMF % calculé C : 55,58; H : 4,66; Cl : 10,25; N : 20,25; % trouvé C : 55,6; H : 4,3; Cl : 9,9; N : 19,9).

### EXEMPLE 19

On opère comme dans l'exemple 18, à partir de 8 g de N-(9-fluorènylméthyloxycarbonyl)glycine, 2,45 ml de chlorure de thionyle et 2 g de 8-amino-5H,10H-imidazo[1,2-a]indéno[1,2-e]pyrazine-4-one. La recristallisation dans un mélange de dioxanne et d'acide chlorhydrique 1N (50/50 en volumes) conduit à 0,19 g de chlorhydrate de 8-aminoacétamido-5H,10H-imidazo[1,2-a]indéno[1,2-e]pyrazine-4-one sous forme de solide beige dont le point de fusion est supérieur à 260°C (Analyse C15H14ClN5O2; 0,74H2O; 0,87HCl; 0,05DMF % calculé C : 61,01; H : 4,44; N : 23,72; % trouvé C : 61,4; H : 4,7; N : 23,6).

### EXEMPLE 20

A une suspension de 1,5 g de chlorhydrate de 8-amino-5H,10H-imidazo[1,2-a]indéno[1,2-e]pyrazine-4-one en suspension dans 15 ml de diméthylformamide, on ajoute 1,53 ml de triéthylamine à une température voisine de 20°C. Après solubilisation,1,79 g de 3-nitrophénylisocyanate en solution dans 10 ml de diméthylformamide sont ajoutés goutte à goutte. Le milieu réactionnel est agité 15 heures à la même température, la suspension filtrée, lavée à l'eau puis avec de l'éther isopropylique et séchée sous vide partiel (1 mm Hg; 0,13 kPa). On obtient ainsi 1,2 g d'hydrate de 8-[3-(3-nitrophényl)uréido]-5H,10H-imidazo[1,2-a]indéno[1,2-e]pyrazine-4-one sous forme de solide jaune dont le point de fusion est supérieur à 260°C (Analyse C20H16N6O5 % calculé C : 57,14; H : 3,84; N : 19,99; % trouvé C : 57,2; H : 4,0; N : 20,1).

### EXEMPLE 21

On opère de la même façon que dans l'exemple 20, à partir de 1 g de chlorhydrate de 8-amino-5H,10H-imidazo[1,2-a]indéno[1,2-e]pyrazine-4-one, 1 ml de triéthylamine et 0,97 ml de 2-méthoxyphénylisocyanate. Après 6 heures de réaction à une température voisine de 20°C, l'insoluble est filtré, lavé à l'eau puis avec de l'éther isopropylique. 0,3 g de 8-[3-(2-méthoxyphényl)uréido]-5H,10H-imidazo[1,2-a]indéno[1,2-e]pyrazine-4-one sont ainsi obtenus sous forme de solide beige dont le point de fusion est supérieur à 260°C (Analyse C21H17N5O3; 0,76H2O % calculé C : 65,11; H : 4,42; N : 18,08; % trouvé C : 64,8; H : 4,3; N : 18,4).

### EXEMPLE 22

On opère de la même façon que dans l'exemple 20, à partir de 1,5 g de chlorhydrate de 8-amino-5H,10H-imidazo[1,2-a]indéno[1,2-e]pyrazine-4-one, 1,53 ml de triéthylamine et 1,79 g de 2-nitrophénylisocyanate. Après 6 heures de réaction à une température voisine de 20°C, l'insoluble est filtré, lavé à l'eau puis avec de l'éther isopropylique. 0,7 g de dihydrate de 8-[3-(2-nitrophényl)uréido]-5H,10H-imidazo[1,2-a]indéno[1,2-e]pyrazine-4-one sont ainsi obtenus sous forme de solide orangé dont le point de fusion est supérieur à 260°C (Analyse C20H18N6O6; 0,83H2O % calculé C : 54,79; H : 4,14; N : 19,17; % trouvé C : 54,5; H : 3,8; N : 19,1).

### EXEMPLE 23

9 ml d'acide chlorhydrique concentré sont ajoutés goutte à goutte à 0,46 g de 8-[3-(4-nitrophényl)uréido]-5H,10H-imidazo[1,2-a]indéno[1,2-e]pyrazine-4-one en suspension dans 5 ml de méthanol à une température voisine de 20°C. Après 15 minutes d'agitation, 0,42 g de fer en poudre, puis 10 ml de diméthylformamide sont ajoutés progressivement. Le milieu réactionnel est chauffé 10 heures à 80°C, puis après refroidissement, l'insoluble est filtré, lavé avec du méthanol puis avec de l'éther isopropylique et séché sous pression réduite (1 mm Hg; 0,13 kPa) à 50°C. On obtient 0,22 g de dichlorhydrate de 8-[3-(4-aminophényl)uréido]-5H,10H-imidazo[1,2-a]indéno [1,2-e]pyrazine-4-one sous forme de solide beige dont le point de fusion est supérieur à 260°C (Spectre de R.M.N. : [200 MHz; (CD₃)₂SO d6; δ en ppm] : 4,10 (s,2H : -CH₂- 10); 7,35 (d, J = 8,5 Hz,2H : -H aromatiques en ortho du -NH₂); 7,48 (d large, J = 8,5 Hz,1H : -H 7); 7,61 (d, J = 8,5 Hz,2H : -H aromatiques en méta du -NH₂); 7,86 (d, J = 8,5 Hz,1H : -H 6); 7,88 (s large,1H : -H 9); 7, 99 et 8,25 (2s larges,1H chacun : -H de l'imidazole); de 8,50 à 9,00 (mf étalé : -NHCONH-); 9,78 (s large,3H : -NH₃⁺Cl⁻); de 12, 90 à 13,2 (mf étalé, 1H : -NHCO- du cycle)).

La 8-[3-(4-nitrophényl)uréido]-5H,10H-imidazo[1,2-a]indéno[1,2-e]pyrazine-4-one peut être obtenue de la manière suivante : on opère comme dans l'exemple 20, à partir de 0,69 g de chlorhydrate de 8-amino-5H,10H-imidazo[1,2-a]indéno[1,2-e]pyrazine-4-one,0,35 ml de triéthylamine et 0,82 g de 4-nitrophénylisocyanate. Après 4 heures d'agitation à une température voisine de 20°C, l'insoluble est filtré, lavé à l'eau distillée et séché sous vide partiel (1 mm Hg; 0,13 kPa) à 20°C. On obtient 0,51 g de 8-[3-(4-nitrophényl)uréido]-5H,10H-imidazo[1,2-a]indéno[1,2-e]pyrazine-4-one sous forme de solide ocre dont le point de fusion est supérieur à 260°C (Analyse C20H14N6O4; 1,1H2O % calculé C : 59,69; H : 3,51; N : 20,89; % trouvé C : 59,3; H : 3,3; N : 20,8).

### EXEMPLE 24

On opère de la même façon que dans l'exemple 20, à partir de 1,5 g de chlorhydrate de 8-amino-5H,10H-imidazo[1,2-a]indéno[1,2-e]pyrazine-4-one, 1,5 ml de triéthylamine et 1,42 ml de 4-méthoxyphénylisocyanate. Après 15 heures de réaction à une température voisine de 20°C, l'insoluble est filtré, lavé à l'eau puis avec de l'éther isopropylique. 0,6 g d'hydrate de 8-[3-(4-méthoxyphényl)uréido]-5H,10H-imidazo[1,2-a]indéno[1,2-e]pyrazine-4-one sont ainsi obtenus sous forme de solide beige dont le point de fusion est supérieur à 260°C (Analyse C21H19N5O4; 0,20H2O % calculé C : 62,22; H : 4,72; N : 17,27; % trouvé C : 62,1; H : 4,4; N : 17,4).

### EXEMPLE 25

On opère comme dans l'exemple 12, à partir de 5,5 ml d'acide isocaproïque, 3,9 ml de chlorure de thionyle, 60 ml de dichlorométhane, 1,5 g de 8-amino-5H,10H-imidazo[1,2-a]indéno[1,2-e]pyrazine-4-one et 75 ml de diméthylformamide. Après 8 heures de reflux, le précipité est filtré puis recristallisé dans le diméthylformamide. Le solide ainsi obtenu est lavé abondemment à l'eau et séché sous vide partiel (1 mm Hg; 0,13 kPa) à 80°C pour conduire à 0,81 g de 8-(4-méthylpentanoyl)amino-5H,10H-imidazo[1,2-a]indéno[1,2-e]pyrazine-4-one sous forme de poudre jaune se décomposant à 220°C (Analyse C19H20N4O2; 1,27H2O % calculé C : 67,84; H :5,99; N : 16,66; % trouvé C : 67,8; H : 5,7; N : 16,8).

### EXEMPLE 26

Une solution de 3 g de 1-[5-(N,N-diméthylsulfamoyl)-1-oxo-indane-2-yl)]imidazole-2-carboxylate d'éthyle dans 300 ml d'une solution 5N de méthanol ammoniacal est agitée pendant 20 heures à une température voisine de 20°C. Le solide apparu est séparé par filtration, lavé 2 fois avec 80 ml au total d'acétone et séché sous pression réduite (15 mm Hg; 2 kPa) à 45°C. On obtient ainsi un premier lot de 1,3 g. Le filtrat est concentré de moitié sous pression réduite (15 mm Hg; 2 kPa) et le solide apparu est séparé par filtration, lavé avec de l'acétone et séché sous pression réduite (15 mm Hg; 2 kPa) à 45°C. On obtient ainsi un deuxième lot de 0,2 g. Les 2 lots réunis sont dissous à 80°C dans 50 ml d'une solution aqueuse d'acide chlorhydrique concentré. Après refroidissement à une température voisine de 5°C et addition de 50 ml d'eau distillée, le solide apparu est séparé par filtration, lavé 10 fois avec 100 ml au total d'eau distillée et séché sous pression réduite (1 mm Hg; 0,13 kPa) à 80°C. On obtient ainsi 1,2 g de N,N-diméthyl-4-oxo-4,5-dihydro-10H-imidazo[1,2-a]indéno[1,2-e]pyrazine-8-sulfonamide fondant au-dessus de 260°C (Spectre de R.M.N. : [200 MHz; (CD₃)₂SO d6; δ en ppm] : 2,66 [s,6H : -N(CH₃)₂]; 4,17 (s,2H : -CH₂- 10); 7,61 et 8,04 (2d, J = 1 Hz,1H chacun : -H de l'imidazole); 7,83 (dd, J = 8,5 et 2 Hz, 1H : -H 7); 7, 97 (d, J = 2Hz,1H : -H 9); 8,10 (d, J = 8,5 Hz,1H : -H 6); 12,46 (mf,1H : -NHCO-)).

Le 1-[5-(N,N-diméthylsulfamoyl)-1-oxo-indane-2-yl)]imidazole-2-carboxylate d'éthyle peut être préparé de la façon suivante : un mélange de 3,8 g d'imidazole-2-carboxylate d'éthyle et de 4,4 g de 2-bromo-5-(N,N-diméthylsulfamoyl)-1-indanone est chauffé pendant 8 minutes à 125°C, refroidi à 20°C et chromatographié sur 1 kg de gel de silice neutre (0,040-0,063 mm) contenus dans une colonne de 10 cm de diamètre en éluant avec un mélange dichlorométhane-acétate d'éthyle (60-40 en volumes) et en recueillant des fractions de 100 ml. Les fractions 16 à 66 sont réunies et concentrées à sec sous pression réduite (15 mm Hg; 2 kPa) à 40°C. Le produit obtenu (4,1 g) est mis en suspension dans 50 ml de dichlorométhane et, après élimination de l'insoluble par filtration, le filtrat est concentré à sec sous pression réduite (15 mm Hg; 2 kPa) à 50°C. On obtient ainsi 3 g de 1-[5-(N,N-diméthylsulfamoyl)-1-oxo-indane-2-yl)]imidazole-2-carboxylate d'éthyle sous la forme d'une laque crème.

La 2-bromo-5-(N,N-diméthylsulfamoyl)-1-indanone peut être préparée de la façon suivante : à une solution de 3,3 g de 5-(N,N-diméthylsulfamoyl)-1-indanone dans un mélange de 30 ml de dichlorométhane, de 14 ml de d'acide acétique et de 0,1 ml d'une solution aqueuse d'acide bromhydrique à 47%, on ajoute goutte à goutte en 30 minutes à -5°C une solution de 2,2 g de brome dans 10 ml de dichlorométhane. Le mélange est ensuite agité pendant 1 heure à une température comprise entre 0°C et 5°C, conservé pendant 16 heures à une température voisine de 5°C, additionné d'un autre mélange préparé dans les mêmes conditions mais à partir de 1,2 g de 5-(N,N-diméthylsulfamoyl)-1-indanone et versé sur 150 ml d'eau distillée. Après décantation et extraction 3 fois avec 150 ml au total de dichlorométhane les extraits organiques réunis sont lavés successivement avec 50 ml d'eau distillée, 50 ml d'une solution aqueuse saturée d'hydrogénocarbonate de sodium, 50 ml d'eau distillée puis séchés sur du sulfate de magnésium anhydre et concentrés à sec sous pression réduite (15 mm Hg; 2 kPa) à 50°C. Le produit obtenu (5,5 g), additionné de 2 g préparés dans les mêmes conditions, est chromatographié sur 700 g de gel de silice neutre (0,040-0,063 mm) contenus dans une colonne de 8 cm de diamètre en éluant avec du dichlorométhane et en recueillant des fractions de 500 ml. Les fractions 3 à 6 sont réunies et concentrées à sec sous pression réduite (15 mm Hg; 2 kPa) à 40°C. On obtient ainsi 4,4 g de 2-bromo-5-(N,N-diméthylsulfamoyl)-1-indanone fondant à 115°C.

La 5-(N,N-diméthylsulfamoyl)-1-indanone peut être préparée de la façon suivante : une solution de 7 g de 5-chlorosulfonyl-1-indanone dans 70 ml de tétrahydrofuranne anhydre est additionnée goutte à goutte en 30 minutes à 70 ml de diméthylamine maintenue à -30°C. Le mélange est ensuite agité pendant 1 heure à -10°C et pendant 16 heures en laissant progressivement remonter la température à 20°C puis on ajoute 300 ml d'eau distillée et 300 ml d'acétate d'éthyle. La solution organique est lavée succesivement avec 100 ml d'eau distillée, avec 100 ml d'une solution aqueuse 1N d'acide chlorhydrique et avec de l'eau distillée jusqu'à neutralité puis séchée sur du sulfate de magnésium anhydre et concentrée à sec sous pression réduite (15 mm Hg; 2 kPa) à 40°C. On obtient ainsi 5,8 g de 5-(N,N-diméthylsulfamoyl)-1-indanone fondant à 142°C.

La 5-chlorosulfonyl-1-indanone peut être préparée comme décrit par J.J. HOWBERT et T.A. CROWELL, Synth. Commun., 20 (20), 3197 (1990).

### EXEMPLE 27

A une suspension de 1,3 g de dichlorhydrate de 8-amino-5H,10H-imidazo[1,2-a]indéno[1,2-e]pyrazine-4-one dans 50 ml de diméthylformamide anhydre, on ajoute 1,2 g de triéthylamine puis, goutte à goutte en 5 minutes à une température voisine de 20°C, une solution de 1,62 g d'isothiocyanate de phényle. Après 1 heure d'agitation à la même température, l'insoluble apparu est séparé par filtration, lavé successivement avec 4 ml de diméthyformamide, 5 ml d'eau distillée, 5 ml d'acétone et séché à l'air. Le produit obtenu (1,15 g) est agité pendant 1 heure dans 15 ml de méthanol, filtré, lavé avec 5 ml de méthanol et séché sous pression réduite (1 mm Hg; 0,13 kPa) à 100°C. On obtient ainsi 0,9 g de 8-(3-phénylthiouréido)-5H,10H-imidazo[1,2-a]indéno[1,2-e]pyrazine-4-one se décomposant sans fondre au-dessus de 280°C (Spectre de R.M.N. : [200 MHz; (CD₃)₂SO d6; δ en ppm] : 4,02 (s,2H : -CH₂- 10); 7,14 (t large, J = 7,5 Hz,1H : -H 4 du phénylthiouréido); 7,36 (t, J = 7,5 Hz,2H : -H 3 et -H 5 du phénylthiouréido); 7,45 (dd, J = 8,5 et 2 Hz,1H : -H 7); 7,51 (d large, J = 7,5 Hz,2H : -H 2 et -H 6 du phénylthiouréido); 7,58 et 7, 95 (2d, J = 1 Hz,1H chacun : -H de l'imidazole); 7,76 (d, J = 2 Hz, 1H : -H 9); 7,80 (d, J = 8,5 Hz,1H : -H 6); 9,85 et 9, 93 (2s larges,1H chacun : NHCSNH- en 8),12,50 (mf étalé,1H : -CONH- du cycle)).

### EXEMPLE 28

A une suspension de 1,3 g de dichlorhydrate de 8-amino-5H,10H-imidazo[1,2-a]indéno[1,2-e]pyrazine-4-one dans 50 ml de diméthylformamide anhydre, on ajoute 1,2 g de triéthylamine puis, goutte à goutte en 5 minutes à une température voisine de 20°C, une solution de 0,9 g d'isothiocyanate de méthyle. Après 16 heures d'agitation à la même température, le mélange est versé dans 100 ml d'eau distillée et agité pendant 15 minutes. L'insoluble apparu est séparé par filtration, lavé 2 fois avec 10 ml au total d'eau distillée et séché sous pression réduite (1 mm Hg; 0,13 kPa) à 100°C. Le produit obtenu (0,25 g) est agité pendant 1 heure dans 2,5 ml de méthanol, filtré, lavé avec 0,5 ml de méthanol et séché sous pression réduite (1 mm Hg; 0,13 kPa) à 100°C. On obtient ainsi 0,21 g de 8-(3-méthylthiouréido)-5H,10H-imidazo[1,2-a]indéno[1,2-e]pyrazine-4-one se décomposant sans fondre au-dessus de 280°C (Spectre de R.M.N. : [200 MHz; (CD₃)₂SO d6; δ en ppm] : 2, 96 (d, J = 2,5 Hz,3H : -CH₃); 4,10 (s large,2H : -CH₂- 10); 7,33 (d large, J = 8,5 Hz,1H : -H 7); 7,56 et 7, 96 (2s larges,1H chacun : -H de l'imidazole); 7,64 (s large,1H : -H 9); de 7,65 à 7,80 et 9,65 (2 mfs,1H chacun : -NHCSNH- en 8); 7,78 (d large, J = 8,5 Hz,1H : -H 6); 12,34 (s large,1H : -NHCO-)).

### EXEMPLE 29

Un mélange de 0,9 g de 1-(5-benzylthio-1-oxo-indane-2-yl)]imidazole-2-carboxamide et de 25 ml d'acide chlorhydrique concentré est chauffé pendant 20 minutes à 80°C, refroidi à une température voisine de 20°C et versé sur 25 ml d'eau distillée. Le solide est séparé par filtration, lavé à l'eau distillée jusqu'à neutralité, 2 fois avec 20 ml au total d'acétone et séché à l'air. Le produit obtenu (0,8 g) est dissous dans 150 ml d'un mélange bouillant de diméthylformamide et d'eau distillée (80-20 en volumes) et, après refroidissement et conservation pendant 1 heure à une température voisine de 5°C, les cristaux apparus sont séparés par filtration, lavés 2 fois avec 20 ml au total d'acétone et séchés sous pression réduite (1 mm Hg; 0,13 kPa) à 100°C. On obtient ainsi 0,5 g de 8-benzylthio-5H,10H-imidazo[1,2-a]indéno[1,2-e]pyrazine-4-one se décomposant sans fondre au-dessus de 260°C (Spectre de R.M.N. : [200 MHz; (CD₃)₂SO d6; δ en ppm] : 3,97 (s,2H : -CH₂- 10); 4,28 (s,2H : Ar-CH₂S-); de 7,15 à 7,45 (mt,6H : -H aromatiques et -H 7); 7,56 et 7, 95 (2s larges,1H chacun : -H de l'imidazole); 7,56 (s large,1H : -H 9); 7,76 (d, J = 8 Hz,1H : -H 6); 12,30 (s large,1H : -NHCO-)).

La 1-(5-benzylthio-1-oxo-indane-2-yl)]imidazole-2-carboxamide peut être préparée de la façon suivante : une solution de 3 g de 1-(5-benzylthio-1-oxo-indane-2-yl)]imidazole-2-carboxylate d'éthyle dans 140 ml d'une solution 5N de méthanol ammoniacal est agitée pendant 20 heures à une température voisine de 20°C. Aprés addition de 80 ml d'oxyde d'isopropyle, le solide apparu est séparé par filtration, lavé 2 fois avec 40 ml au total d'oxyde d'isopropyle et séché sous pression réduite (2 mm Hg; 0,26 kPa) à 60°C. On obtient ainsi 0,9 g de 1-(5-benzylthio-1 oxo-indane-2-yl)]imidazole-2-carboxamide fondant à 219°C.

Le 1-(5-benzylthio-1-oxo-indane-2-yl)]imidazole-2-carboxylate d'éthyle peut être préparé de la façon suivante : un mélange de 3 g d'imidazole-2-carboxylate d'éthyle et de 3,6 g de 2-bromo-5-benzylthio-1-indanone est chauffé pendant 15 minutes à 140°C, refroidi à 20°C et chromatographié sur 300 g de gel de silice neutre (0,040-0,063 mm) contenus dans une colonne de 4,5 cm de diamètre en éluant avec un mélange dichlorométhane-acétate d'éthyle (90-10 en volumes) et en recueillant des fractions de 30 ml. Les fractions 31 à 76 sont réunies et concentrées à sec sous pression réduite (15 mm Hg; 2 kPa) à 40°C. Le produit obtenu (1,7 g) est mis en suspension dans 50 ml d'oxyde d'isopropyle et le mélange est agité pendant 1 heure à 20°C. Le solide est séparé par filtration, lavé 2 fois avec 20 ml au total d'oxyde d'isopropyle et séché sous pression réduite (15 mm Hg; 2 kPa) à 40°C. On obtient ainsi 1,3 g de 1-(5-benzylthio-1-oxo-indane-2-yl)]imidazole-2-carboxylate d'éthyle fondant à 131°C.

La 2-bromo-5-benzylthio-1-indanone peut être préparée de la façon suivante : Une solution de 3,2 g de brome dans 25 ml de dichlorométhane est ajoutée goutte à goutte en 30 minutes à 5°C à une solution de 5,3 g de 5-benzylthio-1-indanone dans 50 ml de dichlorométhane. Le mélange est agité pendant 40 heures à une température voisine de 20°C, dilué avec 75 ml de dichlorométhane, lavé 4 fois avec 200 ml au total d'eau distillée, séché sur du sulfate de sodium anhydre et séché sous pression réduite (15 mm Hg; 2 kPa) à 40°C. Le produit obtenu (7,5 g) est chromatographié sur 300 g de gel de silice neutre (0,040-0,063 mm) contenus dans une colonne de 4,5 cm de diamètre en éluant avec un mélange cyclohexane-acétate d'éthyle (95-5 en volumes) et en recueillant des fractions de 30 ml. Les fractions 4 à 35 sont réunies et concentrées à sec sous pression réduite (15 mm Hg; 2 kPa) à 40°C. On obtient ainsi 4 g de 2-bromo-5-benzylthio-1-indanone fondant à 88°C.

La 5-benzylthio-1-indanone peut être préparée comme décrit par J.J. HOWBERT et T.A. CROWELL, Synth. Commun., 20 (20), 3197 (1990).

### EXEMPLE 30

A une suspension de 1,37 g de 8-[3-(2-chloroéthyl)uréido]-5H,10H-imidazo[1,2-a]indéno[1,2-e]pyrazine-4-one dans 35 ml de diméthylsulfoxyde anhydre on ajoute 0,29 g d'hydrure de sodium à 80% à une température voisine de 20°C. Le mélange est ensuite agité pendant un heure à 50°C, refroidi à 20°C, additionné de 16 ml d'eau distillée et d'acide acétique de façon à ajuster le pH à 4 puis centrifugé. La solution surnageante est éliminée et le solide est lavé sur filtre 2 fois avec 10 ml au total d'eau distillée et avec 5 ml d'acétone puis séché sous pression réduite (1 mm Hg; 0,13 kPa) à 100°C. Le produit obtenu (0,93 g) est chromatographié sur 28 g de gel de silice neutre (0,020-0,045 mm) contenus dans une colonne de 3,5 cm de diamètre en éluant sous pression avec un mélange chloroforme-méthanol-ammoniaque à 28% (82-15-3 en volumes) et en recueillant une fraction de 170 ml et une fraction de 825 ml . Cette dernière est concentrée à sec sous pression réduite (15 mm Hg; 2 kPa) à 40°C. Le produit obtenu (0,2 g) est agité pendant 30 minutes dans 2 ml de méthanol, séparé par filtration, lavé 2 fois avec 1 ml au total de méthanol et séché sous pression réduite (1 mm Hg; 0,13 kPa) à 100°C. On obtient ainsi 0,12 g de 8-(2-oxo-1-imidazolinyl)-5H,10H-imidazo[1,2-a]indéno[1,2-e]pyrazine-4-one se décomposant sans fondre au-dessus de 260°C (Spectre de R.M.N. : [200 MHz; (CD₃)₂SO d6; δ en ppm] : 3.45 (t, J = 7,5 Hz,2H : -CH₂NH-); 3, 93 (t, J = 7,5 Hz,2H : -CH₂-); 4,00 (s,2H : -CH₂- 10); 6, 97 (s large,1H : -CH₂-NH-); 7,47 (dd, J = 8,5 et 2 Hz,1H : -H 7); 7,56 et 7, 92 (2s larges,1H chacun : -H de l'imidazole); 7,80 (d, J = 8,5 Hz,1H : -H 6); 7,99 (d, J = 2 Hz,1H : -H 9); 12,23 (s large,1H : -NHCO- du cycle)).

La 8-[3-(2-chloroéthyl)uréido]-5H,10H-imidazo[1,2-a]indéno[1,2-e]pyrazine-4-one peut être préparée de la façon suivante : à une suspension de 3,1 g de dichlorhydrate de 8-amino-5H,10H-imidazo[1,2-a]indéno[1,2-e]pyrazine-4-one dans 80 ml de diméthylformamide anhydre, on ajoute 2,2 g de triéthylamine puis, goutte à goutte à une température voisine de 20°C,1,2 g d'isocyanate de 2-chloroéthyle. Après 16 heure d'agitation à la même température, l'insoluble apparu est séparé par filtration, lavé 2 fois avec 40 ml au total d'eau distillée et avec 20 ml d'acétone puis séché sous pression réduite (1 mm Hg; 0,13 kPa) à 100°C. 0,3 g de produit obtenu (sur 2,63 g obtenus au total) est agité pendant 5 minutes dans 13 ml de diméthylsulfoxyde, séparé par filtration, lavé 2 fois avec 4 ml au total d'eau distillée et séché sous pression réduite (1 mm Hg; 0,13 kPa) à 100°C. On obtient ainsi 0,15 g de 8-[3-(2-chloroéthyl)uréido]-5H,10H-imidazo[1,2-a]indéno[1,2-e]pyrazine-4-one se décomposant sans fondre au-dessus de 260°C (Spectre de R.M.N. : [200 MHz; (CD₃)₂SO d6; δ en ppm] : 3,47 (q, J = 6 Hz,2H : -CH₂-); 3,71 (t, J = 6 Hz,2H : -CH₂Cl); 3,97 (s,2H : -CH₂- 10); 6,50 (t, J = 6 Hz,1H : -CONH-); 7,32 (dd, J = 8 et 1,5 Hz,1H : -H 7); 7,57 et 7,92 (2s larges, 1H chacun : -H de l'imidazole); 7,73 (d, J = 8 Hz,1H : -H 6); 7,82 (d, J = 1,5 Hz,1H : -H 9); 8,87 (s large,1H : -NHCO- en 8); 12,25 (s large, 1H : -NHCO-du cycle)).

### EXEMPLE 31

Le mélange de 21,4 g d'anhydride acétique et de 11,5 g d'acide formique est chauffé pendant 2 heures à une température comprise entre 50°C et 60°C et refroidi à 20°C. On ajoute ensuite 0,9 g d'acétate de sodium anhydre puis, après dissolution, 1,6 g de dichlorhydrate de 8-amino-5H,10H-imidazo[1,2-a]indéno[1,2-e]pyrazine-4-one. Le mélange est agité pendant 1 heure à 20°C, refroidi à 5°C puis on ajoute 10 ml d'eau distillée. L'insoluble apparu est séparé par filtration, lavé 2 fois avec 20 ml au total d'eau distillée et séché sous pression réduite (5 mm Hg; 0,65 kPa) à 40°C. 0,5 g de produit obtenu (sur 1,34 g obtenu au total) est agité pendant 10 minutes dans 10 ml d'acide acétique bouillant et, après refroidissement, séparé par filtration, lavé 2 fois avec 2 ml au total d'acide acétique et séché sous pression réduite (1 mm Hg; 0,13 kPa) à 100°C. On obtient ainsi 0,45 g de 8-formamido-5H,10H-imidazo[1,2-a]indéno[1,2-e]pyrazine-4-one fondant à 280°C (décomposition) (Spectre de R.M.N. : [200 MHz;(CD₃)₂SO d6; δ en ppm] : Mélange d'isomères : 75% d'isomère E et 25% d'isomère Z : 4,05 (s,2H : -CH₂- 10); 7,25 (dd, J = 8,5 et 2 Hz,0,25 H : -H 7 de l'isomère Z); 7,50 (s,0,25 H : 1 des -H imidazole pour l'isomère Z); 7,60 (dd, J = 8,5 et 2 Hz,0,75 H : -H 7 de l'isomère E); de 7,70 à 7,90 (mt,2H : -H 6 et -H 9); 7,98 et 8,11 (2s,0,75 H chacun : -H de l'imidazole pour l'isomère E); 8,16 (s,0,25 H : l'autre -H imidazole pour l'isomère Z); 8,33 (d, J =2 Hz,0,75 H : -CH=O de l'isomère E); 8,86 (d, J = 10,5 Hz,0,25 H : -CH=O de l'isomère Z); 10,30 (d, J = 10,5 Hz,0,25 H : -NHCO- en 8 de l'isomère Z); 10,41 (s large,0,75 H : -NHCO- en 8 de l'isomère E); 12,70 et 12,74 (2s larges,1H en totalité : -CONH- du cycle)).

### EXEMPLE 32

A une solution de 6 g de chlorhydrate de 8-amino-5H,10H-imidazo[1,2-a]indéno[1,2-e]pyrazine-4-one, 240 ml de diméthylformamide et 10,75 ml de triéthylamine on ajoute en 5 minutes 6,25 ml de 3-(chloroformyl)propionate d'éthyle et on chauffe le mélange réactionnel à reflux pendant 6 heures. Le mélange est concentré sous pression réduite et additionné de 500 ml d'eau. Le précipité obtenu est filtré et séché à l'air pour donner 4,6 g de produit brut. Une partie (2 g) est purifiée par chromatographie sur colonne de silice (80 g) en éluant avec un mélange d'acétate d'éthyle et de méthanol (d'abord 70/30 en volumes, puis 30/70); la silice correspondant à la tête de colonne est alors récupérée et traitée avec 60 ml de diméthylformamide, filtrée et rincée à l'eau. Le filtrat est évaporé sous pression réduite et le résidu d'évaporation est séché à 60°C sous vide (1 mmHg; 0,13 kPa). On obtient 0,27 g de 8-(3-éthoxycarbonylpropionylamino)-5H,10H-imidazo[1,2-a]indéno[1,2-e]pyrazine-4-one sous forme de solide verdâtre fondant au-dessus de 260°C (Analyse % calculé C : 62,29, H : 4,95, N : 15,29, O : 17,47, % % trouvé C : 62,1, H : 4,5, N : 15,7).

### EXEMPLE 33

On chauffe à 50°C pendant 4 heures sous couverture d'argon 1,19 g de 8-(3-éthoxycarbonylpropionylamino)-5H,10H-imidazo[1,2-a]indéno[1,2-e] pyrazine-4-one, 1,2 ml de soude 10N et 48 ml d'éthanol. Le mélange réactionnel est filtré et l'insoluble lavé à l'éthanol (2x15 ml) et séché à l'air. Le solide obtenu est trituré dans 75 ml de méthanol, filtré et séché à 60°C sous vide (1 mmHg; 0,13 kPa). On obtient 0,32 g de 8-(3-carboxypropionylamino)-5H,10H-imidazo[1,2-a]indéno[1,2-e]pyrazine-4-one sous forme de sel disodique de formule C₁₇H₁₂N₄O₄Na₂ (Analyse % calculé C : 53,41, H : 3,16, N : 14,66, O : 16,74, Na : 12,03, % trouvé C : 53,5, H : 2,7, N : 14,2).

### EXEMPLE 34

On opère comme à l'exemple 32 mais à partir de 4 g de chlorhydrate de 8-amino-5H,10H-imidazo[1,2-a]indéno[1,2-e]pyrazine-4-one, 86 ml de diméthylformamide, 7,3 ml de triéthylamine et 4,42 g de 3-isocyanatopropionate d'éthyle et le mélange réactionnel est agité a une température voisine de 20°C pendant 90 heures. Après évaporation du diméthylformamide, on ajoute 300 ml d'eau et on filtre le précipité formé, lave à l'eau (50 ml), puis à l'acétone (2x50 ml) et sèche à 60°C sous vide (1 mmHg; 0,13 kPa). On obtient 2,1 g de 8-[3-(2-éthoxycarbonyléthyl)uréido]-5H,10H-imidazo[1,2-a] indéno[1,2-e]pyrazine-4-one sous forme de solide beige fondant au-dessus de 260°C (Analyse % calculé C : 59,84, H : 5,02, N : 18,36, O : 16,78, % trouvé C : 59,5, H : 5,1, N : 18,5).

### EXEMPLE 35

On opère comme à l'exemple 33 mais à partir de 0,53 g de 8-[3-(2-éthoxycarbonyléthyl)uréido]-5H,10H-imidazo[1,2-a]indéno[1,2-e]pyrazine-4-one, 0,2 ml de soude 10N et 8 ml d'éthanol. On obtient 0,47 g de 8-[3-(2-carboxyéthyl)uréido]-5H,10H-imidazo[1,2-a]indéno[1,2-e]pyrazine-4-one fondant au-dessus de 260°C (Analyse % calculé C : 51,39, H : 3,30, N : 17,63, O : 16,11, Na : 11,57, % trouvé C : 51,1, H : 2,9, N : 17,3).

### EXEMPLE 36

A une solution de 2 g de chlorhydrate de 8-amino-5H,10H-imidazo[1,2-a]indéno[1,2-e]pyrazine-4-one,20 ml de diméthylformamide et 1,3 g de triéthylamine on ajoute en 30 minutes une solution de 1,76 g de 4-fluorophénylisocyanate dans 20 ml de diméthylformamide et on agite pendant 12 heures à une température voisine de 20°C . Le mélange réactionnel est filtré et l'insoluble est lavé avec du diméthylformamide (2x10 ml), puis avec de l'eau (3x15 ml) et séché à l'air. Le produit brut est ensuite trituré dans 30 ml de diméthylformamide, filtré, lavé à l'eau, puis à l'éther isopropylique et séché à 60°C sous vide (1 mmHg; 0,13 kPa). On obtient 1,5 g de 8-[3-(4-fluorophényl)uréido]-5H,10H-imidazo[1,2-a]indéno[1,2-e]pyrazine-4-one sous forme de solide beige fondant au-dessus de 260°C (Analyse % calculé C : 64,00, H : 3,76, F : 5,06, N : 18,66, O : 8,52, % trouvé C : 64,0, H : 3,8, N : 19,0).

### EXEMPLE 37

On opère comme à l'exemple 36 mais à partir de 1,5 g de chlorhydrate de 8-amino-5H,10H-imidazo[1,2-a]indéno[1,2-e]pyrazine-4-one, 30 ml de diméthylformamide, 0,97 g de triéthylamine et 2,64 g de 3-fluorophénylisocyanate. On obtient 0,44 g de 8-[3-(3-fluorophényl)uréido]-5H,10H-imidazo[1,2-a]indéno[1,2-e]pyrazine-4-one sous forme de solide marron clair fondant au-dessus de 260°C (Analyse % calculé C : 64,00, H : 3,76, F : 5,06, N : 18,66, O : 8,52, % trouvé C : 64,1, H : 3,4, N : 18,6).

### EXEMPLE 38

On opère comme à l'exemple 36 mais à partir de 2 g de chlorhydrate de 8-amino-5H,10H-imidazo[1,2-a]indéno[1,2-e]pyrazine-4-one, 40 ml de diméthylformamide, 1,3 g de triéthylamine et 1,76 g de 2-fluorophénylisocyanate. On obtient 1 g de 8-[3-(2-fluorophényl)uréido]-5H,10H-imidazo[1,2-a]indéno[1,2-e]pyrazine-4-one sous forme de solide beige fondant au-dessus de 260°C (Analyse % calculé C : 64,00, H : 3,76, F : 5,06, N : 18,66, O : 8,52, % trouvé C : 64,0, H : 3,5, F : 4,6, N : 18,6).

### EXEMPLE 39

On opère comme à l'exemple 36 mais à partir de 2 g de chlorhydrate de 8-amino-5H,10H-imidazo[1,2-a]indéno[1,2-e]pyrazine-4-one, 40 ml de diméthylformamide, 1,3 g de triéthylamine et 1,82 g d'éthylisocyanate. On obtient 0,85 g de 8-(3-éthyluréido)-5H,10H-imidazo[1,2-a] indéno[1,2-e]pyrazine-4-one sous forme de solide violet fondant au-dessus de 260°C (Analyse % calculé C : 62,13, H : 4,89, N : 22,64, O : 10,34, % trouvé C : 61,7, H : 4,9, N : 22,3, O : 9,9).

### EXEMPLE 40

A 0,3 g d'hydrure de sodium en suspension dans 5 ml de diméthylformamide anhydre sous atmosphère d'azote, on ajoute goutte à goutte 50 ml d'une solution de 1,5 g de 8-amino-5H,10H-imidazo[1,2-a]indéno[1,2-e]pyrazine-4-one dans le diméthylformamide. Après 1 heure d'agitation à une température voisine de 20°C, 2,4 ml de chlorure de triméthylsilyle sont coulés goute à goutte dans le milieu réactionnel et l'agitation poursuivie 30 minutes à la même température. 2,05 g de 1,1'-carbonyldiimidazole sont alors ajoutés progressivement, puis après 4 heures de réaction à 20°C, 2,8 ml de morpholine sont additionnés goutte à goutte et le milieu réactionnel porté à 60°C pendant 1 heure. La poursuite de la réaction pendant 1 nuit à une température voisine de 20°C conduit après filtration de l'insoluble, lavage à l'eau et séchage sous pression réduite (1 mm Hg ; 0,13 kPa) à 50°C, à 1,2 g de produit brut sous forme de poudre noire. Ce produit brut est recristallisé dans un mélange d'eau et de dioxanne (1/1 en volumes) pour conduire après lavage à l'eau puis séchage sous pression réduite à 0,19 g d'hydrate de 8-[3-morpholino-uréido]-5H,10H-imidazo[1,2-a]indéno[1,2-e]pyrazine-4-one sous forme de solide rouge dont le point de fusion est supérieur à 260°C (Analyse C18H19N5O4 ; 0,26H2O % calculé C : 58,53; H : 5,18; N : 18,96; trouvé C : 58,4; H : 5,2; N : 19,0).

### EXEMPLE 41

A une solution de 1 g de 10-hydroxyimino-8-(3-méthyluréido)-5H,10H-imidazo[1,2-a]indéno[1,2-e]pyrazine-4-one, de 0,1 g d'acétate d'ammonium et de 27 ml d'ammoniaque aqueuse (28 %), est ajouté 0,8 g de zinc en poudre. Le milieu réactionnel est porté à une température voisine de 100°C pendant 3 heures. Après refroidissement, 40 ml d'acide chlorhydrique (6N) sont ajoutés et on laisse sous agitation pendant 18 heures à une température voisine de 20°C. La suspension ainsi obtenue est filtrée et lavée par 3 fois 25 ml d'éther éthylique et 2 X 25 ml d'acétone. Le résidu solide ainsi obtenu est séché sous pression réduite (0,5-1,5 bar) à 40°C pour conduire à 0,56 g de 10-amino-8-(3-méthyluréido)-5H,10H-imidazo[1,2-a]indéno[1,2-e]pyrazine-4-one, dichlorhydrate fondant à une température supérieure à 260°C (Analyse C₁₅ H₁₆ Cl₂ N₆ O₂ , 1,31 H₂O : % calculé C : 47,01, H : 4,21, Cl : 18,5, N : 21,93, O : 8,35, % trouvé C : 46,8, H : 4,2, Cl : 16,9, N : 21,2, O : 8,0).

### EXEMPLE 42

A une solution de 5 g de 8-(3-méthyluréido)-5H,10H-imidazo[1,2-a]indéno [1,2-e]pyrazine-4-one et de 50 ml de diméthylsulfoxyde, sont ajoutés 1,23 g de NaH (80 %) spatule par spatule, en maintenant le milieu réactionnel à une température voisine de 20°C. Après une heure d'agitation à cette température, une solution de 2,33 g de nitrite d'isoamyle et de 10 ml de diméthylsulfoxyde est ajoutée goutte à goutte en maintenant la température à 20°C. Le milieu réactionnel est ensuite agité pendant 18 heures puis on ajoute successivement 10 ml d'eau, 10 ml d'acide acétique, 40 ml de méthanol et 15 ml d'eau. Après 4 heures d'agitation, la suspension ainsi obtenue est filtrée et le solide lavé par 3 fois 100 ml d'eau et 2 fois 100 ml de méthanol. Le résidu solide ainsi obtenu est séché sous pression réduite (0,5-1,5 bar) à 40°C pour conduire à 3 g de 10-hydroxyimino-8-(3-méthyluréido)-5H,10H-imidazo[1,2-a]indéno[1,2-e]pyrazine-4-one fondant à une température supérieure à 260°C (Analyse C₁₅ H₁₂ N₆ O₃, 0,25 H₂O, 0,7 DMSO : % calculé C : 55,56, H : 3,73, N : 25,91, O : 14,81, % trouvé C : 55,0, H : 3,2, N : 26,1, O : 14,5).

### EXEMPLE 43

A 2,88 g de chlorhydrate de 8-amino-5H-imidazo[1,2-a]indéno[1,2-e]pyrazine-4,10-dione en solution dans 40 ml de diméthylformamide à une température voisine de 20°C, on ajoute goutte à goutte 5,6 ml de triéthylamine puis 2,36 ml d'isocyanate de méthyle. L'agitation est poursuivie 15 heures à la même température. Le pécipité formé est filtré et recristallisé dans le diméthylsulfoxyde aqueux à 5% d'eau. Après refroidissement à une température voisine de 20°C, le solide ainsi obtenu est filtré, lavé à l'acétone et séché sous pression réduite (1 mm Hg; 0,13 kPa) à 80°C pour conduire à 2,1 g de 8-(3-méthyluréido)-5H-imidazo[1,2-a]indéno[1,2-e]pyrazine-4,10-dione sous forme de solide grenât dont le point de fusion est supérieur à 260°C (Analyse C15H11N5O3; 0,17H2O % calculé C : 58,25; H : 3,58; N : 22,64; trouvé C : 58,1; H : 3,6; N : 22,5).

Le chlorhydrate de 8-amino-5H-imidazo[1,2-a]indéno[1,2-e]pyrazine-4,10-dione peut être préparé selon le protocole suivant : à une solution de 14,2 g de chlorure d'étain (II) dans 350 ml d'acide chlorhydrique concentré agités à une température voisine de 20°C, on ajoute progressivement 5,6 g de 8-nitro-5H-imidazo[1,2-a]indéno[1,2-e]pyrazine-4,10-dione. Le milieu réactionnel est porté à 50°C pendant 2 heures, puis refroidi et versé sur 100 ml d'eau glacée. Le précipité ainsi obtenu est filtré, lavé à l'eau et à l'acétone et séché sous pression réduite (1 mm Hg; 0,13 kPa). On obtient 4,1 g de produit attendu sous forme de solide verdâtre dont le point de fusion est supérieur à 260°C.

La 8-nitro-5H-imidazo[1,2-a]indéno[1,2-e]pyrazine-4,10-dione peut être préparée selon la méthode suivante : à 90 ml d'acide sulfurique concentré refroids à 5°C, on ajoute progressivement 7,11 g de 5H-imidazo[1,2-a] indéno[1,2-e]pyrazine-4,10-dione, puis après 30 minutes d'agitation à 10°C, 3,03 g de nitrate de potassium en une seule fois. La réaction est poursuivie 15 heures à une température voisine de 20°C. Le milieu réactionnel est coulé sur 500 ml d'eau glacée et le précipité formé filtré, lavé à l'eau et à l'acétone, et séché. On obtient ainsi 6,5 g de produit attendu sous forme de solide orangé dont le point de fusion est supérieur à 260°C.

La 5H-imidazo[1,2-a]indéno[1,2-e]pyrazine-4,10-dione peut être obtenue de la manière suivante : une suspension de 1,5 g de 10-(hydroxyimino)-5H-imidazo[1,2-a]indéno[1,2-e]pyrazine-4-one dans 90 ml d'une solution aqueuse d'acide chlorhydrique environ 5N est agitée à l'ébullition pendant 7 heures, refroidie puis concentrée à sec sous pression réduite (15 mm Hg; 2 kPa) à 70°C. Le produit obtenu (2 g) est dissous dans 50 ml de diméthylformamide et la solution, additionnée de 0,1 g de noir décolorant, est filtrée puis le filtre est lavé 3 fois avec 30 ml au total de diméthylformamide. Le filtrat et le lavage sont réunis, additionnés de 600 ml d'eau distillée et centrifugés. Le solide est mis en suspension dans 20 ml d'eau distillée, filtré, lavé avec 20 ml d'acétone et séché sous pression réduite (1 mm Hg; 0,13 kPa) à 100°C. Le produit obtenu (1,13 g) est dissous dans 115 ml de diméthylsulfoxyde et la solution, additionnée de 0,1 g de noir décolorant, est filtrée puis le filtre est lavé 2 fois avec 30 ml au total de diméthylsulfoxyde. Le filtrat et le lavage sont réunis, additionnés de 115 ml d'eau distillée et centrifugés. Le solide est mis en suspension dans 20 ml d'eau distillée, filtré, lavé 2 fois avec 20 ml au total d'acétone et séché sous pression réduite (1 mm Hg; 0,13 kPa) à 100°C. On obtient ainsi 1 g de 5H-imidazo[1,2-a]indéno[1,2-e]pyrazine-4,10-dione sous la forme d'un solide rouge orangé fondant à 360°C (décomposition) [Spectre de R.M.N.: (200 MHz; DMSO d6; δ en ppm) : 7,42 et 7,55 (2t, J =7 Hz, 2H : -H7 et -H8); 7,52 et 7,72 (2d, J =7 Hz, 1H chacun : -H6 et -H9); 7,62 et 8,13 (2d, J =1 Hz, 1H chacun : -H de l'imidazole); 13,60 (mf, 1H : -NH-)].

La 10-(E-hydroxyimino)-5H-imidazo[1,2-a]indéno[1,2-e]pyrazine-4-one peut être préparée de la manière suivante : 0,4 g d'hydrure de sodium à 80% est ajouté à une suspension de 1,1 g de 5H,10H-imidazo[1,2-a]indéno[1,2-e]pyrazine-4-one dans 10 ml de diméthylsulfoxyde anhydre. Après 10 minutes d'agitation à une température voisine de 20°C, une solution de 0,7 g de nitrite d'isoamyle dans 2 ml de diméthylsulfoxyde anhydre est ajoutée goutte à goutte en 5 minutes puis le mélange est agité pendant 1 heure à la même température. 10 ml d'eau distillée sont ajoutés lentement et le mélange est ensuite versé sur 120 g d'eau et de glace, acidifié avec 1 ml d'acide acétique puis centrifugé. Après élimination de la solution surnageante, le solide est mis en suspension dans 25 ml d'eau distillée, filtré, lavé avec 10 ml d'acétone et séché sous pression réduite (15 mm Hg; 2 kPa) à 20°C. Le produit obtenu (1,5 g) est dissous dans 100 ml de diméthylformamide bouillant et la solution, additionnée de 0,1 g de noir décolorant, est filtrée à chaud, refroidie, versée sur 800 ml d'eau distillée et centrifugée. Le solide est mis en suspension dans 20 ml d'eau distillée, filtré, lavé avec 20 ml d'acétone et séché sous pression réduite (15 mm Hg; 2 kPa) à 20°C. Le produit obtenu (0,9 g) est dissous dans 75 ml de diméthysulfoxyde à 20°C et la solution, additionnée de 0,1 g de noir décolorant est filtrée. Le filtre est lavé 2 fois avec 20 ml au total de diméthylsulfoxyde puis le filtrat et le lavage sont réunis, additionnés de 75 ml d'eau distillée et centrifugés. Le solide est mis en suspension dans 25 ml d'eau distillée, filtré, lavé 2 fois avec 50 ml au total d'acétone puis séché sous pression réduite (1 mm Hg; 0,13 kPa) à 60°C. On obtient ainsi 0,63 g de 10-(E-hydroxyimino)-5H-imidazo[1,2-a]indéno[1,2-e]pyrazine-4-one se décomposant sans fondre au-dessus de 300°C [Spectre de R.M.N.: (200 MHz; DMSO d6; δ en ppm) : 7,40 et 7,48 (2t, J =7 Hz, 2H : -H7 et -H8); 7,60 et 8,00 (2s larges, 1H chacun : -H de l'imidazole); 7,82 et 8,20 (2d, J =7 Hz, 1H chacun : -H6 et -H9); 12,70 et 13,00 (2mf, 1H chacun : -NH- et -OH)].

### EXEMPLE 44

A 0,5 g de 8-[3-(3-nitrophényl)uréido]-5H,10H-imidazo[1,2-a]indéno[1,2-e]pyrazine-4-one en suspension dans 2 ml de méthanol, on ajoute, goutte à goutte, 3 ml d'acide chlorhydrique concentré. Cette suspension jaune est alors traitée avec 0, 36 g de fer en poudre et 5 ml de diméthylformamide. Le milieu réactionnel est porté à reflux pendant 5 heures. Après refroidissement à une température voisine de 20°C, l'insoluble est filtré, lavé au méthanol et séché sous vide partiel (1 mm Hg; 0,13 kPa). On obtient ainsi 0,31 g de dichlorhydrate et dihydrate de 8-[3-(3-aminophényl)uréido]-5H,10H-imidazo[1,2-a]indéno[1,2-e]pyrazine-4-one sous forme de poudre ocre dont le point de fusion est supérieur à 260°C (Analyse C20H16N6O2; 2,67HCl; 2,64 H2O % calculé C : 64,51; H : 4,33; N : 22,57; % trouvé C : 64,1; H : 4,0; N : 22,7).

### EXEMPLE 45

0,76 g de 5-trifluorométhoxy-2-(2-éthoxycarbonyl-1H-imidazol-1-yl)-indanone et 19 g d'acétate d'ammonium dans 30 ml d'acide acétique sont chauffés à reflux pendant 45 minutes. Après refroidissement à une température voisine de 20°C, le précipité formé est filtré, lavé à l'eau et séché sous pression réduite (1 mm Hg; 0,13 kPa) à 40°C. On obtient 0,5 g de 8-trifluorométhoxy-5H,10H-imidazo[1,2-a]indéno[1,2-e]pyrazine-4-one sous forme de poudre beige dont le point de fusion est supérieur à 260°C (Analyse C14H8F3N3O2; 0,9 AcOH % calculé C : 54,73; H : 2,62; F : 18,55; N : 13,68; % trouvé C : 55,0; H : 2,6; F : 18,3; N : 13,8).

La 5-trifluorométhoxy-2-(2-éthoxycarbonyl-1H-imidazol-1-yl)-indanone peut être obtenue selon le protocole suivant : 1,5 g de 1H-imidazole-2-carboxylate d'éthyle et 1,6 g de 5-trifluorométhoxy-2-bromo-indanone en solution dans 40 ml de toluène sont chauffés au reflux pendant 15 heures. Après refroidissement à une température voisine de 20°C et concentration à sec du milieu réactionnel, le produit brut est purifié par flash-chromatographie sur colonne de silice en utilisant un mélange acétate d'éthyle-cyclohexane (75/25 en volumes) comme éluant. 0,8 g de produit attendu sont ainsi isolés sous forme d'huile brune utilisée telle quelle dans les synthèses ultérieures.

La 5-trifluorométhoxy-2-bromo-indanone peut être préparée de la façon suivante : à 3,2 g de 5-trifluorométhoxy-indanone et trois gouttes d'acide bromhydrique concentré en solution dans 70 ml d'acide acétique, on ajoute goutte à goutte en 30 minutes, 0,38 ml de brome en solution dans 15 ml d'acide acétique. Après 5 heures d'agitation à une température voisine de 20°C, le milieu réactionnel est versé sur de la glace et la phase organique extraite par du dichlorométhane, lavée à l'eau et séchée. Le produit brut ainsi obtenu est purifié par flash-chromatographie sur colonne de silice en utilisant un mélange de dichlorométhane et de cyclohexane (50/50 en volumes) comme éluant. On isole 1,5 g de produit attendu sous forme d'huile jaune utilisée telle quelle dans les synthèses ultérieures.

La 5-trifluorométhoxy-indanone peut être obtenue selon la méthode suivante : à une suspension refroidie à 0°C de 3,3 g de chlorure d'aluminium anhydre dans 60 ml de 1,2-dichloroéthane, on ajoute goutte à goutte une solution de 6,5 g de chlorure de l'acide 3-(3-trifluorométhoxyphényl)propanoïque dans 30 ml du même solvant. La réaction est poursuivie une nuit à une température voisine de 20°C. Le milieu réactionnel est alors versé sur de l'eau glacée et acidifié à l'aide d'acide chlorhydrique 1N. La phase organique est extraite par de l'éther éthylique, lavée à l'eau, séchée sur sulfate de magnésium puis concentrée à sec sous pression réduite. Le produit brut ainsi obtenu est purifié par flash-chromatographie sur colonne de silice en utilisant un mélange de dichlorométhane et de cyclohexane (50/50 en volumes) comme éluant. On obtient 3,4 g de produit attendu sous forme d'huile jaune utilisée telle quelle dans les synthèses ultérieures.

Le chlorure de l'acide 3-(3-trifluorométhoxyphényl)propanoïque peut être préparé selon la procédure suivante : à 6 g d'acide 3-(3-trifluorométhoxyphényl)propanoïque en solution dans 80 ml de dichlorométhane, on ajoute, goutte à goutte et à une température voisine de 20°C, 2,2 ml de chlorure de thionyle. Le milieu réactionnel est chauffé au reflux pendant 2 heures. Après refroidissement à une température voisine de 20°C et concentration à sec sous pression réduite, on obtient 6,5 g de produit attendu sous forme d'huile brune utilisé sans purification supplémentaire dans les synthèses ultérieures.

L'acide 3-(3-trifluorométhoxyphényl)propanoïque peut être obtenu de la manière suivante : 10 g d'acide 3-trifluorométhoxy-cinnamique sont solubilisés dans 170 ml de méthanol et mis en présence de 2,7 g de chlorure de nickel, hexahydrate à 0°C. A cette solution brune, on ajoute goutte à goutte, en environ 90 minutes, 7,9 g de borohydrure de sodium. Le milieu réactionnel est alors réchauffé à une température voisine de 20°C et la réaction poursuivie une nuit à la même température. L'insoluble est filtré et écarté, et le filtrat concentré à sec sous pression réduite. Le résidu obtenu est repris dans de la potasse aqueuse à 5%, la phase organique extraite par de l'éther éthylique, puis la phase aqueuse acidifiée à l'aide d'acide chlorhydrique concentré et la phase organique extraite par de l'éther éthylique. On obtient ainsi après traitement habituel 7,7 g d'acide attendu sous forme d'huile jaune utilisée telle quelle dans les synthèses ultérieures.

### EXEMPLE 46

1,3 g de 8-cyano-5H,10H-imidazo[1,2-a]indéno[1,2-e]pyrazine-4-one en solution dans 20 ml d'acide sulfurique à 60% sont portés à reflux pendant 2 heures. Le milieu réactionnel est alors refroidi à une température voisine de 20°C, puis versé dans 100 ml d'eau glacée. Le précipité formé est filtré, lavé à l'eau puis repris dans une solution aqueuse de soude. La solution aqueuse est agitée pendant 1 heure à 20°C, puis filtrée et le filtrat acidifié à l'aide d'acide chlorhydrique concentré. Le précipité est alors filtré, lavé à l'eau puis à l'acétone pour conduire après séchage sous pression réduite (1 mm Hg; 0,13 kPa) à 0,44 g d'acide 5H,10H-imidazo[1,2-a]indéno[1,2-e]pyrazine-4-one-8-carboxylique sous forme de poudre brune dont le point de fusion est supérieur à 260°C [Spectre de R.M.N.¹H (300MHz, (CD₃)₂SO d6, δ en ppm) : 4,11 (s, 2H : CH₂10); 7,76 et 8,15 (2 s larges, 1H chacun : H de l'imidazole); 7,95 (d, j = 8 Hz, 1H : H6); 8,05 (d, large, J = 8 Hz, 1H : H7); 8,15 (s, large, 1H : H9); 12,74 (s, large, 1H : NH5)].

La 8-cyano-5H,10H-imidazo[1,2-a]indéno[1,2-e]pyrazine-4-one peut être obtenue de la manière suivante : une solution de 3 g de 5-cyano-2-(2-carboxamido-1H-imidazol-1-yl)-indanone dans 40 ml d'acide acétique est portée à reflux pendant 15 heures. Après refroidissement à une température voisine de 20°C, 100 ml d'éther éthylique sont ajoutés au milieu réactionnel et le précipité formé est filtré, lavé au dichlorométhane et séché pour conduire à 1,3 g de produit attendu sous forme de poudre brune dont le point de fusion est supérieur à 260°C utilisée telle quelle dans les synthèses ultérieures.

La 5-cyano-2-(2-carboxamido-1H-imidazol-1-yl)-indanone peut être préparée selon la procédure suivante : à une solution méthanolique (1 litre) saturée en ammoniac et refroidie à 5°C, on ajoute goutte à goutte 11 g de 5-cyano-2-(2-éthoxycarbonyl-1H-imidazol-1-yl)-indanone en solution dans 200 ml de méthanol. Après une nuit d'agitation à une température voisine de 20°C, le milieu réactionnel est concentré à sec sous pression réduite pour conduire à 11,3 g de produit attendu sous forme de poudre brune utilisée sans purification supplémentaire dans les étapes ultérieures.

La 5-cyano-2-(2-éthoxycarbonyl-1H-imidazol-1-yl)-indanone peut être préparée de la façon suivante : à une solution de 15 g de 2-bromo-5-cyano-indanone dans 400 ml de toluène, on ajoute 17,8 g de 1H-imidazole-2-carboxylate d'éthyle et on porte le mélange à reflux pendant 6 heures. Après refroidissement à une température voisine de 20°C, le précipité est filtré, lavé avec du toluène et le filtrat concentré à sec sous pression réduite. Le produit brut ainsi obtenu est purifié par flash-chromatographie sur colonne de silice en utilisant un mélange de dichlorométhane et de méthanol (98/2 en volumes) comme éluant. On obtient ainsi 11 g de produit attendu sous forme de meringue brune utilisée telle quelle dans les synthèses ultérieures.

La 2-bromo-5-cyano-indanone peut être obtenue selon le protocole suivant : à une solution de 26 g de 5-cyano-indanone dans 600 ml de chloroforme refroids à 5°C, on ajoute goutte à goutte une solution de 8,4 ml de brome dans 100 ml de chloroforme. La réaction est poursuivie 3 heures à une température voisine de 20°C, puis le milieu réactionnel est versé dans 1,5 litre d'une solution saturée de bicarbonate de sodium. La phase organique est décantée, lavée à l'eau, séchée sur sulfate de sodium et concentrée à sec sous pression réduite. Le produit brut ainsi obtenu est purifié par flash-chromatographie sur colonne de silice en utilisant un mélange de cyclohexane et d'acétate d'éthyle (80/20 en volumes) comme éluant. On obtient 17 g de produit attendu sous forme de poudre jaune fondant à 124°C.

La 5-cyano-indanone peut être obtenue selon la méthode décrite par N.L. Allinger et E.S. Jones, J. Org. Chem., 27, 70 (1962).

### EXEMPLE 47

Une solution de 1,4 g d'isocyanate de triméthylsilyle dans 4 ml de diméthylformamide anhydre est ajoutée goutte à goutte à 20°C en 5 minutes à une solution de 1,25 g de chlorhydrate de 8-amino-5H,10H-imidazo[1,2-a]indéno[1,2-e]pyrazine-4-one et de 0,8 g de triéthylamine dans 40 ml de diméthylformamide anhydre. Après 15 heures d'agitation à la même température, l'insoluble apparu est séparé par filtration, lavé successivement 2 fois avec 20 ml au total de diméthylformamide, 2 fois avec 20 ml au total d'acétone, 3 fois avec 60 ml au total d'eau distillée, 2 fois avec 10 ml au total d'acétone puis séché sous pression réduite (1 mm Hg; 0,13 kPa) à une température voisine de 20°C. On obtient ainsi 1 g de 8-uréido-5H,10H-imidazo[1,2-a]indéno[1,2-e]pyrazine-4-one se décomposant sans fondre au-dessus de 260°C (Analyse % calculé C : 59,78, H : 3,94, N : 24,90, O : 11,38, % trouvé C :59,5, H : 3,4, N : 25,3,).

### EXEMPLE 48

On ajoute 1,3 g d'isothiocyanate de 2-*tert*-butyloxycarbonylaminoéthyle à une solution de 0,48 g de 8-amino-5H,10H-imidazo[1,2-a]indéno[1,2-e]pyrazine-4-one dans 20 ml de diméthylformamide anhydre. La solution est agitée pendant 15 heures à une température voisine de 20°C et pendant 2 heures 30 minutes à 60°C puis concentrée à sec sous pression réduite (15 mm Hg; 2 kPa) à la même température. Le produit obtenu est mis en suspension dans 50 ml d'acétone et l'insoluble est séparé par filtration, lavé 2 fois avec 10 ml au total d'acétone et séché. Le produit obtenu (0,88 g) est chromatographié sur 15 g de gel de silice neutre (0,040-0,063 mm) contenus dans une colonne de 2,5 cm de diamètre en éluant par un mélange chloroforme-éthanol- ammoniaque à 28% (75-20-5 en volumes) et en recueillant des fractions de 100 ml. Les fractions contenant le produit attendu sont concentrées à sec sous pression réduite (15 mm Hg; 2 kPa) à 50°C. Le produit obtenu est mis en suspension dans 20 ml d'un mélange chloroformeméthanol (80-20 en volumes), séparé par filtration, lavé avec 5 ml du même mélange et avec 5 ml d'acétone puis séché. On obtient ainsi 0,1 g de 8-[3-(2-*tert*-butyloxycarbonylaminoéthyl)thiouréido]-5H,10H-imidazo[1,2-a]indéno[1,2-e]pyrazine-4-one.

0,45 g de 8-[3-(2-*tert*-butyloxycarbonylaminoéthyl)thiouréido]-5H,10H-imidazo[1,2-a]indéno[1,2-e]pyrazine-4-one est dissous dans 8 ml d'acide trifluoroacétique et la solution est conservée pendant 30 minutes à 20°C puis concentrée à sec sous pression réduite (15 mm Hg; 2 kPa) à 30°C. Le produit obtenu est mis en suspension dans 20 ml d'éthanol bouillant et, après refroidissement à 20°C, séparé par filtration, lavé avec 5 ml d'éthanol et 2 fois avec 10 ml au total d'acétone puis séché sous pression réduite (1 mm Hg; 0,13 kPa) à 20°C. On obtient ainsi 0,32 g de trifluoroacétate de 8-[3-(2-aminoéthyl)thiouréido]-5H,10H-imidazo[1,2-a]indéno[1,2-e]pyrazine-4-one se décomposant sans fondre au-dessus de 260°C (Analyse % calculé C : 47,58, H : 3,77, F : 12,54, N : 18,49, O : 10,56, S: 7,06, % trouvé C : 47,4, H : 3,3, N : 18,8, S: 7,1).

L'isothiocyanate de 2-*tert*-butyloxycarbonylaminoéthyle peut être préparé comme décrit par K. ARIGA et E.V. ANSLYN, J. Org. Chem., 57 (2), 417 (1992).

### EXEMPLE 49

A une solution agitée de 1 g de 8-amino-5H,10H-imidazo[1,2-a]indéno[1,2-e]pyrazine-4-one dans 40 ml de diméthylformamide anhydre, on ajoute 2,1 g d'isothiocyanate de benzoyle et poursuit l'agitation pendant 15 heures à une température voisine de 25°C. L'insoluble apparu est séparé par filtration, lavé 2 fois avec 10 ml au total de méthanol et 2 fois avec 20 ml au total d'acétone; on obtient ainsi un premier lot de 0,5 g. Le filtrat est concentré à sec sous pression réduite (15 mm Hg; 2 kPa) à 60°C et le produit obtenu est mis en suspension dans 50 ml d'acétone, séparé par filtration, mis encore en suspension dans 50 ml de méthanol, séparé par filtration, lavé avec 5 ml de méthanol puis séché; on obtient ainsi un deuxième lot de 0,64 g. Les 2 lots sont réunis et chromatographiés sur 20 g de gel de silice neutre (0,040-0,063 mm) contenus dans une colonne de 2,5 cm de diamètre en éluant par un mélange chloroforme-éthanol-ammoniaque à 28% (75-20-5 en volumes) et en recueillant des fractions de 150 ml. Les fractions contenant le produit attendu sont concentrées à sec sous pression réduite (15 mm Hg; 2 kPa) à 50°C. Le produit obtenu (0,35 g) est mis en suspension dans 40 ml de méthanol, séparé par filtration, lavé 3 fois avec 15 ml au total de méthanol et 2 fois avec 10 ml au total d'acétone et séché sous pression réduite (1 mm Hg; 0,13 kPa) à 70°C. On obtient ainsi 0,3 g de 8-(3-benzoylthiouréido)-5H,10H-imidazo[1,2-a]indéno[1,2-e]pyrazine-4-one se décomposant sans fondre au-dessus de 260°C.

Une suspension de 0,4 g de 8-(3-benzoylthiouréido)-5H,10H-imidazo[1,2-a]indéno[1,2-e]pyrazine-4-one dans 10 ml d'une solution aqueuse 0,12 N de soude est agitée pendant 1 heure à 80°C. Après refroidissement à 20°C, l'insoluble est séparé par filtration, lavé 2 fois avec 10 ml au total d'eau distillée et 3 fois avec 15 ml au total d'acétone puis séché sous pression réduite (1 mm Hg; 0,13 kPa) à 20°C. On obtient ainsi 0,17 g de 8-thiouréido-5H,10H-imidazo[1,2-a]indéno[1,2-e]pyrazine-4-one se décomposant sans fondre au-dessus de 260°C (Analyse % calculé C : 56,55, H : 3,73, N : 23,55, O : 5,38, S : 10,78, % trouvé C : 56,9, H : 3,6, N : 23,1, S : 10,0).

### EXEMPLE 50

0,16 g de sodium est dissous dans 15 ml de méthanol puis une solution de 1,17 g de iodhydrate, trifluoroacétate de 8-[3-(2-aminoéthyl)-2-méthyl-isothiouréido]-5H,10H-imidazo[1,2-a]indéno[1,2-e]pyrazine-4-one dans 25 ml de méthanol est ajoutée goutte à goutte en 15 minutes à une température voisine de 20°C. Après 15 heures d'agitation à la même température, l'insoluble apparu est séparé par filtration, lavé 2 fois avec 10 ml au total de méthanol et 2 fois avec 10 ml au total d'éther éthylique et séché sous pression réduite (1 mm Hg; 0,13 kPa) à 50°C. On obtient ainsi 0,4 g de 8-[(2-imidazoline-2-yl)amino]-5H,10H-imidazo[1,2-a]indéno[1,2-e]pyrazine-4-one se décomposant sans fondre au-dessus de 260°C (Analyse % calculé C : 62,74, H : 4,61, N : 27,43, O : 5,22, % trouvé C : 63,1, N : 27,0).

Le iodhydrate, trifluoroacétate de 8-[3-(2-aminoéthyl)-2-méthyl-isothiouréido]-5H,10H-imidazo[1,2-a]indéno[1,2-e]pyrazine-4-one peut être préparé de la façon suivante : 0,58 g de iodhydrate de 8-[3-(2-*tert*-butoxycarbonylaminoéthyl)-2-méthyl-isothiouréido]-5H,10H-imidazo[1,2-a]indéno[1,2-e]pyrazine-4-one est dissous dans 8 ml d'acide trifluoroacétique et la solution est conservée pendant 30 minutes à une température voisine de 20°C puis concentrée à sec sous pression réduite (15 mm Hg; 2 kPa) à 40°C. Le produit obtenu est dissous dans 50 ml d'éthanol et, après cristallisation puis addition de 10 ml d'éther éthylique, les cristaux apparus sont séparés par filtration, lavés 2 fois avec 10 ml au total d'éther éthylique et séchés sous pression réduite (15 mm Hg; 2 kPa) à 20°C. On obtient ainsi 0,55 g de iodhydrate, trifluoroacétate de 8-[3-(2-aminoéthyl)-2-méthyl-isothiouréido]-5H,10H-imidazo[1,2-a]indéno[1,2-e]pyrazine-4-one fondant à 220°C.

Le iodhydrate de 8-[3-(2-*tert*-butoxycarbonylaminoéthyl)-2-méthyl-isothiouréido]-5H,10H-imidazo[1,2-a]indéno[1,2-e]pyrazine-4-one peut être préparé de la façon suivante : 2,1 g d'iodure de méthyle sont ajoutés à une solution agitée de 6,2 g de 8-[3-(2-*tert*-butoxycarbonylaminoéthyl)-thiouréido]-5H,10H-imidazo[1,2-a]indéno[1,2-e]pyrazine-4-one dans 200 ml de diméthylformamide anhydre. Après 2 heures d'agitation à 40°C, la solution est concentrée à sec sous pression réduite (1 mm Hg; 0,13 kPa) à 50°C. Le produit obtenu est mis en suspension dans 300 ml de méthanol, l'insoluble est éliminé par filtration et le filtrat, additionné de noir décolorant est de nouveau filtré et concentré à sec sous pression réduite (15 mm Hg; 2 kPa) à 40°C. Le produit obtenu (8 g) est dissous dans un mélange bouillant de 150 ml d'éthanol et de 200 ml d'isopropanol, et, après refroidissement à une température voisine de 20°C, les cristaux apparus sont séparés par filtration, lavés 2 fois avec 20 ml au total d'isopropanol, 3 fois avec 30 ml au total d'éther éthylique puis séchés sous pression réduite (1 mm Hg; 0,13 kPa) à 50°C. On obtient ainsi 2,3 g de iodhydrate de 8-[3-(2-*tert*-butoxycarbonylaminoéthyl)-2-méthylisothiouréido]-5H,10H-imidazo[1,2-a]indéno[1,2-e]pyrazine-4-one.

### EXEMPLE 51

Une solution de 1,57 g de 8-amino-5H,10H-imidazo[1,2-a]indéno[1,2-e]pyrazine-4-one dans 75 ml de diméthylformamide anhydre est ajoutée goutte à goutte en 15 minutes à une température voisine de 20°C à une suspension de 0,36 g d'hydrure de sodium à 80% dans 5 ml de diméthylformamide anhydre. Le mélange est ensuite agité pendant 1 heure à la même température puis 2,1 g de chlorure de triméthylsilyle sont ajoutés goutte à goutte en 5 minutes. Le mélange est ensuite agité pendant 30 minutes puis 2,1 g de 1,1'-carbonyldiimidazole sont ajoutés en 5 minutes. Après 4 heures d'agitation, 2,2 g de pyrrolidine sont ajoutés goutte à goutte en 10 minutes et le mélange est ensuite agité pendant 1 heure à 60°C puis pendant 15 heures à une température voisine de 20°C. On ajoute ensuite lentement 10 ml d'eau distillée et 20 ml d'acide acétique puis le mélange est agité pendant 15 minutes et concentré à sec sous pression réduite (1 mm Hg; 0,13 kPa) à 60°C. Le produit obtenu est mis en suspension dans 50 ml d'eau distillée, séparé par filtration, lavé 4 fois avec 40 ml au total d'eau distillée et 5 fois avec 50 ml au total d'acétone puis séché sous pression réduite (1 mm Hg; 0,13 kPa) à 50°C. On obtient ainsi 1,7 g de 8-[(1-pyrrolidinyl)carbonylamino]-5H,10H-imidazo[1,2-a]indéno[1,2-e]pyrazine-4-one (Analyse % calculé C : 64,47, H : 5,11, N : 20,88, O : 9,54, % trouvé C : 64,3, H : 4,6, N : 20,6, O : 9,5).

### EXEMPLE 52

En opérant comme à l'exemple 51 mais en remplaçant la pyrrolidine par l'azétidine (1,8 g), on obtient 1,6 g de 8-[(1-azétidinyl)carbonylamino]-5H,10H-imidazo[1,2-a]indéno[1,2-e]pyrazine-4-one (Spectre de R.M.N.¹H (300 MHz, (CD₃)₂SO d6, δ en ppm) : 2,19 (mt, 2H : CH₂) ; 3,96 (mt, 6H : NCH₂ et CH₂ 10) ; 7,47 (d large, J = 8 Hz, 1H : H 7) ; 7,62 (s large, 1H : H 9); 7,68 (d, J = 8 Hz, 1H : H 6) ; 7,82 et 7,97 (2 s larges, 1H chacun : H de l'imidazole) ; 8,50 (s large, 1H : ArNH) ; 12,40 (s large, 1H : NH 5).

### EXEMPLE 53

A une suspension de 1,6 g de dichlorhydrate de 8-amino-5H,10H-imidazo[1,2-a]indéno[1,2-e]pyrazine-4-one dans 35 ml de diméthylformamide anhydre on ajoute goutte à goutte 1,4 ml de triéthylamine puis 1,3 g d'isocyanate de propyle en solution dans 5 ml de diméthylformamide anhydre. Le mélange réactionnel est agité pendant 15 heures à une température voisine de 20°C. L'insoluble apparu est séparé par filtration, lavé avec 2 ml de diméthylformamide, 2 fois avec 10 ml au total d'eau distillée et avec 5 ml d'acétone puis séché sous pression réduite (15 mm Hg; 2 kPa) à 20°C. Le produit obtenu (1 g) est agité en suspension pendant 15 minutes dans 15 ml de méthanol, séparé par filtration, lavé 2 fois avec 4 ml au total de méthanol et séché sous pression réduite (1 mm Hg; 0,13 kPa) à 100°C. On obtient ainsi 0,82 g de 8-(3-propyluréido)-5H,10H-imidazo[1,2-a]indéno [1,2-e]pyrazine-4-one (Analyse % calculé C : 63,15, H : 5,30, N : 21,66, O : 9,90, % trouvé C : 62,8, H : 5,5, N : 21,7).

### EXEMPLE 54

A une suspension de 1,6 g de dichlorhydrate de 8-amino-5H,10H-imidazo[1,2-a]indéno[1,2-e]pyrazine-4-one dans 35 ml de diméthylformamide anhydre on ajoute goutte à goutte 1,4 ml de triéthylamine puis 1,3 g d'isocyanate d'isopropyle en solution dans 5 ml de diméthylformamide anhydre. Le mélange réactionnel est agité pendant 15 heures à une température voisine de 20°C. L'insoluble apparu est séparé par filtration, lavé avec 2 ml de diméthylformamide, 2 fois avec 10 ml au total d'eau distillée et avec 5 ml d'acétone puis séché sous pression réduite (15 mm Hg; 2 kPa) à 20°C. Le produit obtenu (1,14 g) est agité en suspension pendant 15 minutes dans 10 ml de méthanol, séparé par filtration, lavé avec 2 ml de méthanol et séché sous pression réduite (1 mm Hg; 0,13 kPa) à 100°C. Le produit obtenu (0,75 g) est agité en suspension pendant 15 minutes dans 15 ml d'acide acétique , séparé par filtration, lavé avec 3 ml d'acide acétique, 2 fois avec 10 ml au total d'eau distillée et avec 5 ml d'acétone puis séché sous pression réduite (1 mm Hg; 0,13 kPa) à 100°C.On obtient ainsi 0,52 g de 8-(3-isopropyluréido)-5H,10H-imidazo[1,2-a]indéno[1,2-e]pyrazine-4-one (Analyse % calculé C : 63,15, H : 5,30, N : 21,66, O : 9,90, % trouvé C : 62,9, H : 4,6, N : 21,8).

### EXEMPLE 55

A une suspension de 1,6 g de dichlorhydrate de 8-amino-5H,10H-imidazo[1,2-a]indéno[1,2-e]pyrazine-4-one dans 35 ml de diméthylformamide anhydre on ajoute goutte à goutte 1,4 ml de triéthylamine puis 1,5 g d'isocyanate de butyle en solution dans 5 ml de diméthylformamide anhydre. Le mélange réactionnel est agité pendant 15 heures à une température voisine de 20°C. L'insoluble apparu est séparé par filtration, lavé avec 2 ml de diméthylformamide, 2 fois avec 10 ml au total d'eau distillée et avec 5 ml d'acétone puis séché sous pression réduite (15 mm Hg; 2 kPa) à 20°C. Le produit obtenu (1,2 g) est purifié par chromatographie sur colonne de silice en éluant avec un mélange chloroforme-éthanol-ammoniaque à 28% (75-20-5 en volumes). Le produit obtenu (0,66 g) est agité en suspension pendant 30 minutes dans 10 ml de méthanol, séparé par filtration, lavé 2 fois avec 2 ml au total de méthanol et séché sous pression réduite (1 mm Hg; 0,13 kPa) à 100°C. On obtient ainsi 0,49 g de 8-(3-butyluréido)-5H,10H-imidazo[1,2-a]indéno[1,2-e]pyrazine-4-one (Analyse % calculé C : 64,08, H : 5,68, N : 20,76, O : 9,48, % trouvé C : 64,1, H : 5,2, N : 20,1).

### EXEMPLE 56

A une suspension de 1,6 g de dichlorhydrate de 8-amino-5H,10H-imidazo[1,2-a]indéno[1,2-e]pyrazine-4-one dans 35 ml de diméthylformamide anhydre on ajoute goutte à goutte 1,4 ml de triéthylamine puis 1,9 g d'isothiocyanate de 2-chloroéthyle en solution dans 5 ml de diméthylformamide anhydre. Le mélange réactionnel est agité pendant 15 heures à une température voisine de 20°C. L'insoluble apparu est séparé par filtration, lavé avec 2 ml de diméthylformamide, 2 fois avec 10 ml au total d'eau distillée et avec 5 ml d'acétone puis séché sous pression réduite (15 mm Hg; 2 kPa) à 20°C. Le produit obtenu (1,7 g) est purifié par chromatographie sur colonne de silice en éluant avec un mélange chloroforme-éthanol-ammoniaque à 28% (75-20-5 en volumes). Le produit obtenu (0,78 g) est agité en suspension pendant 30 minutes dans 10 ml de propanol, séparé par filtration, lavé 2 fois avec 2 ml au total de propanol et séché sous pression réduite (1 mm Hg; 0,13 kPa) à 100°C. On obtient ainsi 0,63 g de 8-[(2-thiazolin-2-yl)amino]-5H,10H-imidazo[1,2-a]indéno[1,2-e]pyrazine-4-one partiellement salifiée (40%) sous forme de chlorhydrate (Analyse % calculé C : 56,86, H : 4,00, Cl : 4,20, N : 20,72, O : 4,73, S: 9,49 % trouvé C : 56,9, H : 3,7, Cl : 4,2, N : 20,7, O : 4,4, S: 9,8).

### EXEMPLE 57

A une suspension de 0,96 g du mélange 70-30 respectivement de chlorhydrate de 8-amino-5H,10H-imidazo[1,2-a]indéno[1,2-e]pyrazine-4-one et de chlorhydrate de 8-méthylamino-5H,10H-imidazo[1,2-a]indéno[1,2-e]pyrazine-4-one dans 25 ml de diméthylformamide anhydre on ajoute goutte à goutte 0,5 ml de triéthylamine puis 0,5 g d'isocyanate de méthyle en solution dans 2 ml de diméthylformamide anhydre. Le mélange réactionnel est agité pendant 15 heures à une température voisine de 20°C. L'insoluble apparu est séparé par filtration et le filtrat est concentré à sec sous pression réduite (1 mm Hg; 0,13 kPa) à 60°C. Le produit obtenu (1,26 g) est agité en suspension pendant 30 minutes dans 20 ml d'eau distillée, séparé par filtration, lavé 2 fois avec 10 ml au total d'eau distillée et séché sous pression réduite (5 mm Hg; 0,65 kPa) à 100°C. Le produit obtenu (0,14 g) est purifié par chromatographie sur colonne de silice en éluant avec un mélange chloroforme-méthanol-ammoniaque à 28% (75-20-5 en volumes). Les fractions contenant le produit attendu sont concentrées à sec sous pression réduite (1 mm Hg; 0,13 kPa) à 60°C. On obtient ainsi 26 mg de 8-(1,3-diméthyluréido)-5H,10H-imidazo[1,2-a]indéno[1,2-e]pyrazine-4-one (Spectre de R.M.N.¹H (300 MHz, (CD₃)₂SO d6, δ en ppm) : 2,57 (d, J = 5,5 Hz, 3H : CH₃) ; 3,17 (s, 3H : ArNCH₃) ; 4,00 (s, 2H : CH₂ 10) ; 5,95 (t, J = 5,5 Hz, 1H : NHCO) ; 7,28 (d large, J = 8 Hz, 1H : H 7) ; 7,50 (s large, 1H : H 9) ; 7,60 et 7,99 (2 s larges, 1H chacun : H de l'imidazole) ; 7,85 (d, J = 8 Hz, 1H : H 6) ; 12,40 (s large, 1H : NH 5).

Le chlorhydrate de 8-méthylamino-5H,10H-imidazo[1,2-a]indéno[1,2-e]pyrazine-4-one peut être préparé de la façon suivante :A une suspension de 3 g de 8-formylamino-5H,10H-imidazo[1,2-a]indéno[1,2-e]pyrazine-4-one dans 155 ml de tétrahydrofuranne anhydre on ajoute goutte à goutte, à une température voisine de 20°C, 14 ml d'une solution à 2 moles par litre dans le tétrahydrofuranne du complexe borane-diméthylsulfure. Le mélange est ensuite agité pendant 3 heures à l'ébullition et, après refroidissement et addition de 155 ml de méthanol, saturé par un courant de gaz chlorhydrique, agité de nouveau à l'ébullition pendant 1 heure puis pendant 15 heures à une température voisine de 20°C. L'insoluble est séparé par filtration, lavé avec 40 ml de méthanol et séché sous pression réduite (5 mm Hg; 0,65 kPa) à 40°C. On obtient ainsi 2,64 g d'un mélange 70-30 respectivement de chlorhydrate de 8-amino-5H,10H-imidazo[1,2-a]indéno[1,2-e]pyrazine-4-one et de chlorhydrate de 8-méthylamino-5H,10H-imidazo[1,2-a]indéno[1,2-e]pyrazine-4-one utilisé tel quel à l'étape suivante.

Le 8-formylamino-5H,10H-imidazo[1,2-a]indéno[1,2-e]pyrazine-4-one peut être préparé de la façon suivante : Le mélange de 171 g d'anhydride acétique et de 92 g d'acide formique est agité pendant 2 heures à 55°C et refroidi à une température voisine de 20°C. On ajoute ensuite 7,2 g d'acétate de sodium anhydre et par petites fractions, 12,7 g de dichlorhydrate de 8-amino-5H,10H-imidazo[1,2-a]indéno[1,2-e]pyrazine-4-one. Le mélange est agité pendant 15 heures à une température voisine de 20°C et, après addition lente de 80 ml d'eau distillée à 5°C, l'insoluble est séparé par filtration, lavé 2 fois avec 160 ml au total d'eau distillée et séché sous pression réduite (5 mm Hg; 0,65 kPa) à 80°C. On obtient ainsi 8,37 g de 8-formylamino-5H,10H-imidazo[1,2-a]indéno[1,2-e]pyrazine-4-one.

### EXEMPLE 58

A une suspension de 3 g de chlorhydrate de 8-amino-5H,10H-imidazo[1,2-a]indéno[1,2-e]pyrazine-4-one dans 30 ml de diméthylformamide, on ajoute goutte à goutte 2,70 ml de triéthylamine, puis 3,41 g de 2-carbométhoxyphénylisocyanate en solution dans 30 ml de diméthylformamide. Le milieu réactionnel est agité pendant une nuit à une température voisine de 20°C. Le précipité formé est filtré, lavé avec de l'eau distillée, de l'acétone et de l'éther diéthylique, puis séché sous pression réduite (1 mm Hg; 0,13 kPa) à 30°C. Le solide ainsi obtenu est repris par 20 ml de diméthylformamide, filtré, rincé avec 10 ml de diméthylformamide et séché sous pression réduite (1 mm Hg; 0,13 kPa) à 47°C. On obtient ainsi 2,45 g de 8-[3-(2-carbométhoxyphényl)uréido]-5H,10H-imidazo[1,2-a]indéno[1,2-e]pyrazine-4-one sous forme de solide beige dont le point de fusion est supérieur à 260°C (Analyse C22 H17 N5 O4; 0,26H20; 0,60DMF % calculé C : 63,61; H : 4,12; N : 16,86; O : 15,41; % trouvé C : 63,1; H : 4,4; N : 16,3; O : 14,9).

### EXEMPLE 59

A une suspension de 3 g de chlorhydrate de 8-amino-5H,10H-imidazo[1,2-a]indéno[1,2-e]pyrazine-4-one dans 30 ml de diméthylformamide, on ajoute goutte à goutte 2,70 ml de triéthylamine, puis 3,41 g de 3-carbométhoxyphénylisocyanate en solution dans 30 ml de diméthylformamide. Le milieu réactionnel est agité pendant une nuit à une température voisine de 20°C. Le précipité formé est filtré, lavé avec du diméthylformamide, de l'eau distillée, puis séché sous pression réduite (1 mm Hg; 0,13 kPa) à 50°C. Le solide ainsi obtenu est repris par 50 ml de diméthylformamide, filtré, rincé avec du diméthylformamide, de l'eau distillée et de l'acétone, puis séché sous pression réduite (1 mm Hg; 0,13 kPa) à 50°C. On obtient ainsi 1,80 g de 8-[3-(3-carbométhoxyphényl)uréido]-5H,10H-imidazo[1,2-a]indéno[1,2-e]pyrazine-4-one sous forme de solide violet dont le point de fusion est supérieur à 260°C (Analyse C22 H17 N5 O4; 0,80H20; 0,44DMF % calculé C : 63,61; H : 4,12; N : 16,86; O : 15,41; % trouvé C : 63,6; H : 4,0; N : 16,7; O : 15,4).

Le 3-carbométhoxyphénylisocyanate peut être obtenu selon la méthode décrite par R.KATAKAI, J.Org.Chem., 50,715 (1885).

### EXEMPLE 60

A une suspension de 1 g de 8-[3-(3-carbométhoxyphényl)uréido]-5H,10H-imidazo[1,2-a]indéno[1,2-e]pyrazine-4-one dans 35 ml d'éthanol, on ajoute goutte à goutte 1 ml de soude aqueuse à 30% à une température voisine de 20°C. La réaction est poursuivie 5 heures à 50 °C. Après refroisissement, le milieu réactionnel est concentré sous pression réduite (1 mm Hg; 0,13 kPa) à 45°C. On ajoute au solide ainsi obtenu 10 ml d'eau distillée et 15 ml d'acide chlorhydrique 1N. Après filtration, lavage au méthanol puis séchage sous pression réduite (1 mm Hg; 0,13 kPa) à 40°C, on obtient 0,9 g de 8-[3-(3-carboxyphényl)uréido]-5H,10H-imidazo[1,2-a]indéno[1,2-e]pyrazine-4-one, chlorhydrate sous forme de solide verdâtre dont le point de fusion est supérieur à 260°C (Analyse C21 H16 Cl1 N5 O4; 0,47H20 % calculé C : 57,61; H : 3,68; Cl : 8,10; N : 16,00; O : 14,62; % trouvé C : 57,3; H : 4,0; N : 15,7; O : 14,6).

### EXEMPLE 61

A une suspension de 3 g de chlorhydrate de 8-amino-5H,10H-imidazo[1,2-a]indéno[1,2-e]pyrazine-4-one dans 30 ml de diméthylformamide, on ajoute goutte à goutte 2,70 ml de triéthylamine, puis 3,68 g de 4-carbéthoxyphénylisocyanate en solution dans 30 ml de diméthylformamide. Le milieu réactionnel est agité pendant une nuit à une température voisine de 20°C. Le précipité formé est filtré, lavé avec du diméthylformamide, de l'eau distillée, de l'éther éthylique puis séché sous pression réduite (1 mm Hg; 0,13 kPa) à 42°C. Le solide ainsi obtenu est repris par de l'eau distillée, filtré, rincé avec de l'acétone, puis séché sous pression réduite (1 mm Hg; 0,13 kPa) à 45°C. On obtient ainsi 2,5 g de 8-[3-(4-carbéthoxyphényl)uréido]-5H,10H-imidazo[1,2-a]indéno[1,2-e]pyrazine-4-one sous forme de solide beige dont le point de fusion est supérieur à 260°C (Analyse C23 H19 N5 O4; 0,18H20; 0,51DMF % calculé C : 64,33; H : 4,46; N : 16,31; O : 14,90; % trouvé C : 64,0; H : 4,5; N : 15,5; O : 14,3).

### EXEMPLE 62

A une suspension de 1,74 g de 8-[3-(4-carboéthoxyphényl)uréido]-5H,10H-imidazo[1,2-a]indéno[1,2-e]pyrazine-4-one dans 55 ml d'éthanol, on ajoute goutte à goutte 1,7 ml de soude aqueuse à 30% à une température voisine de 20°C. La réaction est poursuivie 12 heures à 50 °C. Après refroisissement du milieu réactionnel, le précipité formé est filtré, lavé avec de l'éthanol, de l'acétone et séché sous pression ordinaire à 20°C. Le solide ainsi obtenu est repris par 20 ml d'eau distillée et acidifié par 10 ml d'acide chlorhydrique 1N. Après une heure d'agitation, le solide est filtré, lavé par de l'eau distillée, de l'acétone,du méthanol, puis séché sous pression réduite (1 mm Hg; 0,13 kPa) à 45°C. On obtient ainsi 0,5 g de 8-[3-(4-carboxyphényl)uréido]-5H,10H-imidazo[1,2-a]indéno[1,2-e]pyrazine-4-one, sous forme de solide ocre dont le point de fusion est supérieur à 260°C (Analyse C21 H15 N5 O4; 2,15H20 % calculé C : 62,84; H : 3,77; N : 17,45; O : 15,94; % trouvé C : 62,8; H : 3,5; N : 17,3).

### EXEMPLE 63

A une suspension de 3,58 g de chlorhydrate de 8-amino-5H,10H-imidazo[1,2-a]indéno[1,2-e]pyrazine-4-one dans 36 ml de diméthylformamide, on ajoute goutte à goutte 3,20 ml de triéthylamine, puis 6,16 g de 4-carbométhoxybenzylisocyanate en solution dans 36 ml de diméthylformamide. Le milieu réactionnel est agité pendant une nuit à une température voisine de 20°C. Le précipité formé est filtré, lavé avec de l'eau distillée, du diméthylformamide, de l'éther diéthylique, puis séché sous pression réduite (1 mm Hg; 0,13 kPa) à 30°C. Le solide ainsi obtenu est repris par 30 ml de méthanol et lavé par de l'éther diéthylique. Après séchage, le résidu est recristallisé dans 200 ml de diméthylformamide, on obtient ainsi 1,1 g de 8-[3-(4-carbométhoxybenzyl)uréido]-5H,10H-imidazo[1,2-a]indéno[1,2-e]pyrazine-4-one sous forme de solide beige dont le point de fusion est supérieur à 260°C (Analyse C23 H19 N5 O4; 0,82H20; 0,46DMF % calculé C : 64,33; H : 4,46; N : 16,31; O : 14,90; % trouvé C : 64,0; H : 4,8; N : 16,8; O : 14,3).

Le 4-carbométhoxybenzylisocyanate peut être obtenu selon la méthode décrite par R.KATAKAI, J.Org.Chem., 50,715 (1885).

### EXEMPLE 64

A une suspension de 1,5 g de 8-[3-(4-carbométhoxybenzyl)uréido]-5H,10H-imidazo[1,2-a]indéno[1,2-e]pyrazine-4-one dans 52 ml de méthanol, on ajoute goutte à goutte 3,9 ml de soude aqueuse à 30% à une température voisine de 20°C. La réaction est poursuivie 20 heures à 50 °C. Après refroisissement du milieu réactionnel, le précipité formé est filtré, lavé avec du méthanol, de l'acétone et séché sous pression ordinaire à 20°C. Le solide ainsi obtenu est repris par 20 ml d'eau distillée et acidifié par 4 ml d'acide chlorhydrique 6N. Après une heure d'agitation, le solide est filtré, lavé par de l'eau distillée, de l'éther diéthylique, puis séché sous pression réduite (1 mm Hg; 0,13 kPa) à 45°C. Le résidu est recristallisé dans 80 ml de diméthylformamide, on obtient ainsi 0,15g de 8-[3-(4-carboxybenzyl)uréido]-5H,10H-imidazo[1,2-a]indéno[1,2-e]pyrazine-4-one, sous forme de solide rose dont le point de fusion est supérieur à 260°C (Analyse C22 H17 N5 O4; 0,05H20; 2,30DMF % calculé C : 63,61; H : 4,12; N : 16,86; O : 15,41; % trouvé C : 63,3; H : 3,4; N : 16,8).

### EXEMPLE 65

A une suspension de 1,5 g de chlorhydrate de 8-amino-5H,10H-imidazo[1,2-a]indéno[1,2-e]pyrazine-4-one dans 15 ml de diméthylformamide, on ajoute goutte à goutte 1,35 ml de triéthylamine, puis 1,67 g de 2-fluorobenzylisocyanate en solution dans 15 ml de diméthylformamide. Le milieu réactionnel est agité pendant une nuit à une température voisine de 20°C. Le précipité formé est filtré, lavé avec du diméthylformamide, de l'eau distillée, de l'acétone et de l'éther diéthylique, puis séché sous pression réduite (1 mm Hg; 0,13 kPa) à 50°C.Le solide ainsi obtenu est repris par 20 ml de diméthylformamide, filtré, rincé avec 40 ml d'eau distillée et de l'éther diéthylique, puis séché sous pression réduite (1 mm Hg; 0,13 kPa) à 40°C. On obtient ainsi 0,45 g de 8-[3-(2-fluorobenzyl)uréido]-5H,10H-imidazo[1,2-a]indéno[1,2-e]pyrazine-4-one sous forme de solide violet dont le point de fusion est supérieur à 260°C (Analyse C21 H16 F1 N5 O2; 1,45H20; % calculé C : 64,78; H : 4,14; F : 4,88; N : 17,99; O : 8,22; % trouvé C : 64,7; H : 4,0; F : 4,2; N : 18,3).

Le 2-fluorobenzylisocyanate peut être obtenu selon la méthode décrite par R.KATAKAI, J.Org.Chem., 50,715 (1885).

### EXEMPLE 66

A une suspension de 2 g de chlorhydrate de 8-amino-5H,10H-imidazo[1,2-a]indéno[1,2-e]pyrazine-4-one dans 20 ml de diméthylformamide, on ajoute goutte à goutte 1,8 ml de triéthylamine, puis 3,88 g de 3-fluorobenzylisocyanate en solution dans 20 ml de diméthylformamide. Le milieu réactionnel est agité pendant une nuit à une température voisine de 20°C. Le précipité formé est filtré, lavé avec de l'eau distillée, de l'acétone et de l'éther diéthylique, puis séché sous pression réduite (1 mm Hg; 0,13 kPa) à 50°C. Le solide ainsi obtenu est repris par 20 ml de diméthylformamide, filtré, rincé avec 30 ml d'eau distillée et de l'oxyde diisopropylique, puis séché sous pression réduite (1 mm Hg; 0,13 kPa) à 40°C. Le résidu est recristallisé dans 20 ml de diméthylformamide, on obtient ainsi 0,38g de 8-[3-(3-fluorobenzyl)uréido]-5H,10H-imidazo[1,2-a]indéno[1,2-e]pyrazine-4-one sous forme de solide beige dont le point de fusion est supérieur à 260°C (Analyse C21 H16 F1 N5 O2; 0,56H20; 0,33DMF % calculé C : 64,78; H : 4,14; F : 4,88; N : 17,99; O : 8,22; % trouvé C : 64,9; H : 3,8; F : 4,4; N : 18,8).

Le 3-fluorobenzylisocyanate peut être obtenu selon la méthode décrite par R. KATAKAI, J. Org. Chem., 50,715 (1885).

### EXEMPLE 67

A une suspension de 1,5 g de chlorhydrate de 8-amino-5H,10H-imidazo[1,2-a]indéno[1,2-e]pyrazine-4-one dans 15 ml de diméthylformamide, on ajoute goutte à goutte 1,35 ml de triéthylamine, puis 1,67 g de 4-fluorobenzylisocyanate en solution dans 15 ml de diméthylformamide. Le milieu réactionnel est agité pendant une nuit à une température voisine de 20°C. Le précipité formé est filtré, lavé avec de l'eau distillée, de l'acétone et de l'éther diéthylique, puis séché sous pression réduite (1 mm Hg; 0,13 kPa) à 50°C. Le solide ainsi obtenu est repris par 20 ml de diméthylformamide, filtré, rincé avec de l'éther éthylique, de l'acétone, puis séché sous pression réduite (1 mm Hg; 0,13 kPa) à 40°C. Le résidu est recristallisé dans 15 ml de diméthylformamide, on obtient ainsi 0,2 g de 8-[3-(4-fluorobenzyl)uréido]-5H,10H-imidazo[1,2-a]indéno[1,2-e]pyrazine-4-one sous forme de solide gris dont le point de fusion est supérieur à 260°C (Analyse C21 H16 F1 N5 O2; 0,57H20; 0,93DMF % calculé C : 64,78; H : 4,14; F : 4,88; N : 17,99; O : 8,22; % trouvé C : 64,8; H : 3,9; F : 4,3; N : 18,3).

Le 4-fluorobenzylisocyanate peut être obtenu selon la méthode décrite par R. KATAKAI, J. Org. Chem., 50,715 (1885).

### EXEMPLE 68

0,24 g de 9-cyano-5H,10H-imidazo[1,2-a]indéno[1,2-e]pyrazine-4-one et 4 ml d'acide sulfurique concentré sont portés au reflux pendant 2 heures. Le milieu réactionnel est versé dans de l'eau glacée. Le précipité formé est filtré et séché sous pression réduite (1 mm Hg; 0,13 kPa) à 40°C. On obtient ainsi 240 mg de 9-carboxy-5H,10H-imidazo[1,2-a]indéno[1,2-e]pyrazine-4-one sous forme de solide beige dont le point de fusion est supérieur à 260°C (Analyse C14 H9 N3 O3; 1,32H20; 0,18H2SO4 % calculé C : 62,92; H : 3,39; N : 15,72; O : 17,96; % trouvé C : 62,5; H : 3,2; N : 15,5; O : 17,6).

La 9-cyano-5H,10H-imidazo[1,2-a]indéno[1,2-e]pyrazine-4-one peut être obtenue de la manière suivante : 1 g de 1-[2-(4-cyano-1-oxo-indanyl)]imidazole-2-carboxamide sont dissous dans 10 ml d'acide acétique et la solution est portée au reflux pendant 22 heures. Après refroidissement du milieu réactionnel, la suspension est filtrée, lavée par 5 ml d'acide acétique, de l'eau distillée et de l'éther éthylique. Le solide est séché sous pression réduite (15 mm Hg; 2kPa) à 30°C, on obtient ainsi 0,4 g de 9-cyano-5H,10H-imidazo[1,2-a]indéno[1,2-e]pyrazine-4-one fondant à une température supérieure à 260°C (Analyse C14 H8 N4 O1; 0,21H20 % calculé C : 67,74; H : 3,25; N : 22,57; O : 6,45; % trouvé C : 68,1; H : 3,3; N : 22,6; O : 6,7).

Le 1-[2-(4-cyano-1-oxo-indanyl)]imidazole-2-carboxamide peut être obtenu de la manière suivante : Une solution de 1,5 g de 1-[2-(4-cyano-1-oxo-indanyl)]imidazole-2-carboxylate d'éthyle dans 130 ml de méthanol est maintenue saturée pendant une heure à une température voisine de 20°C par un courant de gaz ammoniac. Le milieu réactionnel est ensuite concentré à sec sous pression réduite (15 mm Hg; 2kPa) à 50°C. On obtient ainsi 1,2 g de 1-[2-(4-cyano-1-oxo-indanyl)]imidazole-2-carboxamide fondant à 216°C.

Le 1-[2-(4-cyano-1-oxo-indanyl)]imidazole-2-carboxylate d'éthyle peut être obtenu de la manière suivante : Un mélange de 3,92 g d'imidazole-2-carboxylate d'éthyle et de 3,4 g de 2-bromo-4-cyano-1-indanone est chauffé à 130°C pendant 20 minutes, refroidi à 20°C et dissous dans 20 ml de dichlorométhane. Le milieu est ensuite concentré à sec sous pression réduite (15 mm Hg; 2kPa) à 40°C. Le résidu ainsi obtenu est purifié par flashchromatographie sur colonne de silice, sous courant d'azote à moyenne pression (0,5 bar) avec un mélange dichlorométhane-méthanol (95-5 en volumes) comme éluant. On obtient ainsi 1,5 g de 1-[2-(4-cyano-1-oxo-indanyl)]imidazole-2-carboxylate d'éthyle fondant à 159°C.

La 2-bromo-4-cyano-1-indanone peut être obtenue de la manière suivante : Une solution de 6,9 g de 4-cyano-1-indanone et de 95 ml de chloroforme est refroidie à 5°C. Une solution de 7,04 g de brome et de 20 ml de chloroforme est alors ajoutée goutte à goutte en deux heures à une température comprise entre 0 et 5°C. Après avoir laissé le milieu réactionnel sous agitation pendant une heure en conservant la température d'introduction, on laisse revenir la solution à température ambiante et on agite encore une nuit. Le milieu réactionnel est ensuite concentré à sec sous pression réduite (15 mm Hg; 2kPa) à 40°C. Le résidu ainsi obtenu est purifié par flash-chromatographie sur colonne de silice, sous courant d'azote à moyenne pression (0,5 bar) avec un mélange éther éthylique-cyclohexane (20-80 en volumes) comme éluant. On obtient ainsi 3,43 g de 2-bromo-4-cyano-1-indanone fondant à 124°C.

La 4-cyano-1-indanone peut être préparée de la manière suivante : A une solution de 10 g de 4-bromo-1-indanone dans 94 ml de diméthylformamide, on ajoute 12,61 g de cyanure de cuivre, puis le milieu réactionnel est porté au reflux pendant 6 heures et agité à une température voisine de 20°C pendant une nuit. La solution est ensuite versée dans une solution aqueuse de cyanure de sodium (5%), agitée pendant 15 minutes, et extraite par trois fois 500 ml d'acétate d'éthyle. Les phases organiques sont séchées sur sulfate de sodium et concentrées sous pression réduite (15 mm Hg; 2kPa) à 40°C. Le résidu ainsi obtenu est purifié par flash-chromatographie sur colonne de silice, sous courant d'azote à moyenne pression (0,5 bar) avec un mélange acétate d'éthyle-cyclohexane (20-80 en volumes) comme éluant. On obtient ainsi 5,9 g de 4-cyano-1-indanone fondant à 122°C.

La 4-bromo-1-indanone peut être préparée comme décrit par F.G. HOLLIMAN, F.G. MANNE et D.A. THORNTON, J. Chem. Soc.,9 (1960).

### EXEMPLE 69

0,5 g de 9-carboxy-5H,10H-imidazo[1,2-a]indéno[1,2-e]pyrazine-4-one et 14,2 ml de chlorure de thionyle sont portés au reflux pendant 3 heures. Après refroidissement de la masse réactionnelle et distillation de l'excès de chlorure de thionyle, 20 ml de toluène sont ajoutés. A une température voisine de 5°C, on fait barboter dans la solution de l'ammoniac gazeux pendant 15 minutes. Après avoir laisser agiter pendant une nuit à une température voisine de 20°C, on fait barboter à nouveau de l'ammoniac gazeux pendant 15 minutes et on porte le milieu réactionnel au reflux pendant deux heures. Après refroidissement , le milieu réactionnel est concentré sous pression réduite (1 mm Hg; 0,13 kPa) à 40°C. Le résidu est repris par 10 ml d'eau distillée, filtré et lavé par de l'eau distillée et par de l'acétone. Le solide ainsi obtenu est repris par 10 ml d'eau distillée, 5 ml d'acide chlorhydrique concentré et est agité pendant une nuit à une température voisine de 20°C. Le précipité formé est filtré, lavé par 40 ml d'éther éthylique chlorhydrique et séché sous pression réduite (1 mm Hg; 0,13 kPa) à 40°C. On obtient ainsi 0,27 g de 9-carboxamido-5H,10H-imidazo[1,2-a]indéno[1,2-e]pyrazine-4-one, chlorhydrate sous forme de solide marron dont le point de fusion est supérieur à 260°C (Analyse C14 H11 Cl1 N4 O2; 0,77H20; % calculé C : 55,55; H : 3,66; Cl: 11,71; N : 18,51; O : 10,57; % trouvé C : 52,2; H : 3,9; Cl: 11,1; N : 17,3).

### EXEMPLE 70

0,4 g de 9-carboxy-5H,10H-imidazo[1,2-a]indéno[1,2-e]pyrazine-4-one et 3,2 ml de chlorure de thionyle sont portés au reflux pendant 2 heures. Après refroidissement de la masse réactionnelle et distillation de l'excès de chlorure de thionyle, 10 ml de toluène sont ajoutés. A une température voisine de 20 C, on ajoute goutte à goutte 2 ml de diéthylamine et on porte le milieu réactionnel au reflux pendant trois heures. Après refroidissement , le milieu réactionnel est repris par 10 ml d'eau distillée et agité pendant une heure. Le précipité formé est filtré, lavé avec de l'eau distillée et de l'éther éthylique. Le solide ainsi obtenu est recristallisé dans 2 ml de diméthylformamide, on obtient ainsi 0,10 g de 9-(N-diéthylcarboxamido)-5H,10H-imidazo[1,2-a]indéno[1,2-e]pyrazine-4-one sous forme de solide rose dont le point de fusion est supérieur à 260°C (Analyse C18 H18 N4 O2; 1,33H20; % calculé C : 67,07; H : 5,63; N : 17,38; O : 9,93; % trouvé C : 67,8; H : 5,5; N : 17,7).

### EXEMPLE 71

0,6 g de 9-carboxy-5H,10H-imidazo[1,2-a]indéno[1,2-e]pyrazine-4-one et 10 ml de chlorure de thionyle sont portés au reflux pendant 1 heure et 30 minutes. Après refroidissement de la masse réactionnelle et distillation de l'excès de chlorure de thionyle, 20 ml de toluène sont ajoutés. A une température voisine de 20 °C, on ajoute goutte à goutte 20 ml d'une solution toluènique d'éthylamine (10 %). Après avoir laisser agiter pendant une nuit à une température voisine de 20°C, le milieu réactionnel est concentré sous pression réduite (1 mm Hg; 0,13 kPa) à 55°C. Le résidu est repris par 10 ml d'eau distillée, filtré et lavé par de l'eau distillée et par de l'éthanol. Le solide ainsi obtenu est recristallisé dans 10 ml de diméthylformamide, filtré, lavé par de l'oxyde diisopropylique et séché sous pression réduite (1 mm Hg; 0,13 kPa) à 50°C. On obtient ainsi 0,17 g de 9-(N-éthylcarboxamido)-5H,10H-imidazo[1,2-a]indéno[1,2-e]pyrazine-4-one sous forme de solide beige dont le point de fusion est supérieur à 260°C (Analyse C16 H14 N4 O2; 0,88H20; % calculé C : 65,30; H : 4,79; N : 19,04; O : 10,87; % trouvé C : 64,7; H : 4,5; N : 18,7).

### EXEMPLE 72

A une suspension de 1 g de 9-amino-5H,10H-imidazo[1,2-a]indéno[1,2-e]pyrazine-4-one dans 55 ml de diméthylformamide sont ajoutés 3,5 g de p-nitrophényl-N-méthylcarbamate. Après 4 heures de chauffage au reflux, le mélange réactionnel est versé dans 500 ml d'eau glacée. La solution obtenue est extraite trois fois avec 150 ml de dichlorométhane, puis elle est évaporée. Le résidu est mis en suspension dans 50 ml d'acide chlorhydrique 0,5 N, et il est filtré. On obtient ainsi 62 mg de 9-(3-méthyluréido)-5H,10H-imidazo[1,2-a]indéno[1,2-e]pyrazine-4-one sous forme d'un solide beige fondant au-dessus de 260°C [Spectre de R.M.N.¹H (250 MHz, (CD₃)₂SO d6, δ en ppm) : 2,70 (s, 3H : NCH₃) ; 4,01 (s, 2H : CH₂ 10) ; 6,56 (mf, 1H : CONH) ; 7,37 (t, J = 8 Hz, 1H : H 7) ; 7,60 et 7,78 (2 d larges, 1H chacun : H 6 et H 8) ; 8,00 et 8,17 (2 s larges, 1H chacun : H de l'imidazole) ; 8,54 (s large, 1H : ArNHCO) ; 13,07 (mf, 1H : NH 5)].

Le p-nitrophényl-N-méthylcarbamate peut être préparé comme décrit par T. KONAKAHARA et coll., Synthesis, 103 (1993).

### EXEMPLE 73

A une suspension de 1,5 g de 8-amino-5H,10H-imidazo[1,2-a]indéno[1,2-e]pyrazine-4-one dans 15 ml de 1,4-dioxanne maintenue sous azote, on ajoute 4,1 g d'isocyanate de p-méthoxybenzyle en solution dans 15 ml de 1,4-dioxanne. Après 18 heures d'agitation à une température voisine de 20°C, le mélange réactionnel est filtré sur verre fritté. Le solide est trituré avec 100 ml d'acide chlorhydrique 0,1 N. Le surnageant est éliminé, et le résidu est mis en suspension dans 200 ml de méthanol. Le mélange est chauffé au reflux pendant 15 heures, puis il est filtré. On obtient ainsi 220 mg de 8-[3-(4-méthoxybenzyl)-uréido]-5H,10H-imidazo[1,2-a]indéno[1,2-e]pyrazine-4-one sous forme d'un solide marron fondant au-dessus de 260°C [Spectre de R.M.N.¹H (250 MHz, (CD₃)₂SO d6, δ en ppm) : 3,75 (s, 2H : CH₂ 10) ; 4,00 (s, 3H : OCH₃) ; 4,26 (d, J = 6 Hz, 2H : ArCH₂N) ; 678 (t, J = 6 Hz, 1H : NHCO) ; 6,90 (d, J = 8 Hz, 2H : H aromatiques en ortho du OCH₃) ; 7,00 (d large, J = 8 Hz, 1H : H 7) ; 7,22 - 7,75 et 7,88 (3 s larges, 1H chacun : H de l'imidazole et H 9) ; 7,28 (d, J = 8 Hz, 2H : H aromatiques en méta du OCH₃) ; 7,32 (d, J = 8 Hz, 1H : H 6) ; 9,00 (mf, 1H : ArNH) ; 12,64 (mf, 1H : NH 5)].

L'isocyanate de p-méthoxybenzyle peut être préparé selon la méthode décrite par R.KATAKAI, J.Org.Chem., 50,715 (1985).

### EXEMPLE 74

A une suspension de 1 g de 8-amino-5H,10H-imidazo[1,2-a]indéno[1,2-e]pyrazine-4-one dans 20 ml de diméthylformamide maintenue sous azote, on ajoute 1,2 ml de triéthylamine, puis 0,65 ml de chlorure de méthanesulfonyle. Après 15 heures d'agitation à une température voisine de 20°C, on additionne à nouveau 0,32 ml de chlorure de méthanesulfonyle. La réaction est poursuivie pendant 4 heures à la même température. Le mélange réactionnel est alors filtré sur verre fritté et rincé successivement avec 10 ml de diméthylformamide et deux fois 10 ml d'eau distillée. On obtient ainsi 600 mg de 8-méthylsulfonamido-5H,10H-imidazo[1,2-a]indéno[1,2-e]pyrazine-4-one sous forme d'un solide marron fondant au-dessus de 260°C [Spectre de R.M.N.¹H (400 MHz, (CD₃)₂SO d6, δ en ppm) : 3,02 (s, 3H : SO₂CH₃) ; 4,02 (s, 2H : CH₂ 10) ; 7,25 (dd, J = 8 et 2 Hz, 1H : H 7) ; 7,45 (s large, 1H : H 9) ; 7,68 et 7,95 (2 s, 1H chacun : H de l'imidazole) ; 7,82 (d, J = 8 Hz, 1H : H 6) ; 9,83 (mf, 1H : ArNH) ; 12,34 (mf, 1H : NH 5)].

### EXEMPLE 75

A une solution de 0,5 g de 8-amino-5H,10H-imidazo[1,2-a]indéno[1,2-e]pyrazine-4-one dans 15 ml de 1,3-diméthyl-2-imidazolidinone maintenue sous azote, on ajoute 0,6 ml de triéthylamine puis 0,45 ml de chlorure de diméthylsulfamoyle. Après 15 heures d'agitation à une température voisine de 20°C, on additionne à nouveau 0,6 ml de triéthylamine puis 0,22 ml de chlorure de diméthylsulfamoyle. La réaction est poursuivie pendant 15 heures à la même température. Le mélange réactionnel est alors filtré sur verre fritté. Le filtrat est additionné de 150 ml d'acétate d'éthyle et le précipité est recueilli sur verre fritté. Le solide est cristallisé dans un mélange de 30 ml de 1,4-dioxanne et de 20 ml d'eau distillée. On obtient ainsi 120 mg de 8-(N,N-diméthylaminosulfonamido)-5H,10H-imidazo[1,2-a]indéno[1,2-e]pyrazine-4-one sous forme d'un solide marron fondant au-dessus de 260°C [Spectre de R.M.N.¹H (300 MHz, (CD₃)₂SO d6, δ en ppm) : 2,72 (s, 6H : SO₂N(CH₃)₂); 4,02 (s, 2H : CH₂ 10) ; 7,25 (d large, J = 8 Hz, 1H : H 7) ; 7,42 (s large, 1H : H 9) ; 7,73 et 8,05 (2 s, 1H chacun : H de l'imidazole) ; 7,79 (d, J = 8 Hz, 1H : H 6) ; 10,05 (mf, 1H : ArNH) ; 12,64 (mf, 1H : NH 5)].

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile doive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischémie cérébrale, les effets dus à une anoxie, l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON, vis-à-vis des manifestations épileptogènes et/ou convulsives, pour le traitement des traumatismes cérébraux et spinaux, des traumatismes liés à le dégénérescence de l'oreille interne ou de la rétine, de l'anxiété, de la dépression, de la schizophrénie, du syndrome de TOURETTE, de l'encéphalopathie hépatique, en tant qu'analgésiques, antiinflammatoires, antianorexiques, antimigraineux, antiémétiques et pour traiter les empoisonnements par des neurotoxines ou d'autres substances agonistes du récepteur NMDA, ainsi que les troubles neurologiques associés aux maladies virales telles que le sida, la rage, la rougeole et le tétanos. Ces composés sont aussi utiles pour la prévention des symptomes d'abstinence aux drogues et à l'alcool et de l'inhibition de l'accoutumance et de la dépendance aux opiacés ainsi que pour le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :

| | |
|---|---|
| - Composé de formule (I) | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

### EXEMPLE B

On prépare selon la technique habituelle des comprimés dosés à 50 mg de produit actif ayant la composition suivante :

| | |
|---|---|
| - Composé de formule (I) | 50 mg |
| - Lactose | 104 mg |
| - Cellulose | 40 mg |
| - Polyvidone | 10 mg |
| - Carboxyméthylamidon sodique | 22 mg |
| - Talc | 10 mg |
| - Stéarate de magnésium | 2 mg |
| - Silice colloïdale | 2 mg |
| - Mélange d'hydroxyméthylcellulose, glycérine, oxyde de titane (72-3,5-24,5) q.s.p. 1 comprimé pelliculé terminé à | 245 mg |

### EXEMPLE C

On prépare une solution injectable contenant 10 mg de produit actif ayant la composition suivante :

| | |
|---|---|
| - Composé de formule (I) | 10 mg |
| - Acide benzoïque | 80 mg |
| - Alcool benzylique | 0,06 ml |
| - Benzoate de sodium | 80 mg |
| - Ethanol à 95 % | 0,4 ml |
| - Hydroxyde de sodium | 24 mg |
| - Propylène glycol | 1,6 ml |
| - Eau q.s.p. | 4 ml |

## Revendications

1. Composés de formule : 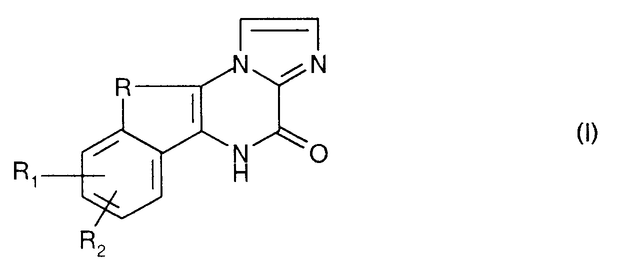 dans laquelle
- R représente un radical C=R₃ ou CH-R₆,
- R₁ représente un radical hydroxy, polyfluoroalcoxy, carboxy, -NH-CHO, -N(alk)-CO-NR₈R₉, -NH-CO-NR₉R₁₂, -NH-CS-NR₈R₉, -NH-CO-R₁₀, -NH-SO₂-NR₇R₉, -CO-NR₇R₉, -NH-SO₂-alk, -NR₉R₁₁, -S-alk-Ar, -SO₂-NR₇R₉, 2-oxo-1-imidazolidinyle,
- R₂ représente un atome d'hydrogène,
- R₃ représente un atome d'oxygène ou un radical NOH,
- R₆ représente un radical atome d'hydrogène ou un radical amino,
- R₇ représente un atome d'hydrogène ou un radical alkyle,
- R₈ représente un atome d'hydrogène ou un radical alkyle, -alk-NR₉R₇ ou phényle,
- R₉ représente un atome d'hydrogène ou un radical alkyle,
- R₁₀ représente un radical alkyle (5-9 C en chaîne droite ou ramifiée), alcoxy, -alk-COOR₂₁, -alk-NR₉R₇, phénylalkyle, phényle ou un hétérocycle choisi parmi pyrrolidine, azétidine et morpholine,
- R₁₁ représente un hétérocycle choisi parmi imidazoline et thiazoline,
- R₁₂ représente un atome d'hydrogène ou un radical alkyle, -alk-COOR₂₁, phénylalkyle dont le noyau phényle est éventuellement substitué par un ou plusieurs substituants choisis parmi les atomes d'halogène et les radicaux alcoxy et -COOR₂₁ et phényle substitué par un ou plusieurs substituants choisis parmi les atomes d'halogène et les radicaux alcoxy, nitro, amino, -COOR₂₁ et cyano,
- R₂₁ représente un atome d'hydrogène ou un radical alkyle,
- Ar représente un radical phényle,
- alk représente un radical alkyle ou alkylène,
étant entendu que, sauf menton contraire, les radicaux et portions alkyle et alcoxy contiennent 1 à 6 atomes de carbone en chaîne droite ou ramifiée,
les isomères E et Z des composés pour lesquels R₃ représente un radical NOH, les énantiomères et diastéréoisomères des composés pour lesquels R représente CH-R₆.
ainsi que les sels de ces composés.

2. Les composés de formule (I) selon la revendication 1 suivants :
- 9-phénylacétamido-5H,10H-imidazo[1,2-a]indéno[1,2-e]pyrazine-4-one,
- 8-éthoxycarbonylamino-5H,10H-imidazo[1,2-a]indéno[1,2-e]pyrazine-4-one,
- 8-[3-(3-cyanophényl)uréido]-5H,10H-imidazo[1,2-a]indéno[1,2-e]pyrazine-4-one,
- 8-[3-(3-méthoxyphényl)uréido]-5H,10H-imidazo[1,2-a]indéno[1,2-e]pyrazine-4-one,
- 8-phénylacétamido-5H,10H-imidazo[1,2-a]indéno[1,2-e]pyrazine-4-one,
- 8-(3-phénéthyluréido)-5H,10H-imidazo[1,2-a]indéno[1,2-e]pyrazine-4-one,
- 8-(3-méthyluréido)-5H,10H-imidazo[1,2-a]indéno[1,2-e]pyrazine-4-one,
- 8-(3-benzyluréido)-5H,10H-imidazo[1,2-a]indéno[1,2-e]pyrazine-4-one,
- 8-(3-tert-butyluréido)-5H,10H-imidazo[1,2-a]indéno[1,2-e]pyrazine-4-one,
- 8-(3-phénylpropionamido)-5H,10H-imidazo[1,2-a]indéno[1,2-e]pyrazine-4-one,
- 8-benzamido-5H,10H-imidazo[1,2-a]indéno[1,2-e]pyrazine-4-one,
- 8-(4-phénylbutyrylamino-5H,10H-imidazo[1,2-a]indéno[1,2-e]pyrazine-4-one,
- 8-(5-phénylvalérylamino-5H,10H-imidazo[1,2-a]indéno[1,2-e]pyrazine-4-one,
- 8-(3-éthoxycarbonylméthyluréido)-5H,10H-imidazo[1,2-a]indéno[1,2-e]pyrazine-4-one,
- 8-(3-carboxyméthyluréido)-5H,10H-imidazo[1,2-a]indéno[1,2-e]pyrazine-4-one,
- 8-(3,3-diméthyluréido)-5H,10H-imidazo[1,2-a]indéno[1,2-e]pyrazine-4-one,
- 8-hydroxy-5H,10H-imidazo[1,2-a]indéno[1,2-e]pyrazine-4-one,
- 8-(3-aminopropionamido-5H,10H-imidazo[1,2-a]indéno[1,2-e]pyrazine-4-one,
- 8-aminoacétamido-5H,10H-imidazo[1,2-a]indéno[1,2-e]pyrazine-4-one,
- 8-[3-(3-nitrophényl)uréido]-5H,10H-imidazo[1,2-a]indéno[1,2-e]pyrazine-4-one,
- 8-[3-(2-méthoxyphényl)uréido]-5H,10H-imidazo[1,2-a]indéno[1,2-e]pyrazine-4-one,
- 8-[3-(2-nitrophényl)uréido]-5H,10H-imidazo[1,2-a]indéno[1,2-e]pyrazine-4-one,
- 8-[3-(4-aminophényl)uréido]-5H,10H-imidazo[1,2-a]indéno[1,2-e]pyrazine-4-one,
- 8-[3-(4-méthoxyphényl)uréido]-5H,10H-imidazo[1,2-a]indéno[1,2-e]pyrazine-4-one,
- 8-(4-méthylpentanoyl)amino-5H,10H-imidazo[1,2-a]indéno[1,2-e]pyrazine-4-one,
- N,N-diméthyl-4-oxo-4,5-dihydro-10H-imidazo[1,2-a]indéno[1,2-e]pyrazine-8-sulfonamide,
- 8-(3-phénylthiouréido)-5H,10H-imidazo[1,2-a]indéno[1,2-e]pyrazine-4-one,
- 8-(3-méthylthiouréido)-5H,10H-imidazo[1,2-a]indéno[1,2-e]pyrazine-4-one,
- 8-(2-oxo-1-imidazolinyl)-5H,10H-imidazo[1,2-a]indéno[1,2-e]pyrazine-4-one,
- 8-formamido-5H,10H-imidazo[1,2-a]indéno[1,2-e]pyrazine-4-one,
- 8-(3-éthoxycarbonylpropionylamino)-5H,10H-imidazo[1,2-a]indéno[1,2-e] pyrazine-4-one,
- 8-[3-(2-éthoxycarbonyléthyl)uréido]-5H,10H-imidazo[1,2-a]indéno[1,2-e]pyrazine-4-one,
- 8-[3-(2-carboxyéthyl)uréido]-5H,10H-imidazo[1,2-a]indéno[1,2-e]pyrazine-4-one,
- 8-[3-(4-fluorophényl)uréido]-5H,10H-imidazo[1,2-a]indéno[1,2-e]pyrazine-4-one,
- 8-[3-(3-fluorophényl)uréido]-5H,10H-imidazo[1,2-a]indéno[1,2-e]pyrazine-4-one,
- 8-[3-(2-fluorophenyl)uréido]-5H,10H-imidazo[1,2-a]indéno[1,2-e]pyrazine-4-one,
- 8-(3-éthyluréido)-5H,10H-imidazo[1,2-a] indéno[1,2-e]pyrazine-4-one,
- 8-[3-morpholino-uréido]-5H,10H-imidazo[1,2-a]indéno[1,2-e]pyrazine-4-one,
- 10-amino-8-(3-méthyluréido)-5H,10H-imidazo[1,2-a]indéno[1,2-e]pyrazine-4-one,
- 10-hydroxyimino-8-(3-méthyluréido)-5H,10H-imidazo[1,2-a]indéno[1,2-e]pyrazine-4-one,
- 8-(3-méthyluréido)-5H-imidazo[1,2-a]indéno[1,2-e]pyrazine-4,10-dione,
- 8-[3-(3-aminophényl)uréido]-5H,10H-imidazo[1,2-a]indéno[1,2-e]pyrazine-4-one,
- 8-[(1-azétidinyl)carbonylamino]-5H,10H-imidazo[1,2-a]indéno[1,2-e]pyrazine-4-one,
- 8-(3-propylureido)-5H,10H-imidazo[1,2-a]indéno[1,2-e]pyrazine-4-one,
- 8-(3-isopropyluréido)-5H,10H-imidazo[1,2-a]indéno[1,2-e]pyrazine-4-one,
- 8-(3-butyluréido)-5H,10H-imidazo[1,2-a]indeno[1,2-e]pyrazine-4-one,
- 8-[(2-thiazolin-2-yl)amino]-5H,10H-imidazo[1,2-a]indéno[1,2-e]pyrazine-4-one,
- 8-[3-(3-carbométhoxyphényl)uréido]-5H,10H-imidazo[1,2-a]indéno[1,2-e]pyrazine-4-one,
- 8-[3-(4-carbométhoxybenzyl)uréido]-5H,10H-imidazo[1,2-a]indéno[1,2-e]pyrazine-4-one,
- 8-[3-(4-carboxybenzyl)uréido]-5H,10H-imidazo[1,2-a]indéno[1,2-e]pyrazine-4-one,
- 8-[3-(2-fluorobenzyl)uréido]-5H,10H-imidazo[1,2-a]indéno[1,2-e]pyrazine-4-one,
- 8-[3-(3-fluorobenzyl)uréido]-5H,10H-imidazo[1,2-a]indéno[1,2-e]pyrazine-4-one,
- 8-[3-(4-fluorobenzyl)uréido]-5H,10H-imidazo[1,2-a]indéno[1,2-e]pyrazine-4-one,
- 9-(N-éthylcarboxamido)-5H,10H-imidazo[1,2-a]indéno[1,2-e]pyrazine-4-one,
- 9-(3-méthyluréido)-5H,10H-imidazo[1,2-a]indéno[1,2-e]pyrazine-4-one,
- 8-[3-(4-méthoxybenzyl)-uréido]-5H,10H-imidazo[1,2-a]indéno[1,2-e]pyrazine-4-one,
et leurs sels.

3. Procédé de préparation des composés de formule (I) selon la revendication 1 pour lesquels R représente un radical C=R₃ dans lequel R₃ représente un atome d'oxygène caractérisé en ce que l'on hydrolyse un composé de formule (I) correspondant pour lequel R représente un radical C=R₃ et R₃ représente un radical NOH, isole le produit et le transforme éventuellement en sel.

4. Procédé de préparation des composés de formule (I) selon la revendication 1 pour lesquels R représente un radical C=R₃ et R₃ représente un radical NOH caractérisé en ce que l'on fait réagir un nitrite d'alkyle sur un composé de formule (I) correspondant pour lequel R représente un radical CH-R₆ et R₆ représente un atome d'hydrogène, isole le produit et le transforme éventuellement en sel.

5. Procédé de préparation des composés de formule (I) selon la revendication 1 pour lesquels R représente un radical CH-R₆ et R₆ représente un atome d'hydrogène caractérisé en ce que l'on désalkyle et désalifie un dérivé de formule : 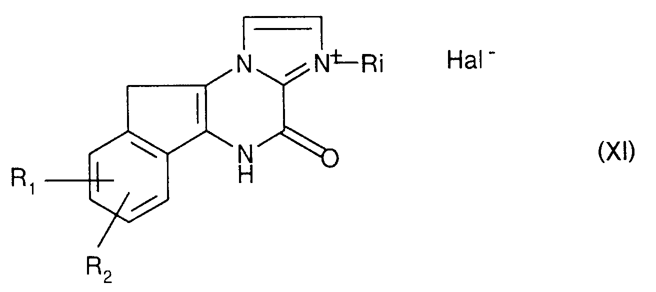 dans laquelle R₁ et R₂ ont les mêmes significations que dans la revendication 1, Ri représente un radical alkyle et Hal représente un atome d'halogène, isole le produit et le transforme éventuellement en sel.

6. Procédé de préparation des composés de formule (I) selon la revendication 1 pour lesquels R représente un radical CH-R₆ et R₆ représente un atome d'hydrogène caractérisé en ce que l'on cyclise éventuellement en présence d'acétate d'ammonium un dérivé de formule : 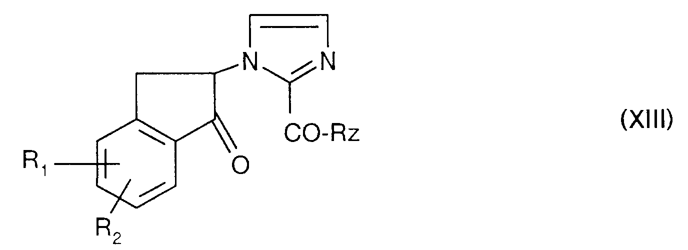 dans laquelle R₁ et R₂ ont les mêmes significations que dans la revendication 1 et Rz représente un radical -NH₂ ou -Oalk dans lequel alk représente un radical alkyle, isole le produit et le transforme éventuellement en sel.

7. Procédé de préparation des composés de formule (I) selon la revendication 1 pour lesquels R représente un radical CH-R₆ dans lequel R₆ représente un radical amino caractérisé en ce que l'on hydrolyse un composé de formule : 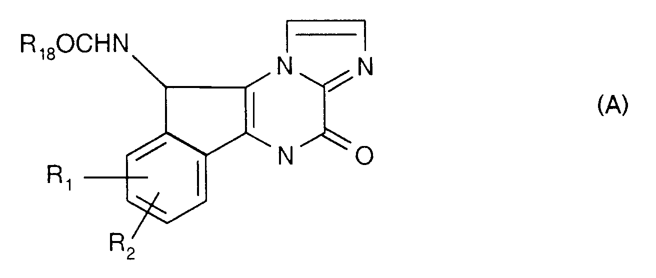 dans laquelle R₁ et R₂ ont les mêmes significations que dans la revendication 1, et R₁₈ représente un radical alkyle, isole le produit et le transforme éventuellement en sel.

8. Procédé de préparation des composés de formule (I) selon la revendication 1 pour lesquels R représente un radical CH-R₆ dans lequel R₆ représente un radical amino caractérisé en ce que l'on réduit un composé de formule (I) correspondant pour lequel R représente un radical C=R₃ dans lequel R₃ représente un radical NOH, isole le produit et le transforme éventuellement en sel.

9. Procédé de préparation des composés de formule (I) selon la revendication 1 pour lesquels R₁ représente un radical hydroxy caractérisé en ce que l'on hydrolyse un dérivé de formule : 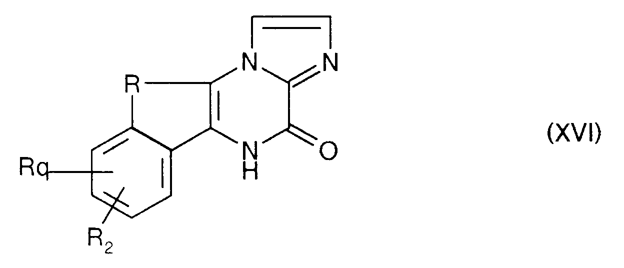 dans laquelle R et R₂ ont les mêmes significations que dans la revendication 1 et Rq représente un radical alcoxy, isole le produit et le transforme éventuellement en sel.

10. Procédé de préparation des composés de formule (I) selon la revendication 1 pour lesquels R₁ représente un radical carboxy caractérisé en ce que l'on hydrolyse un dérivé de formule : 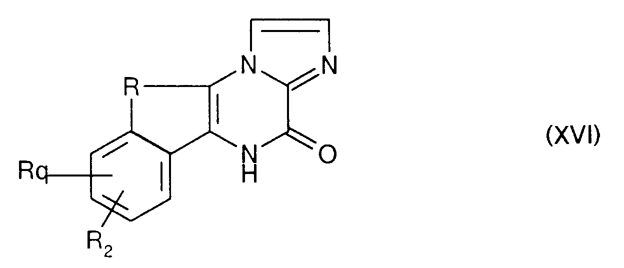 pour lequel R et R₂ ont les mêmes significations que dans la revendication 1 et Rq représente un radical cyano, isole le produit et le transforme éventuellement en sel.

11. Procédé de préparation des composés de formule (I) selon la revendication 1 pour lesquels R₁ représente un radical -NHCHO caractérisé en ce que l'on fait réagir un composé de formule : 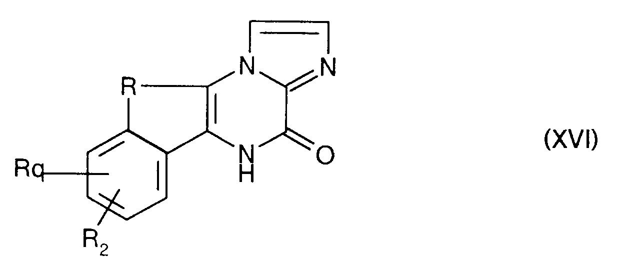 pour lequel R et R₂ ont les mêmes significations que dans la revendication 1 et Rq représente un radical amino, sur H₃C-COOCHO, isole le produit et le transforme éventuellement en sel.

12. Procédé de préparation des composés de formule (I) selon la revendication 1 pour lesquels R₁ représente un radical -N(alk)-CO-NR₈R₉ ou -NH-CSNR₈R₉ pour lequel R₈ et R₉ ne sont pas des atomes d'hydrogène, -NH-CO-NR₉R₁₂ pour lequel R₉ et R₁₂ ne sont pas des atomes d'hydrogène, -NH-COR₁₀, -NH-SO₂-NR₇R₉ pour lequel R₇ est un radical alkyle ou -NH-SO₂-alk caractérisé en ce que l'on fait réagir un composé de formule : 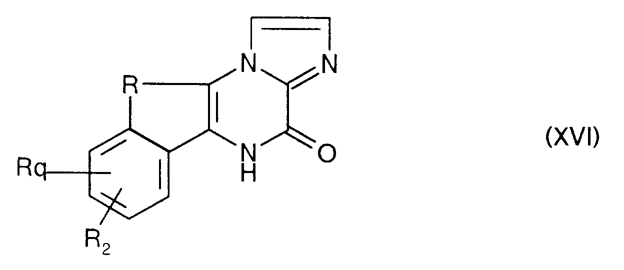 pour lequel R et R₂ ont les mêmes significations que dans la revendication 1 et Rq représente un radical -NH₂ ou -NH-alk dans lequel alk a les mêmes significations que dans la revendication 1, sur un dérivé de formule Hal-Rr dans laquelle Hal représente un atome d'halogène, Rr représente un radical -CO-NR₈R₉,-CSNR₈R₉ pour lesquels R₈ et R₉ ont les mêmes significations que dans la revendication 1 à l'exception d'hydrogène, -CO-NR₉R₁₂ pour lequel R₉ et R₁₂ ont les mêmes significations que dans la revendication 1 à l'exception d'hydrogène, -COR₁₀ dans lequel R₁₀ a les mêmes significations que dans la revendication 1, -SO₂-NR₇R₉ dans lequel R₉ a les mêmes significations que dans la revendication 1 et R₇ est un radical alkyle ou -SO₂-alk dans lequel alk a les mêmes significations que dans la revendication 1, isole le produit et le transforme éventuellement en sel.

13. Procédé de préparation des composés de formule (I) selon la revendication 1 pour lesquels R₁ représente un radical -N(alk)-CO-NR₈R₉ ou -NH-CO-NR₉R₁₂, caractérisé en ce que l'on fait réagir un dérivé de formule : 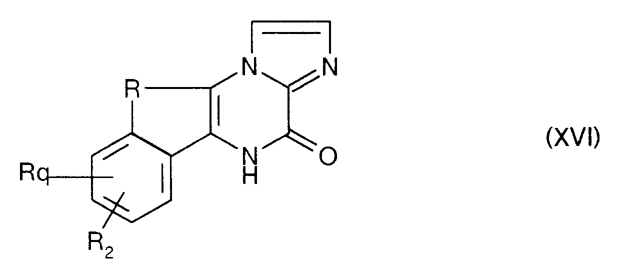 pour lequel R et R₂ ont les mêmes significations que dans la revendication 1 et Rq représente un radical -NH₂ ou -NH-alk dans lequel alk a les mêmes significations que dans la revendication 1, sur un halogénotrialkylsilane en présence d'un hydrure de métal alcalin, puis un agent de couplage et une amine de formule HNR₈R₉ ou HN-R₉R₁₂ dans laquelle R₈, R₉ et R₁₂ ont les mêmes significations que dans la revendication 1, isole le produit et le transforme éventuellement en sel.

14. Procédé de préparation des composés de formule (I) selon la revendication 1 pour lesquels R₁ représente un radical -NH-CO-NR₉R₁₂ ou -N(alk)-CO-NR₈R₉ caractérisé en ce que l'on fait réagir un dérivé de formule : 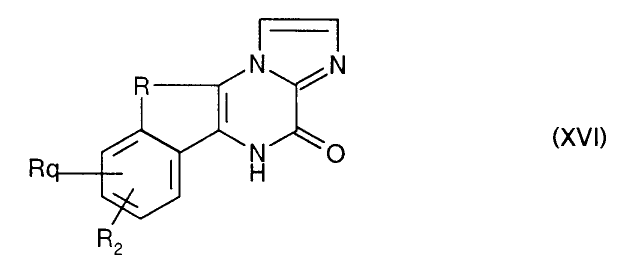 pour lequel R et R₂ ont les mêmes significations que dans la revendication 1 et Rq représente un radical -NH₂ ou -NH-alk pour lequel alk a les mêmes significations que dans la revendication 1 sur un dérivé de formule : 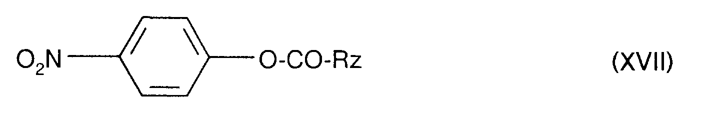 dans laquelle Rz représente un radical -NR₉R₁₂ ou -NR₈R₉, R₇, R₈ et R₁₂ ayant les mêmes significations que dans la revendication 1, isole le produit et le transforme éventuellement en sel.

15. Procédé de préparation des composés de formule (I) selon la revendication 1 pour lesquels R₁ représente un radical -NH-CONR₉R₁₂, R₉ représente un atome d'hydrogène et R₁₂ représente un radical phényle substitué par un radical amino caractérisé en ce que l'on réduit un composé de formule (I) correspondant pour lequel R₁ représente un radical -NH-CONR₉R₁₂, R₉ représente un atome d'hydrogène et R₁₂ représente un radical phényle substitué par un radical nitro, isole le produit et le transforme éventuellement en sel.

16. Procédé de préparation des composés de formule (I) selon la revendication 1 pour lesquels R₁ représente un radical -NH-CONR₉R₁₂, -N(alk)CONR₈R₉, -NHCSNR₈R₉ dans lesquels R₉ représente un atome d'hydrogène, R₈ représente un atome d'hydrogène ou un radical alkyle ou phényle et R₁₂ représente un atome d'hydrogène ou un radical alkyle, phénylalkyle éventuellement substitué, -alk-COOR₂₁ ou phényle substitué caractérisé en ce que l'on fait réagir un composé de formule : 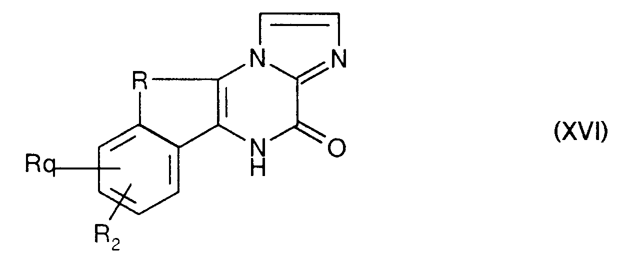 pour lequel R et R₂ ont les mêmes significations que dans la revendication 1 et Rq représente un radical -NH₂ ou -NHalk alk ayant les mêmes significations que dans la revendication 1, sur un dérivé de formule Rs=C=N-Rt dans lequel Rt représente un radical -Si(CH₃)₃, alkyle, phénylalkyle dont le noyau phényle est éventuellement substitué par un ou plusieurs substituants choisis parmi les atomes d'halogène, les radicaux alcoxy et -COOR₂₁, -alk-COOR₂₁ ou phényle substitué par un ou plusieurs substituants choisis parmi les atomes d'halogène et les radicaux alcoxy, nitro, amino, cyano et -COOR₂₁ dans lesquels alk et R₂₁ ont les mêmes significations que dans la revendication 1 et Rs représente un atome d'oxygène ou de soufre, isole le produit et le transforme éventuellement en sel.

17. Procédé de préparation des composés de formule (I) selon la revendication 1 pour lesquels R₁ représente un radical -NH-CO-R₁₀ dans lequel R₁₀ représente un radical alcoxy caractérisé en ce que l'on fait réagir un dérivé de formule : 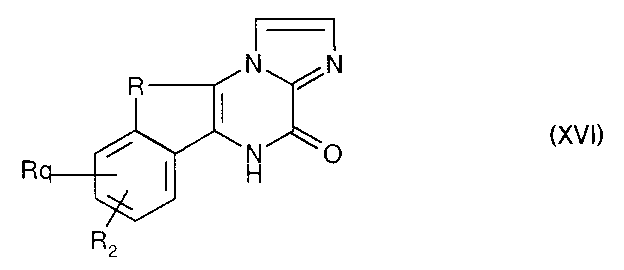 dans laquelle R et R₂ ont les mêmes significations que dans la revendication 1 et Rq représente un radical amino sur un dérivé Hal-COOalk pour lequel Hal représente un atome d'halogène et alk représente un radical alkyle et chauffage du dialcoxy obtenu éventuellement en présence d'une amine, isole le produit et le transforme éventuellement en sel.

18. Procédé de préparation des composés de formule (I) selon la revendication 1 pour lesquels R₁ représente un radical -CO-NR₇R₉ caractérisé en ce que l'on fait réagir un composé de formule (I) correspondant pour lequel R₁ représente un radical carboxy ou un dérivé réactif de cet acide sur une amine HNR₇R₉, isole le produit et le transforme éventuellement en sel.

19. Procédé de préparation des composés de formule (I) selon la revendication 1 pour lesquels R₁ représente un radical -NR₉R₁₁ caractérisé en ce que l'on fait réagir un dérivé de formule : 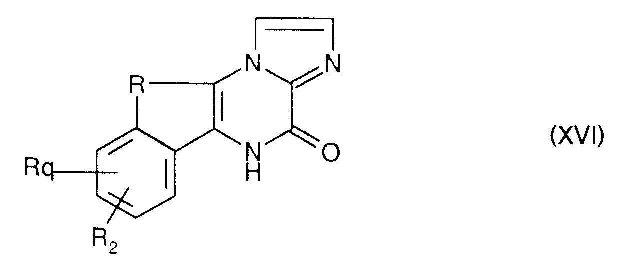 pour lequel R et R₂ ont les mêmes significations que dans la revendication 1 et Rq représente un radical -NH₂ ou -NHalk dans lequel alk a les mêmes significations que dans la revendication 1 sur un dérivé Hal-R₁₁ dans lequel R₁₁ a les mêmes significations que dans la revendication 1, isole le produit et le transforme éventuellement en sel.

20. Procédé de préparation des composés de formule (I) selon la revendication 1 pour lesquels R₁ représente un radical 2-oxo-1-imidazolidinyle caractérisé en ce que l'on cyclise un dérivé de formule : 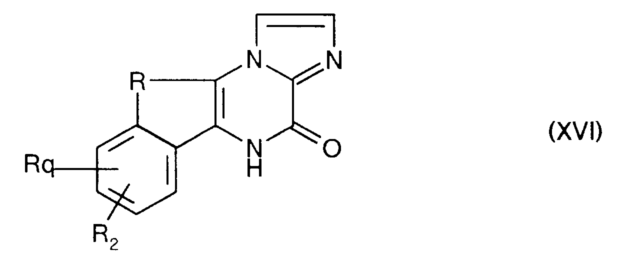 pour lequel R et R₂ ont les mêmes significations que dans la revendication 1 et Rq représente un radical -NH-CO-NH-(CH₂)ₙ-Hal dans lequel Hal représente un atome d'halogène, alk représente un radical alkyle et n est égal à 2, isole le produit et le transforme éventuellement en sel.

21. Procédé de préparation des composés de formule (I) selon la revendication 1 pour lesquels R₁ représente un radical -NH-CO-NR₉R₁₂ ou -NH-COR₁₀ dans lesquels R₁₂ et R₁₀ représentent des radicaux -alk-COOR₂₁ et R₂₁ représente un atome d'hydrogène caractérisé en ce que l'on hydrolyse un composé de formule (I) correspondant pour lequel R₁ représente un radical -NH-CO-NR₉R₁₂ ou -NH-COR₁₀ dans lesquels R₁₂ et R₁₀ représentent des radicaux -alk-COOR₂₁ et R₂₁ représente un radical alkyle, isole le produit et le transforme éventuellement en sel.

22. Procédé de préparation des composés de formule (I) selon la revendication 1 pour lesquels R₁ représente un radical -N R₉R₁₁, R₁₁ représente un radical imidazolinyle-2 caractérisé en ce que l'on cyclise un dérivé de formule : 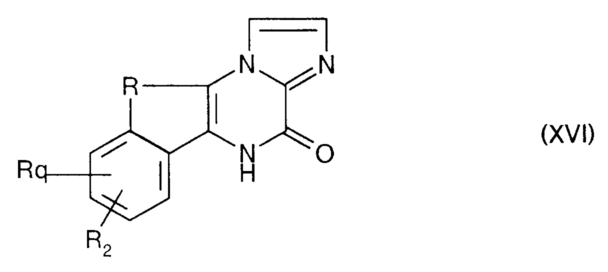 pour lequel R et R₂ ont les mêmes significations que dans la revendication 1 et Rq représente un radical -N=C(SCH₃)-NH-(CH₂)₂-NH₂, isole le produit et le transforme éventuellement en sel.

23. Médicaments contenant en tant que principe actif au moins un composé selon l'une des revendications 1 ou 2 ou un sel pharmaceutiquement acceptable d'un tel composé.

## Claims

1. Compounds of formula: 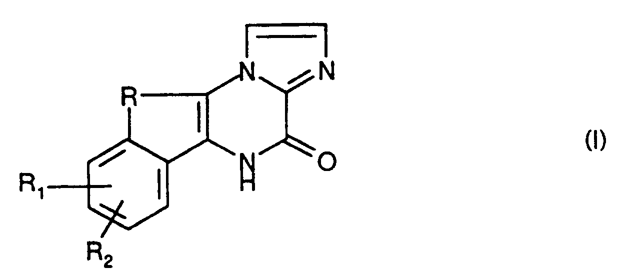 in which
- R represents a C=R₃ or CH-R₆ radical,
- R₁ represents a hydroxyl, polyfluoroalkoxy, carboxyl, -NH-CHO, -N(alk)-CO-NR₈R₉, -NH-CO-NR₉R₁₂, -NH-CS-NR₈R₉, -NH-CO-R₁₀, -NH-SO₂-NR₇R₉, -CO-NR₇R₉, -NH-SO₂-alk, -NR₉R₁₁, -S-alk-Ar, -SO₂-NR₇R₉ or 2-oxo-1-imidazolidinyl radical,
- R₂ represents a hydrogen atom,
- R₃ represents an oxygen atom or an NOH radical,
- R₆ represents a hydrogen atom or an amino radical,
- R₇ represents a hydrogen atom or an alkyl radical,
- R₈ represents a hydrogen atom or an alkyl, -alk-NR₉R₇ or phenyl radical,
- R₉ represents a hydrogen atom or an alkyl radical,
- R₁₀ represents an alkyl (5-9 C in a straight or branched chain), alkoxy, -alk-COOR₂₁, -alk-NR₉R₇, phenylalkyl or phenyl radical or a heterocycle chosen from the pyrrolidine, azetidine and morpholine,
- R₁₁ represents a heterocycle chosen from imidazoline and thiazoline,
- R₁₂ represents a hydrogen atom or a radical of the type alkyl, -alk-COOR₂₁, phenylalkyl in which the phenyl ring is optionally substituted by one or a number of substituents chosen from halogen atoms and radicals of the type alkoxy and -COOR₂₁ phenyl substituted by one or a number of substituents chosen from halogen atoms and radicals of the type alkoxy, nitro, amino, -COOR₂₁ and cyano,
- R₂₁ represents a hydrogen atom or an alkyl radical,
- Ar represents a phenyl radical,
- alk represents an alkyl or alkylene radical,
it being understood that, except where otherwise mentioned, the alkyl and alkoxy radicals and portions contain 1 to 6 straight- or branched-chain carbon atoms, the E and Z isomers of the compounds in which R₃ represents an NOH radical, the enantiomers and diastereoisomers of the compounds in which R represents CH-R₆, and the salts of these compounds.

2. The following compounds of formula (I) according to claim 1:
- 9-phenylacetamido-5H,10H-imidazo[1,2a]indeno[1,2-e]pyrazin-4-one,
- 8-ethoxycarbonylamino-5H,10H-imidazo[1,2-a]indeno[1,2-e]pyrazin-4-one,
- 8-[3-(3-cyanophenyl)ureido]-5H,10H-imidazo[1,2-a]indeno[1,2-e]pyrazin-4-one,
- 8-[3-(3-methoxyphenyl)ureido]-5H,10H-imidazo[1,2-a]indeno[1,2-e]pyrazin-4-one,
- 8-phenylacetamido-5H,10H-imidazo[1,2-a]indeno[1,2-e]pyrazin-4-one,
- 8-(3-phenylethylureido)-5H,10H-imidazo[1,2-a]indeno[1,2-e]pyrazin-4-one,
- 8-(3-methylureido)-5H,10H-imidazo[1,2-a]indeno[1,2-e]pyrazin-4-one,
- 8-(3-benzylureido)-5H,10H-imidazo[1,2-a]indeno[1,2-e]pyrazin-4-one,
- 8-(3-tert-butylureido)-5H,10H-imidazo[1,2-a]indeno[1,2-e]pyrazin-4-one,
- 8-(3-phenylpropionamido)-5H,10H-imidazo[1,2-a]indeno[1,2-e]pyrazin-4-one,
- 8-benzamido-5H,10H-imidazo[1,2-a]indeno[1,2-e]-pyrazin-4-one,
- 8-(4-phenylbutyrylamino)-5H,10H-imidazo[1,2-a]indeno[1,2-e]pyrazin-4-one,
- 8-(5-phenylvalerylamino)-5H,10H-imidazo[1,2-a]indeno[1,2-e]pyrazin-4-one,
- 8-(3-ethoxycarbonylmethylureido)-5H,10H-imidazo[1,2-a]indeno[1,2-e]pyrazin-4-one,
- 8-(3-carboxymethylureido)-5H,10H-imidazo[1,2-a]-indeno[1,2-e]pyrazin-4-one,
- 8-(3,3-dimethylureido)-5H,10H-imidazo[1,2-a]indeno[1,2-e]pyrazin-4-one,
- 8-hydroxy-5H,10H-imidazo[1,2-a]indeno[1,2-e]pyrazin-4-one,
- 8-(3-aminopropionamido)-5H,10H-imidazo[1,2-a]indeno[1,2-e]pyrazin-4-one,
- 8-aminoacetamido-5H,10H-imidazo[1,2-a]indeno[1,2-e]pyrazin-4-one,
- 8-[3-(3-nitrophenyl)ureido]-5H,10H-imidazo[1,2-a]indeno[1,2-e]pyrazin-4-one,
- 8-[3-(2-methoxyphenyl)ureido]-5H,10H-imidazo[1,2-a]indeno[1,2-e]pyrazin-4-one,
- 8-[3-(2-nitrophenyl)ureido]-5H,10H-imidazo[1,2-a]indeno[1,2-e]pyrazin-4-one,
- 8-[3-(4-aminophenyl)ureido]-5H,10H-imidazo[1,2-a]indeno[1,2-e]pyrazin-4-one,
- 8-[3-(4-methoxyphenyl)ureido]-5H,10H-imidazo[1,2-a]indeno[1,2-e]pyrazin-4-one,
- 8-(4-methylpentanoyl)amino-5H,10H-imidazo[1,2-a]indeno[1,2-e]pyrazin-4-one,
- N,N-dimethyl-4-oxo-4,5-dihydro-10H-imidazo[1,2-a]indeno[1,2-e]pyrazine-8-sulphonamide,
- 8-(3-phenylthioureido)-5H,10H-imidazo[1,2-a]indeno[1,2-e]pyrazin-4-one,
- 8-(3-methylthioureido)-5H,10H-imidazo[1,2-a]indeno[1,2-e]pyrazin-4-one,
- 8-(2-oxo-1-imidazolidinyl)-5H,10H-imidazo[1,2-a]indeno-[1,2-e]pyrazin-4-one,
- 8-formamido-5H,10H-imidazo[1,2-a]indeno[1,2-e]-pyrazin-4-one,
- 8-(3-ethoxycarbonylpropionylamino)-5H,10H-imidazo-[1,2-a]indeno[1,2-e]pyrazin-4-one,
- 8-[3-(2-ethoxycarbonylethyl)ureido]-5H,10H-imidazo[1,2-a]indeno[1,2-e]pyrazin-4-one,
- 8-[3-(2-carboxyethyl)ureido]-5H,10H-imidazo[1,2-a]indeno[1,2-e]pyrazin-4-one,
- 8-[3-(4-fluorophenyl)ureido]-5H,10H-imidazo[1,2-a]indeno[1,2-e]pyrazin-4-one,
- 8-[3-(3-fluorophenyl)ureido]-5H,10H-imidazo[1,2-a]indeno[1,2-e]pyrazin-4-one,
- 8-[3-(2-fluorophenyl)ureido]-5H,10H-imidazo[1,2-a]indeno[1,2-e]pyrazin-4-one,
- 8-(3-ethylureido)-5H,10H-imidazo[1,2-a]indeno[1,2-e]pyrazin-4-one,
- 8-[3-morpholinoureido]-5H,10H-imidazo[1,2-a]indeno[1,2-e]pyrazin-4-one,
- 10-amino-8-(3-methylureido)-5H,10H-imidazo[1,2-a]indeno[1,2-e]pyrazin-4-one,
- 10-hydroxyimino-8-(3-methylureido)-5H,10H-imidazo[1,2-a]indeno[1,2-e]pyrazin-4-one,
- 8-(3-methylureido)-5H-imidazo[1,2-a]indeno[1,2-e]pyrazin-4,10-dione,
- 8-[3-(3-aminophenyl)ureido]-5H,10H-imidazo[1,2-a]indeno[1,2-e]pyrazin-4-one,
- 8-[(1-azetidinyl)carbonylamino]-5H,10H-imidazo-[1,2-a]indeno[1,2-e]pyrazin-4-one,
- 8-(3-propylureido)-5H,10H-imidazo[1,2-a]indeno-[1,2-e]pyrazin-4-one,
- 8-(3-isopropylureido)-5H,10H-imidazo[1,2-a]indeno[1,2-e]pyrazin-4-one,
- 8-(3-butylureido)-5H,10H-imidazo[1,2-a]indeno-[1,2-e]pyrazin-4-one,
- 8-[(2-thiazolin-2-yl)amino]-5H,10H-imidazo[1,2-a]indeno[1,2-e]pyrazin-4-one,
- 8-[3-(3-carbomethoxyphenyl)ureido]-5H,10H-imidazo[1,2-a]indeno[1,2-e]pyrazin-4-one,
- 8-[3-(4-carbomethoxybenzyl)ureido]-5H,10H-imidazo[1,2-a]indeno[1,2-e]pyrazin-4-one,
- 8-[3-(4-carboxybenzyl)ureido]-5H,10H-imidazo[1,2-a]indeno[1,2-e]pyrazin-4-one,
- 8-[3-(2-fluorobenzyl)ureido]-5H,10H-imidazo[1,2-a]indeno[1,2-e]pyrazin-4-one,
- 8-[3-(3-fluorobenzyl)ureido]-5H,10H-imidazo[1,2-a]indeno[1,2-e]pyrazin-4-one,
- 8-[3-(4-fluorobenzyl)ureido]-5H,10H-imidazo[1,2-a]indeno[1,2-e]pyrazin-4-one,
- 9-(N-ethylcarbamoyl)-5H,10H-imidazo[1,2-a]indeno[1,2-e]pyrazin-4-one,
- 9-(3-methylureido)-5H,10H-imidazo[1,2-a]indeno-[1,2-e]pyrazin-4-one,
- 8-[3-(4-methoxybenzyl)ureido]-5H,10H-imidazo[1,2-a]indeno[1,2-e]pyrazin-4-one,
and their salts.

3. Process for the preparation of the compounds of formula (I) according to claim 1 in which R represents a C=R₃ radical in which R₃ represents an oxygen atom, characterized in that a corresponding compound of formula (I), in which R represents a C=R₃ radical and R₃ represents an NOH radical, is hydrolysed and the product is isolated and optionally converted to a salt.

4. Process for the preparation of the compounds of formula (I) according to claim 1 in which R represents a C=R₃ radical and R₃ represents an NOH radical, characterized in that an alkyl nitrite is reacted with a corresponding compound of formula (I) in which R represents a CH-R₆ radical and R₆ represents a hydrogen atom and the product is isolated and optionally converted to a salt.

5. Process for the preparation of the compounds of formula (I) according to claim 1 in which R represents a CH-R₆ radical and R₆ represents a hydrogen atom, characterized in that a derivative of formula: 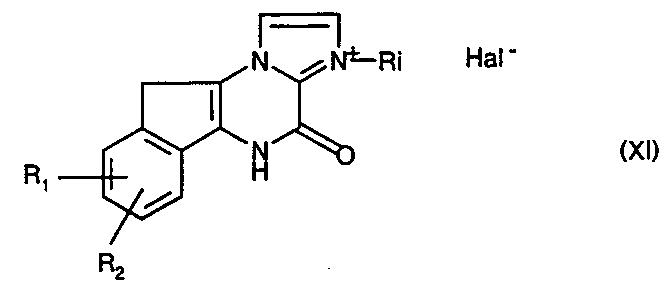 in which R₁ and R₂ have the same meanings as in claim 1, Ri represents an alkyl radical and Hal represents a halogen atom, is dealkylated and desalified and the product is isolated and optionally converted to a salt.

6. Process for the preparation of the compounds of formula (I) according to claim 1 in which R represents a CH-R₆ radical and R₆ represents a hydrogen atom, characterized in that a derivative of formula: 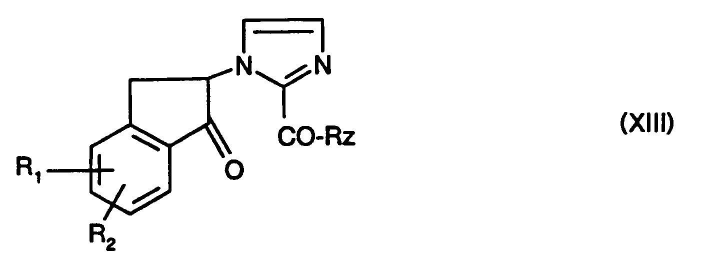 in which R₁ and R₂ have the same meanings as in claim 1 and Rz represents an -NH₂ or -Oalk radical in which alk represents an alkyl radical, is cyclized, optionally in the presence of ammonium acetate, and the product is isolated and optionally converted to a salt.

7. Process for the preparation of the compounds of formula (I) according to claim 1 in which R represents a CH-R₆ radical in which R₆ represents an amino radical, characterized in that a compound of formula: 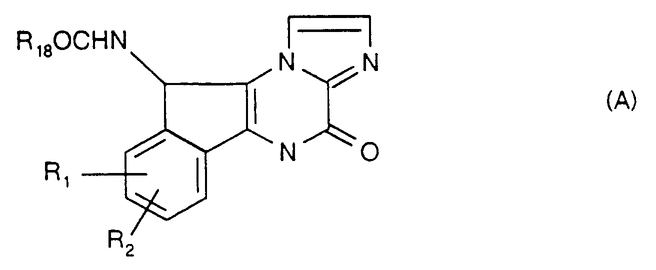 in which R₁ and R₂ have the same meanings as in claim 1 and R₁₈ represents an alkyl radical is hydrolysed and the product is isolated and optionally converted to a salt.

8. Process for the preparation of the compounds of formula (I) according to claim 1 in which R represents a CH-R₆ radical in which R₆ represents an amino radical, characterized in that a corresponding compound of formula (I) in which R represents a C=R₃ radical in which R₃ represents an NOH radical is reduced and the product is isolated and optionally converted to a salt.

9. Process for the preparation of the compounds of formula (I) according to claim 1 in which R₁ represents a hydroxyl radical, characterized in that a derivative of formula: 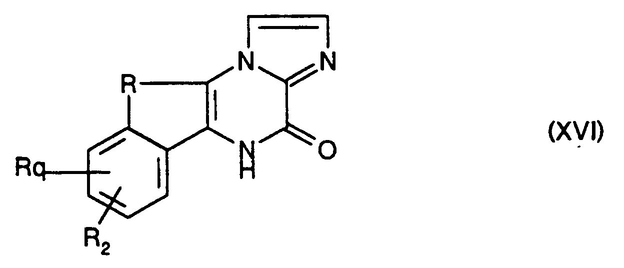 in which R and R₂ have the same meanings as in claim 1 and R_{q} represents an alkoxy radical, is hydrolysed and the product is isolated and optionally converted to a salt.

10. Process for the preparation of the compounds of formula (I) according to claim 1 in which R₁ represents a carboxyl radical, characterized in that a derivative of formula: 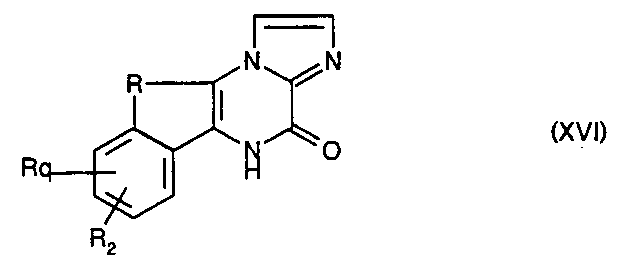 in which R and R₂ have the same meanings as in claim 1 and Rq represents a cyano radical, is hydrolysed and the product is isolated and optionally converted to a salt.

11. Process for the preparation of the compounds of formula (I) according to claim 1 in which R₁ represents an -NHCHO radical, characterized in that a compound of formula: 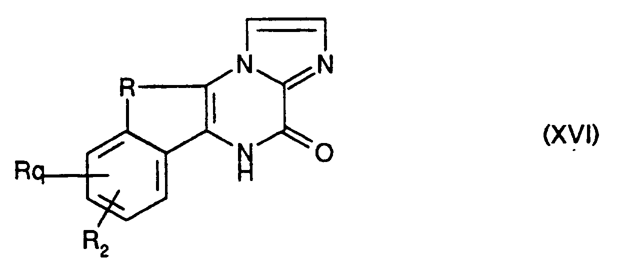 in which R and R₂ have the same meanings as in claim 1 and Rq represents an amino radical, is reacted with H₃C-COOCHO and the product is isolated and optionally converted to a salt.

12. Process for the preparation of the compounds of formula (I) according to claim 1 in which R₁ represents an -N(alk)-CO-NR₈R₉ or -NH-CSNR₈R₉ radical in which R₈ and R₉ are not hydrogen atoms, an -NH-CO-NR₉R₁₂ radical in which R₉ and R₁₂ are not hydrogen atoms, an -NH-COR₁₀ radical, an -NH-SO₂-NR₇R₉ radical in which R₇ is an alkyl radical, or an -NH-SO₂-alk radical, characterized in that a compound of formula: 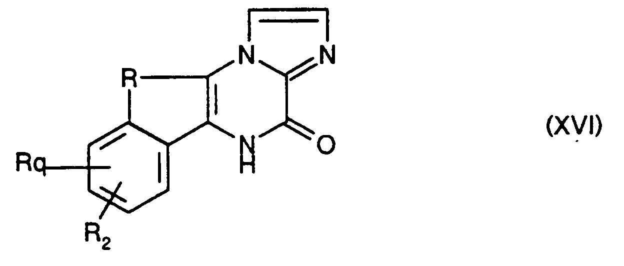 in which R and R₂ have the same meanings as in claim 1 and Rq represents an -NH₂ or -NH-alk radical in which alk has the same meanings as in claim 1, is reacted with a derivative of formula Hal-Rr in which Hal represents a halogen atom and Rr represents a -CO-NR₈R₉ or -CSNR₈R₉ radical in which R₈ and R₉ have the same meanings as in claim 1 with the exception of hydrogen, a -CO-NR₉R₁₂ radical in which R₉ and R₁₂ have the same meanings as in claim 1 with the exception of hydrogen, a -COR₁₀ radical in which R₁₀ has the same meanings as in claim 1, an -SO₂-NR₇R₉ radical in which R₉ has the same meanings as in claim 1 and R₇ is an alkyl radical, or an -SO₂-alk radical in which alk has the same meanings as in claim 1 and the product is isolated and optionally converted to a salt.

13. Process for the preparation of the compounds of formula (I) according to claim 1 in which R₁ represents an -N(alk)-CO-NR₈R₉ or -NH-CO-NR₉R₁₂ radical, characterized in that a derivative of formula: 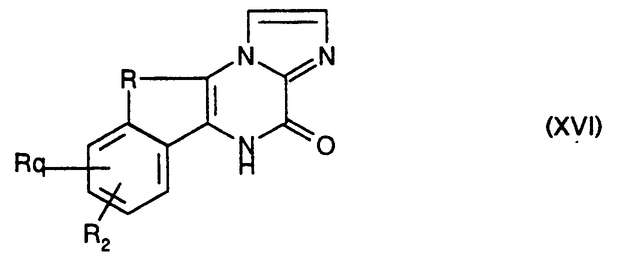 in which R and R₂ have the same meanings as in claim 1 and Rq represents an -NH₂ or -NH-alk radical in which alk has the same meanings as in claim 1, is reacted with a halotrialkylsilane in the presence of an alkali metal hydride, then a coupling agent and an amine of formula HNR₈R₉ or HN-R₉R₁₂ in which R₈, R₉ and R₁₂ have the same meanings as in claim 1 and the product is isolated and optionally converted to a salt.

14. Process for the preparation of the compounds of formula (I) according to claim 1 in which R₁ represents an -NH-CO-NR₉R₁₂ or -N(alk)-CO-NR₈R₉ radical, characterized in that a derivative of formula: 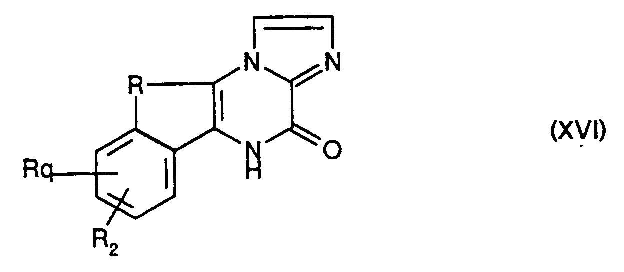 in which R and R₂ have the same meanings as in claim 1 and Rq represents an -NH₂ or -NH-alk radical in which alk has the same meanings as in claim 1, is reacted with a derivative of formula: 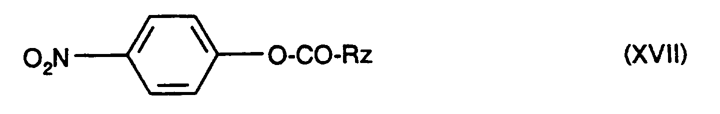 in which Rz represents an -NR₉R₁₂ or -NR₈R₉ radical, R₈, R₉ and R₁₂ having the same meanings as in claim 1, and the product is isolated and optionally converted to a salt.

15. Process for the preparation of the compounds of formula (I) according to claim 1 in which R₁ represents an -NH-CONR₉R₁₂ radical, R₉ represents a hydrogen atom and R₁₂ represents a phenyl radical substituted by an amino radical characterized in that a corresponding compound of formula (I) in which R₁ represents an -NH-CONR₉R₁₂ radical, R₉ represents a hydrogen atom and R₁₂ represents a phenyl radical substituted by a nitro radical is reduced and the product is isolated and optionally converted to a salt.

16. Process for the preparation of the compounds of formula (I) according to claim 1 in which R₁ represents an -NH-CONR₉R₁₂, -N(alk)CONR₈R₉ or -NHCSNR₈R₉ radical in which R₉ represents a hydrogen atom, R₈ represents a hydrogen atom or an alkyl or phenyl radical and R₁₂ represents a hydrogen atom or an optionally substituted phenylalkyl or alkyl radical, an -alk-COOR₂₁ radical or a substituted phenyl radical, characterized in that a compound of formula: 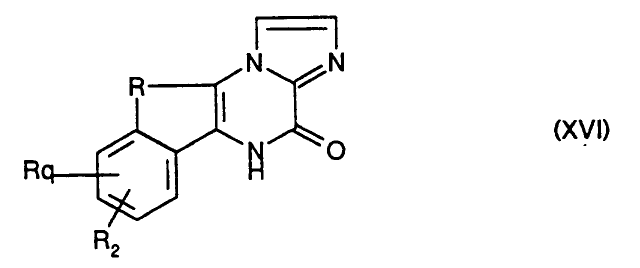 in which R and R₂ have the same meanings as in claim 1 and Rq represents an -NH₂ or -NHalk radical, alk having the same meanings as in claim 1, is reacted with a derivative of formula Rs=C=N-Rt in which Rt represents a radical of the type -Si(CH₃)₃, alkyl, phenylalkyl in which the phenyl ring is optionally substituted by one or a number of substituents chosen from halogen atoms, radicals of the type alkoxy and -COOR₂₁, -alk-COOR₂₁ or phenyl substituted by one or a number of substituents chosen from halogen atoms and radicals of the type alkoxy, nitro, amino, cyano and -COOR₂₁ in which alk and R₂₁ have the same meanings as in claim 1, and Rs represents an oxygen or sulphur atom and the product is isolated and optionally converted to a salt.

17. Process for the preparation of the compounds of formula (I) according to claim 1 in which R₁ represents an -NH-CO-R₁₀ radical in which R₁₀ represents an alkoxy radical, characterized in that a derivative of formula: 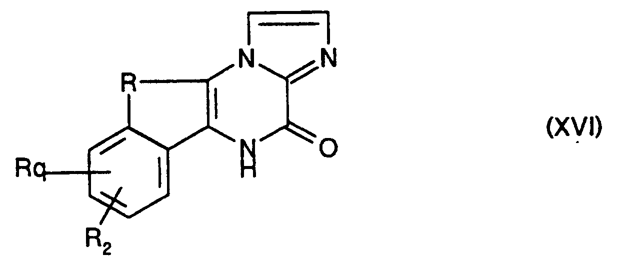 in which R and R₂ have the same meanings as in claim 1 and Rq represents an amino radical, is reacted with a Hal-COOalk derivative in which Hal represents a halogen atom and alk represents an alkyl radical and the dialkoxy obtained is heated, optionally in the presence of an amine, and the product is isolated and optionally converted to a salt.

18. Process for the preparation of the compounds of formula (I) according to claim 1 in which R₁ represents a -CO-NR₇R₉ radical, characterized in that a corresponding compound of formula (I) in which R₁ represents a carboxyl radical or a reactive derivative of this acid is reacted with an HNR₇R₉ amine and the product is isolated and optionally converted to a salt.

19. Process for the preparation of the compounds of formula (I) according to claim 1 in which R₁ represents an -NR₉R₁₁ radical characterized in that a derivative of formula: 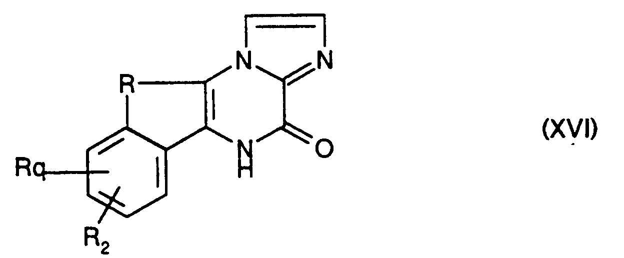 in which R and R₂ have the same meanings as in claim 1 and Rq represents an -NH₂ or -NHalk radical in which alk has the same meanings as in claim 1, is reacted with a Hal-R₁₁ derivative in which R₁₁ has the same meanings as in claim 1 and the product is isolated and optionally converted to a salt.

20. Process for the preparation of the compounds of formula (I) according to claim 1 in which R₁ represents a 2-oxo-1-imidazolidinyl radical, characterized in that a derivative of formula: 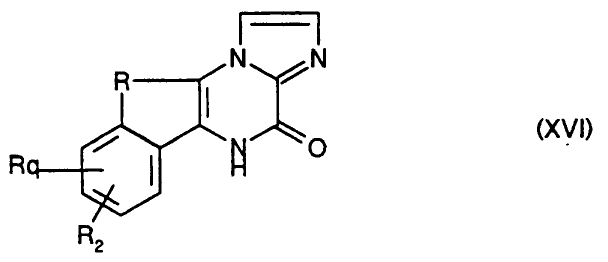 in which R and R₂ have the same meanings as in claim 1 and Rq represents an -NH-CO-NH-(CH₂)ₙ-Hal radical in which Hal represents a halogen atom, alk represents an alkyl radical and n is equal to 2, is cyclized and the product is isolated and optionally converted to a salt.

21. Process for the preparation of the compounds of formula (I) according to claim 1 in which R₁ represents an -NH-CO-NR₉R₁₂ or -NH-COR₁₀ radical in which R₁₂ and R₁₀ represent -alk-COOR₂₁ radicals and R₂₁ represents a hydrogen atom, characterized in that a corresponding compound of formula (I) in which R₁ represents an -NH-CO-NR₉R₁₂ or -NH-COR₁₀ radical in which R₁₂ and R₁₀ represent -alk-COOR₂₁ radicals and R₂₁ represents an alkyl radical is hydrolysed and the product is isolated and optionally converted to a salt.

22. Process for the preparation of the compounds of formula (I) according to claim 1 in which R₁ represents an -NR₉R₁₁ radical and R₁₁ represents a 2-imidazolinyl radical, characterized in that a derivative of formula: 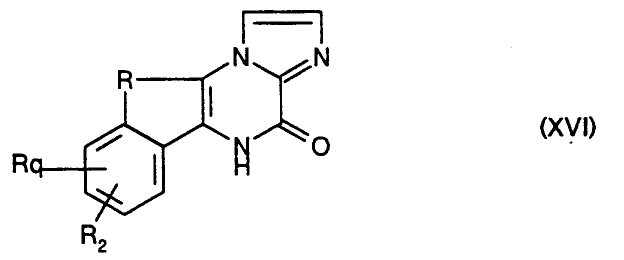 in which R and R₂ have the same meanings as in claim 1 and Rq represents an -N=C(SCH₃)-NH-(CH₂)₂-NH₂ radical, is cyclized and the product is isolated and optionally converted to a salt.

23. Medicaments containing, as active principle, at least one compound according to either of claims 1 and 2 or a pharmaceutically acceptable salt of such a compound.

## Patentansprüche

1. Verbindungen der Formel; 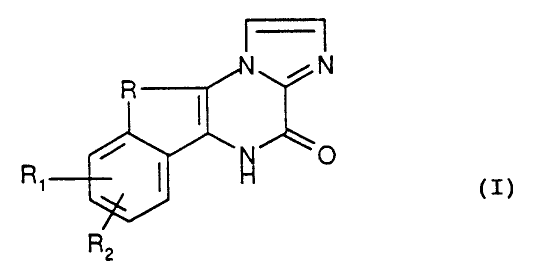 worin
- R einen Rest C=R₃ oder CH-R₆ bedeutet,
- R₁ einen Hydroxy-, Polyfluoralkoxy-, Carboxy-, -NH-CHO-, -N(alk)-CO-NR₈R₉-, -NH-CO-NR₉R₁₂-, -NH-CS-NR₈R₉-, -NH-CO-R₁₀-, -NH-SO₂-NR₇R₉-, -CO-NR₇R₉-, -NH-SO₂-alk-, -NR₉R₁₁-, -S-alk-Ar-, -SO₂-NR₇R₉-, 2-Oxo-1-imidazolidinylrest bedeutet,
- R₂ ein Wasserstoffatom bedeutet,
- R₃ ein Sauerstoffatom oder einen NOH-Rest bedeutet,
- R₆ ein Wasserstoffatom oder einen Aminorest bedeutet,
- R₇ ein Wasserstoffatom oder einen Alkylrest bedeutet,
- R₈ ein Wasserstoffatom oder einen Alkyl-, -alk-NR₉R₇- oder Phenylrest bedeutet,
- R₉ ein Wasserstoffatom oder einen Alkylrest bedeutet,
- R₁₀ einen Alkyl- (5-9 C in gerader oder verzweigter Kette), Alkoxy-, -alk-COOR₂₁-, -alk-NR₉R₇-, Phenylalkyl-, Phenyl- oder heterocyclischen Rest, ausgewählt aus Pyrrolidin, Azetidin und Morpholin, bedeutet,
- R₁₁ einen Heterocyclus, ausgewählt aus Imidazolin und Thiazolin, bedeutet,
- R₁₂ ein Wasserstoffatom oder einen Alkyl-, -alk-COOR₂₁-, Phenylalkylrest, dessen Phenylkern gegebenenfalls durch einen oder mehrere Substituenten, ausgewählt aus Halogenatomen und Alkoxy- und -COOR₂₁-Resten, substituiert ist, und Phenylrest, substituiert durch einen oder mehrere Substituenten, ausgewählt aus Halogenatomen und Alkoxy-, Nitro-, Amino-, -COOR₂₁- und Cyanoresten, bedeutet,
- R₂₁ ein Wasserstoffatom oder einen Alkylrest bedeutet,
- Ar einen Phenylrest bedeutet,
- alk einen Alkyl- oder Alkylenrest bedeutet,
mit der Maßgabe, daß, außer anderweitig angegeben, die Alkyl- und Alkoxyreste und -anteile 1 bis 6 Kohlenstoffatome in gerader oder verzweigter Kette enthalten,
die E- und Z-Isomeren der Verbindungen, in denen R₃ einen NOH-Rest bedeutet, die Enantiomeren und Diastereoisomeren der Verbindungen, in denen R CH-R₆ bedeutet,
sowie die Salze dieser Verbindungen.

2. Verbindungen der Formel (I) nach Anspruch 1, nämlich:
- 9-Phenylacetamido-5H,10H-imidazo[1,2-a]indeno[1,2-e]pyrazin-4-on,
- 8-Ethoxycarbonylamino-5H,10H-imidazo[1,2-a]indeno[1,2-e]pyrazin-4-on,
- 8-[3-(3-Cyanophenyl)ureido]-5H,10H-imidazo[1,2-a]indeno[1,2-e]pyrazin-4-on,
- 8-[3-(3-Methoxyphenyl)ureido]-5H,10H-imidazo[1,2-a]indeno[1,2-e]pyrazin-4-on,
- 8-Phenylacetamido-5H,10H-imidazo[1,2-a]indeno[1,2-e]pyrazin-4-on,
- 8-(3-Phenethylureido)-5H,10H-imidazo[1,2-a]indeno[1,2-e]pyrazin-4-on,
- 8-(3-Methylureido)-5H,10H-imidazo[1,2-a]indeno[1,2-e]pyrazin-4-on,
- 8-(3-Benzylureido)-5H,10H-imidazo[1,2-a]indeno[1,2-e]pyrazin-4-on,
- 8-(3-tert.-Butylureido)-5H,10H-imidazo[1,2-a]indeno[1,2-e]pyrazin-4-on,
- 8-(3-Phenylpropionamido)-5H,10H-imidazo[1,2-a]indeno[1,2-e]pyrazin-4-on,
- 8-Benzamido-5H,10H-imidazo[1,2-a]indeno[1,2-e]pyrazin-4-on,
- 8-(4-Phenylbutyrylamino)-5H,10H-imidazo[1,2-a]indeno[1,2-e]pyrazin-4-on,
- 8-(5-Phenylvalerylamino)-5H,10H-imidazo[1,2-a]indeno[1,2-e]pyrazin-4-on,
- 8-(3-Ethoxycarbonylmethylureido)-5H,10H-imidazo[1,2-a]indeno[1,2-e]pyrazin-4-on,
- 8-(3-Carboxymethylureido)-5H,10H-imidazo[1,2-a]indeno[1,2-e]pyrazin-4-on,
- 8-(3,3-Dimethylureido)-5H,10H-imidazo[1,2-a]indeno[1,2-e]pyrazin-4-on,
- 8-Hydroxy-5H,10H-imidazo[1,2-a]indeno[1,2-e]pyrazin-4-on,
- 8-(3-Aminopropionamido)-5H,10H-imidazo[1,2-a]indeno[1,2-e]pyrazin-4-on,
- 8-Aminoacetamido-5H,10H-imidazo[1,2-a]indeno[1,2-e]pyrazin-4-on,
- 8-[3-(3-Nitrophenyl)ureido]-5H,10H-imidazo[1,2-a]indeno[1,2-e]pyrazin-4-on,
- 8-[3-(2-Methoxyphenyl)ureido]-5H,10H-imidazo[1,2-a]indeno[1,2-e]pyrazin-4-on,
- 8-[3-(2-Nitrophenyl)ureido]-5H,10H-imidazo[1,2-a]indeno[1,2-e]pyrazin-4-on,
- 8-[3-(4-Aminophenyl)ureido]-5H,10H-imidazo[1,2-a]indeno[1,2-e]pyrazin-4-on,
- 8-[3-(4-Methoxyphenyl)ureido]-5H,10H-imidazo[1,2-a]indeno[1,2-e]pyrazin-4-on,
- 8-(4-Methylpentanoyl)amino-5H,10H-imidazo[1,2-a]indeno[1,2-e]pyrazin-4-on,
- N,N-Dimethyl-4-oxo-4,5-dihydro-10H-imidazo[1,2-a]indeno[1,2-e]pyrazin-8-sulfonamid,
- 8-(3-Phenylthioureido)-5H,10H-imidazo[1,2-a]indeno[1,2-e]pyrazin-4-on,
- 8-(3-Methylthioureido)-5H,10H-imidazo[1,2-a]indeno[1,2-e]pyrazin-4-on,
- 8-(2-Oxo-1-imidazolinyl)-5H,10H-imidazo[1,2-a]indeno[1,2-e]pyrazin-4-on,
- 8-Formamido-5H,10H-imidazo[1,2-a]indeno[1,2-e]pyrazin-4-on,
- 8-(3-Ethoxycarbonylpropionylamino)-5H,10H-imidazo[1,2-a]indeno[1,2-e]pyrazin-4-on,
- 8-[3-(2-Ethoxycarbonylethyl)ureido]-5H,10H-imidazo[1,2-a]indeno[1,2-e]pyrazin-4-on,
- -8-[3-(2-Carboxyethyl)ureido]-5H,10H-imidazo[1,2-a]indeno[1,2-e]pyrazin-4-on,
- 8-[3-(4-Fluorphenyl)ureido]-5H,10H-imidazo[1,2-a]indeno[1,2-e]pyrazin-4-on,
- 8-[3-(3-Fluorphenyl)ureido]-5H,10H-imidazo[1,2-a]indeno[1,2-e]pyrazin-4-on,
- 8-[3-(2-Fluorphenyl)ureido]-5H,10H-imidazo[1,2-a]indeno[1,2-e]pyrazin-4-on,
- 8-(3-Ethylureido)-5H,10H-imidazo[1,2-a]indeno[1,2-e]pyrazin-4-on,
- 8-[3-Morpholinoureido]-5H,10H-imidazo[1,2-a]indeno[1,2-e]pyrazin-4-on,
- 10-Amino-8-(3-methylureido)-5H,10H-imidazo[1,2-a]indeno[1,2-e]pyrazin-4-on,
- 10-Hydroxyimino-8-(3-methylureido)-5H,10H-imidazo[1,2-a]indeno[1,2-e]pyrazin-4-on,
- 8-(3-Methylureido)-5H-imidazo[1,2-a]indeno[1,2-e]pyrazin-4,10-dion,
- 8-[3-(3-Aminophenyl)ureido]-5H,10H-imidazo[1,2-a]indeno[1,2-e]pyrazin-4-on,
- 8-[(1-Azetidinyl)carbonylamino]-5H,10H-imidazo[1,2-a]indeno[1,2-e]pyrazin-4-on,
- 8-(3-Propylureido )-5H,10H-imidazo[1,2-a]indeno[1,2-e]pyrazin-4-on,
- 8-(3-Isopropylureido)-5H,10H-imidazo[1,2-a]indeno[1,2-e]pyrazin-4-on,
- 8-(3-Butylureido)-5H,10H-imidazo[1,2-a]indeno[1,2-e]pyrazin-4-on,
- 8-[(2-Thiazolin-2-yl)amino]-5H,10H-imidazo[1,2-a]indeno[1,2-e]pyrazin-4-on,
- 8-[3-(3-Carbomethoxyphenyl)ureido]-5H,10H-imidazo[1,2-a]indeno[1,2-e]pyrazin-4-on,
- 8-[3-(4-Carbomethoxybenzyl)ureido]-5H,10H-imidazo[1,2-a]indeno[1,2-e]pyrazin-4-on,
- 8-[3-(4-Carboxybenzyl)ureido]-5H,10H-imidazo[1,2-a]indeno-[1,2-e]pyrazin-4-on,
- 8-[3-(2-Fluorbenzyl)ureido]-5H,10H-imidazo[1,2-a]indeno[1,2-e]pyrazin-4-on,
- 8-[3-(3-Fluorbenzyl)ureido]-5H,10H-imidazo[1,2-a]indeno[1,2-e]pyrazin-4-on,
- 8-[3-(4-Fluorbenzyl)ureido-5H,10H-imidazo[1,2-a]indeno[1,2-e]pyrazin-4-on,
- 9-(N-Ethylcarboxamido)-5H,10H-imidazo[1,2-a]indeno[1,2-e]pyrazin-4-on,
- 9-(3-Methylureido)-5H,10H-imidazo[1,2-a]indeno[1,2-e]pyrazin-4-on,
- 8-[3-(4-Methoxybenzyl)ureido]-5H,10H-imidazo[1,2-a]indeno[1,2-e]pyrazin-4-on,
und ihre Salze.

3. Verfahren zur Herstellung von Verbindungen der Formel (I) nach Anspruch 1, in denen R einen C=R₃-Rest bedeutet, worin R₃ ein Sauerstoffatom darstellt, dadurch **gekennzeichnet,** daß man eine entsprechende Verbindung der Formel (I), in der R einen C=R₃-Rest bedeutet und R₃ einen NOH-Rest bedeutet, hydrolysiert, das Produkt isoliert und gegebenenfalls in ein Salz umwandelt.

4. Verfahren zur Herstellung von Verbindungen der Formel (I) nach Anspruch 1, in denen R einen C=R₃-Rest bedeutet und R₃ einen NOH-Rest bedeutet, dadurch **gekennzeichnet,** daß man ein Alkylnitrit mit einer entsprechenden Verbindung der Formel (I), in der R einen CH-R₆-Rest bedeutet und R₆ ein Wasserstoffatom bedeutet, umsetzt, das Produkt isoliert und gegebenenfalls in ein Salz überführt.

5. Verfahren zur Herstellung von Verbindungen der Formel (I) nach Anspruch 1, in denen R einen CH-R₆-Rest bedeutet und R₆ ein Wasserstoffatom bedeutet, dadurch **gekennzeichnet,** daß man ein Derivat der Formel: 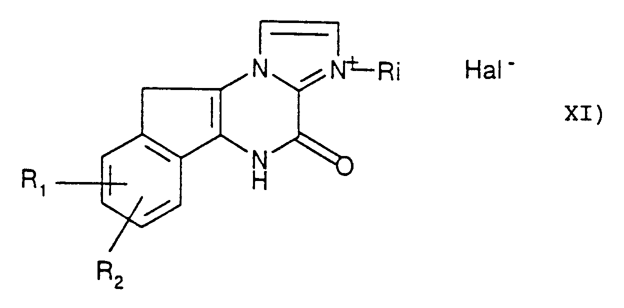 desalkyliert und von der Salzform befreit, worin R₁ und R₂ wie in Anspruch 1 definiert sind, Ri einen Alkylrest bedeutet und Hal ein Halogenatom bedeutet, das Produkt isoliert und gegebenenfalls in ein Salz überführt.

6. Verfahren zur Herstellung von Verbindungen der Formel (I) nach Anspruch 1, in denen R einen CH-R₆-Rest bedeutet und R₆ ein Wasserstoffatom bedeutet, dadurch **gekennzeichnet,** daß man gegebenenfalls in Anwesenheit von Ammoniumacetat ein Derivat der Formel: 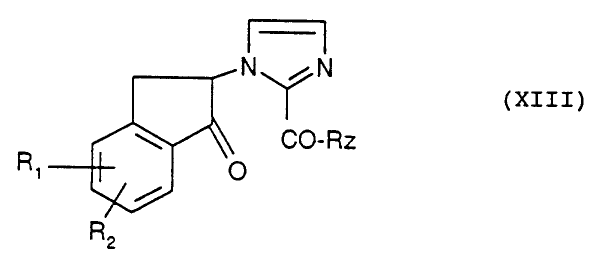 cyclisiert, worin R₁ und R₂ wie in Anspruch 1 definiert sind und Rz einen -NH₂-Rest oder -Oalk-Rest, worin alk einen Alkylrest bedeutet, bedeutet, das Produkt isoliert und gegebenenfalls in ein Salz überführt.

7. Verfahren zur Herstellung von Verbindungen der Formel (I) nach Anspruch 1, worin R einen CH-R₆-Rest bedeutet, in dem R₆ einen Aminorest bedeutet, dadurch **gekennzeichnet,** daß man eine Verbindung der Formel: 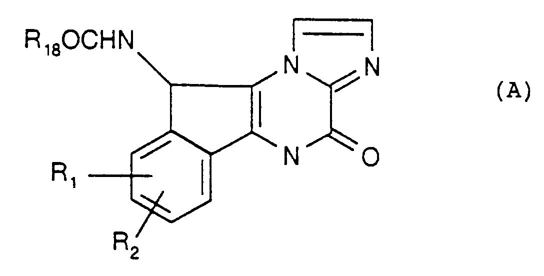 hydrolysiert, in der R₁ und R₂ wie in Anspruch 1 definiert sind und R₁₈ einen Alkylrest bedeutet, das Produkt isoliert und gegebenenfalls in ein Salz überführt.

8. Verfahren zur Herstellung von Verbindungen der Formel (I) nach Anspruch 1, in denen R einen CH-R₆-Rest bedeutet, worin R₆ einen Aminorest bedeutet, dadurch **gekennzeichnet,** daß man eine entsprechende Verbindung der Formel (I), in der R einen C=R₃-Rest bedeutet, worin R₃ einen NOH-Rest bedeutet, reduziert, das Produkt isoliert und gegebenenfalls in ein Salz überführt.

9. Verfahren zur Herstellung von Verbindungen der Formel (I) nach Anspruch 1, in denen R₁ einen Hydroxyrest bedeutet, dadurch **gekennzeichnet,** daß man ein Derivat der Formel: 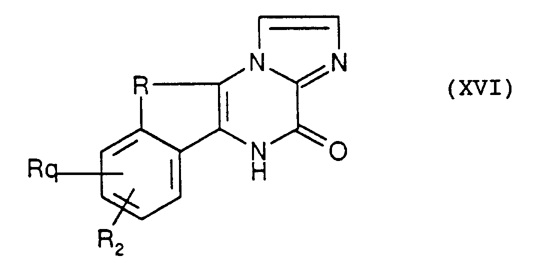 hydrolysiert, worin R und R₂ wie in Anspruch 1 definiert sind und Rq einen Alkoxyrest bedeutet, das Produkt isoliert und gegebenenfalls in ein salz überführt.

10. Verfahren zur Herstellung von Verbindungen der Formel (I) nach Anspruch 1, worin R₁ einen Carboxyrest bedeutet, dadurch **gekennzeichnet,** daß man ein Derivat der Formel: 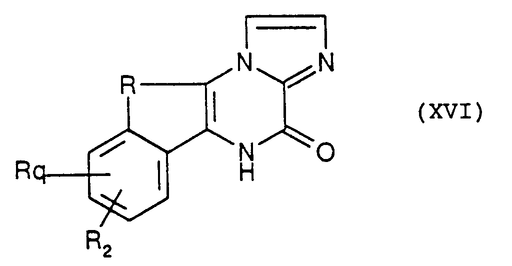 hydrolysiert, worin R und R₂ wie in Anspruch 1 definiert sind und Rq einen Cyanorest bedeutet, das Produkt isoliert und gegebenenfalls in ein Salz überführt.

11. Verfahren zur Herstellung von Verbindungen der Formel (I) nach Anspruch 1, in denen R₁ einen -NHCHO-Rest bedeutet, dadurch **gekennzeichnet,** daß man eine Verbindung der Formel: 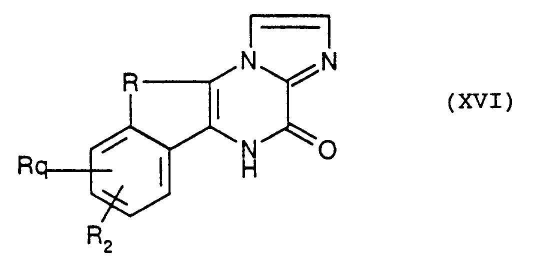 in der R und R₂ wie in Anspruch 1 definiert sind und Rq einen Aminorest bedeutet, mit H₃C-COOCHO umsetzt, das Produkt isoliert und gegebenenfalls in ein Salz überführt.

12. Verfahren zur Herstellung von Verbindungen der Formel (I) nach Anspruch 1, worin R₁ einen -N(alk)-CO-NR₈R₉- oder -NH-CSNR₈R₉-Rest, worin R₈ und R₉ keine Wasserstoffatome sind, -NH-CO-NR₉R₁₂-Rest, worin R₉ und R₁₂ keine Wasserstoffatome sind, -NH-COR₁₀-, -NH-SO₂-NR₇R₉-Rest, worin R₇ ein Alkylrest ist, oder -NH-SO₂-alk-Rest bedeutet, dadurch **gekennzeichnet,** daß man eine Verbindung der Formel: 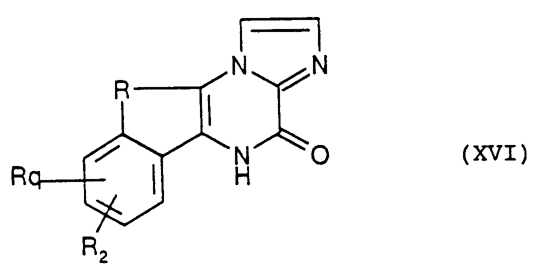 in der R und R₂ wie in Anspruch 1 definiert sind und Rq einen -NH₂- oder -NH-alk-Rest bedeutet, worin alk wie in Anspruch 1 definiert ist, mit einem Derivat der Formel Hal-Rr umsetzt, worin Hal ein Halogenatom bedeutet, Rr einen CO-NR₈R₉-, -CSNR₈R₉-Rest, in denen R₈ und R₉ wie in Anspruch 1 definiert sind, ausgenommen Wasserstoff, -CO-NR₉R₁₂-Rest, worin R₉ und R₁₂ wie in Anspruch 1 definiert sind, ausgenommen Wasserstoff, -COR₁₀-Rest, worin R₁₀ wie in Anspruch 1 definiert ist, -SO₂-NR₇R₉-Rest, worin R₉ wie in Anspruch 1 definiert ist, bedeutet, und R₇ einen Alkyl- oder -SO₂-alk-Rest bedeutet, worin alk wie in Anspruch 1 definiert ist, umsetzt, das Produkt isoliert und gegebenenfalls in ein Salz überführt.

13. Verfahren zur Herstellung von Verbindungen der Formel (I) nach Anspruch 1, in denen R₁ einen -N(alk)-CO-NR₈R₉- oder -NH-CO-NR₉R₁₂-Rest bedeutet, dadurch **gekennzeichnet,** daß man ein Derivat der Formel: 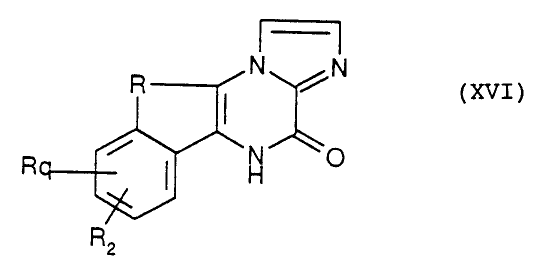 in der R und R₂ wie in Anspruch 1 definiert sind und Rq einen -NH₂- oder -NH-alk-Rest bedeutet, worin alk wie in Anspruch 1 definiert ist, mit einem Halogenotrialkylsilan in Gegenwart eines Alkalimetallhydrids, anschließend miteinem Kopplungsmittel und einem Amin der Formel HNR₈R₉ oder HN-R₉R₁₂, worin R₈, R₉ und R₁₂ wie in Anspruch 1 definiert sind, umsetzt, das Produkt isoliert und gegebenenfalls in ein Salz überführt.

14. Verfahren zur Herstellung von Verbindungen der Formel (I) nach Anspruch 1, in denen R₁ einen -NH-CO-NR₉R₁₂- oder -N(alk)-CO-NR₈R₉-Rest bedeutet, dadurch **gekennzeichnet,** daß man ein Derivat der Formel: 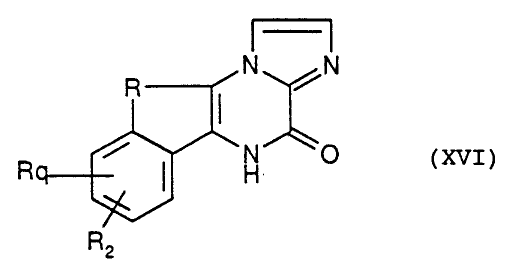 in dem R und R₂ wie in Anspruch 1 definiert sind und Rq einen -NH₂- oder -NH-alk-Rest bedeutet, in dem alk die gleichen Bedeutungen wie in Anspruch 1 besitzt, mit einem Derivat der Formel: 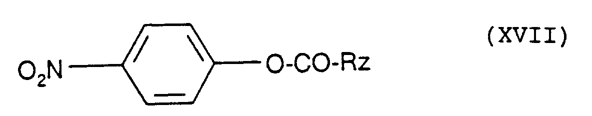 umsetzt, worin Rz einen -NR₉R₁₂- oder -NR₈R₉-Rest bedeutet, wobei R₇, R₈ und R₁₂ wie in Anspruch 1 definiert sind, das Produkt isoliert und gegebenenfalls in ein Salz überführt.

15. Verfahren zur Herstellung von Verbindungen der Formel (I) nach Anspruch 1, in denen R₁ einen -NH-CONR₉R₁₂-Rest bedeutet, R₉ ein Wasserstoffatom bedeutet und R₁₂ einen Phenylrest, substituiert durch einen Aminorest, bedeutet, dadurch **gekennzeichnet,** daß man eine entsprechende Verbindung der Formel (I), in der R₁ einen -NH-CONR₉R₁₂-Rest bedeutet, R₉ ein Wasserstoffatom bedeutet und R₁₂ einen durch eine Nitrogruppe substituierten Phenylrest bedeutet, reduziert, das Produkt isoliert und gegebenenfalls in ein Salz überführt.

16. Verfahren zur Herstellung von Verbindungen der Formel (I) nach Anspruch 1, in denen R₁ einen -NH-CONR₉R₁₂-, -N(alk)-CONR₈R₉-, -NHCSNR₈R₉-Rest, worin R₉ ein Wasserstoffatom bedeutet, R₈ ein Wasserstoffatom oder einen Alkyl- oder Phenylrest bedeutet und R₁₂ ein Wasserstoffatom oder einen Alkylrest, Phenylalkylrest, der gegebenenfalls substituiert ist, bedeutet, -alk-COOR₂₁ oder substituierten Phenylrest bedeutet, dadurch **gekennzeichnet,** daß man eine Verbindung der Formel: 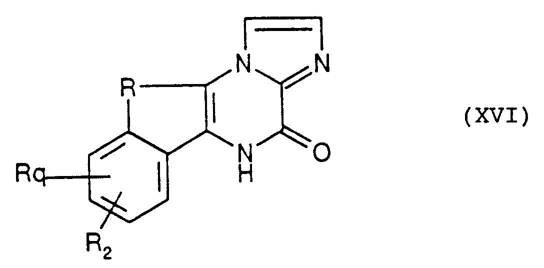 worin R und R₂ wie in Anspruch 1 definiert sind und Rq einen -NH₂- oder -NHalk-Rest bedeutet, wobei alk wie in Anspruch 1 definiert ist, mit einem Derivat der Formel Rs=C=N-Rt umsetzt, worin Rt einen -Si(CH₃)₃-, Alkyl-, Phenylalkylrest, dessen Phenylkern gegebenenfalls durch einen oder mehrere Substituenten, ausgewählt aus Halogenatomen, Alkoxyresten und den Resten -COOR₂₁, -alk-COOR₂₁ oder Phenylresten, substituiert durch einen oder mehrere Substituenten, ausgewählt aus Halogenatomen und Alkoxy-, Nitro-, Amino-, Cyano- und -COOR₂₁-Resten, substituiert ist, bedeutet, worin alk und R₂₁ wie in Anspruch 1 definiert sind und Rs ein Sauerstoff- oder Schwefelatom bedeutet, das Produkt isoliert und gegebenenfalls in ein Salz überführt.

17. Verfahren zur Herstellung von Verbindungen der Formel (I) nach Anspruch 1, in denen R₁ einen -NH-CO-R₁₀-Rest bedeutet, worin R₁₀ einen Alkoxyrest bedeutet, dadurch **gekennzeichnet,** daß man ein Derivat der Formel: 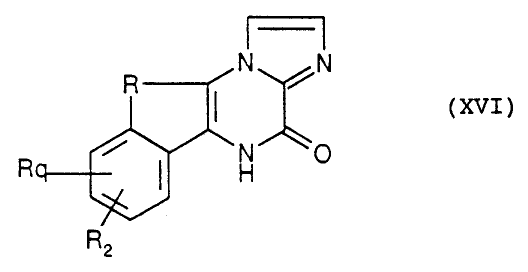 worin R und R₂ wie in Anspruch 1 definiert sind und Rq eine Aminogruppe bedeutet, mit einem Hal-COOalk-Derivat, worin Hal ein Halogenatom bedeutet und alk einen Alkylrest bedeutet, umsetzt und die so erhaltene Dialkoxygruppe gegebenenfalls in Gegenwart eines Amins erhitzt, das Produkt isoliert und gegebenenfalls in ein salz überführt.

18. Verfahren zur Herstellung von Verbindungen der Formel (I) nach Anspruch 1, in denen R₁ einen -CO-NR₇R₉-Rest bedeutet, dadurch **gekennzeichnet,** daß man eine entsprechende Verbindung der Formel (I), worin R₁ eine Carboxygruppe oder ein reaktives Derivat dieser Säure bedeutet, mit einem Amin HNR₇R₉ umsetzt, das Produkt isoliert und gegebenenfalls in ein Salz überführt.

19. Verfahren zur Herstellung von Verbindungen der Formel (I) nach Anspruch 1, in denen R₁ einen -NR₉R₁₁-Rest bedeutet, dadurch **gekennzeichnet,** daß man ein Derivat der Formel: 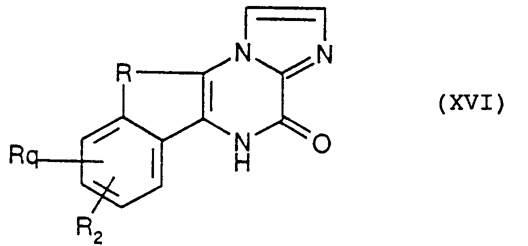 worin R und R₂ wie in Anspruch 1 definiert sind und Rq einen -NH₂- oder -NHalk-Rest bedeutet, worin alk wie in Anspruch 1 definiert ist, mit einem Hal-R₁₁-Derivat umsetzt, worin R₁₁ wie in Anspruch 1 definiert ist, das Produkt isoliert und gegebenenfalls in ein Salz überführt.

20. Verfahren zur Herstellung von Verbindungen der Formel (I) nach Anspruch 1, in denen R₁ einen 2-oxo-1-imidazolidinylrest bedeutet, dadurch **gekennzeichnet,** daß man ein Derivat der Formel: 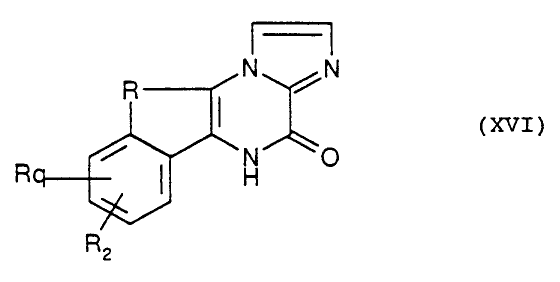 in dem R und R₂ wie in Anspruch 1 definiert sind und Rq einen -NH-CO-NH-(CH₂)ₙ-Hal-Rest bedeutet, worin Hal ein Halogenatom bedeutet, alk einen Alkylrest bedeutet und n den Wert 2 hat, cyclisiert, das Produkt isoliert und gegebenenfalls in ein Salz überführt.

21. Verfahren zur Herstellung von Verbindungen der Formel (I) nach Anspruch 1, in denen R₁ einen -NH-CO-NR₉R₁₂- oder -NH-COR₁₀-Rest bedeutet, worin R₁₂ und R₁₀ -alk-COOR₂₁-Reste bedeuten und R₂₁ ein Wasserstoffatom bedeutet, dadurch **gekennzeichnet,** daß man eine entsprechende Verbindung der Formel (I), in der R₁ einen -NH-CO-NR₉R₁₂- oder -NH-COR₁₀-Rest bedeutet, worin R₁₂ und R₁₀ -alk-COOR₂₁-Reste bedeuten und R₂₁ einen Alkylrest bedeutet, hydrolysiert, das Produkt isoliert und gegebenenfalls in ein Salz überführt.

22. Verfahren zur Herstellung von Verbindungen der Formel (I) nach Anspruch 1, in denen R₁ einen -NR₉R₁₁-Rest bedeutet, R₁₁ einen 2-Imidazolinylrest bedeutet, dadurch **gekennzeichnet,** daß man ein Derivat der Formel: 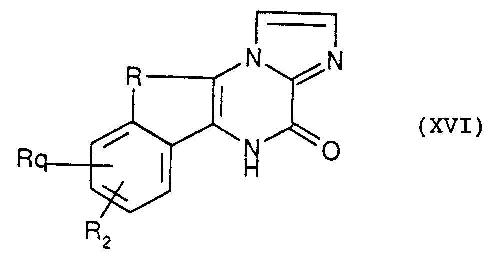 in dem R und R₂ wie in Anspruch 1 definiert sind und Rq einen -N=C(SCH₃)-NH-(CH₂)₂-NH₂-Rest bedeutet, cyclisiert, das Produkt isoliert und gegebenenfalls in ein Salz überführt.

23. Medikamente, enthaltend als Wirkstoff mindestens eine Verbindung nach einem der Ansprüche 1 oder 2 oder ein pharmazeutisch verträgliches Salz einer solchen Verbindung.
